# EUROPEAN PATENT SPECIFICATION

(11) **EP 1 694 274 B1**
(45) Date of publication and mention of the grant of the patent: **24.04.2013**
(21) Application number: 04813334.2
(22) Date of filing: 03.12.2004
(51) Int. Cl.: A01N 43/04, A61K 47/48, C07K 14/535, A61K 38/19, A61P 31/00

(54) **Glycopegylated granulocyte colony stimulating factor**
Glykopegylierter Granulocyten-Kolonie-stimulierender Faktor
Facteur de stimulation de colonies de granulocytes glycopegylé

(30) Priority: 03.12.2003 US 526796 P; 26.01.2004 US 539387 P; 23.03.2004 US 555813 P; 11.05.2004 US 570282 P; 29.07.2004 US 592744 P; 29.09.2004 US 614518 P; 29.10.2004 US 623387 P
(43) Date of publication of application: 30.08.2006
(73) Proprietor: BioGeneriX AG, 68199 Mannheim (DE)
(72) Inventor: DEFREES, Shawn, North Wales, PA 19454 (US); CLAUSEN, Henrik, DK-2840 Holte (DK); ZOPF, David, A., Wayne, PA 19087 (US); WANG, Zhi-Guang, Dresher, PA 19015 (US); SCHWARTZ, Marc, West Windsor, NJ 08550 (US); WU, Bingyuan, Horsham, PA 19044 (US); BOWE, Caryn, Doylestown, PA 19044 (US)
(74) Representative: Neuefeind, Regina
(86) International application number: PCT/US2004/041004
(87) International publication number: WO 2005/055946

(56) References cited:
- WO-A-01/87925
- WO-A-03/006501
- WO-A-03/031464
- US-A- 4 414 147
- US-A- 5 932 462
- US-A1- 2002 004 483
- US-A1- 2002 142 964
- US-B1- 6 183 738
- DUDZIAK G ET AL: "Cyclodextrin-assisted Glycan Chain Extension on a Protected Glycosyl Amino Acid" TETRAHEDRON, ELSEVIER SCIENCE PUBLISHERS, AMSTERDAM, NL, vol. 56, no. 32, 4 August 2000 (2000-08-04), pages 5865-5869, XP004229184 ISSN: 0040-4020

## Description

### BACKGROUND OF THE INVENTION

Granulocyte colony stimulating factor (G-CSF) is a glycoprotein which stimulates the survival, proliferation, differentiation and function of neutrophil granulocyte progenitor cells and mature neutrophils. The two forms of recombinant human G-CSF in clinical use are potent stimulants of neutrophil granulopoiesis and have demonstrated efficacy in preventing infectious complications of some neutropenic states. They can be used to accelerate neutrophil recovery from myelosuppressive treatments.

G-CSF decreases the morbidity of cancer chemotherapy by reducing the incidence of febrile neutropenia, the morbidity of high-dose chemotherapy supported by marrow transplantation, and the incidence and duration of infection in patients with severe chronic neutropenia. Further, G-CSF has recently been shown to have therapeutic when administered after the onset of myocardial infarction.

The human form of G-CSF was cloned by groups from Japan and the U.S.A. in 1986 *(see e.g.,* Nagata et al. Nature 319: 415-418, 1986). The natural human glycoprotein exists in two forms, one of 175 and the other of 178 amino acids. The more abundant and more active 175 amino acid form has been used in the development of pharmaceutical products by recombinant DNA technology.

The recombinant human G-CSF synthesised in an *E. coli* expression system is called filgrastim. The structure of filgrastim differs slightly from the natural glycoprotein. The other form of recombinant human G-CSF is called *lenograstim* and is synthesised in Chinese hamster ovary (CHO) cells.

hG-CSF is a monomeric protein that dimerizes the G-CSF receptor by formation of a 2:2 complex of 2 G-CSF molecules and 2 receptors (Horan et al. Biochemistry, 35(15): 4886-96 (1996)). The following hG-CSF residues have been identified by X-ray crystalographic studies as being part of the receptor binding interfaces: G4, P5, A6, S7, S8, L9, P10, Q11, S12, L15, K16, E19, Q20, L108, D109, D112, T115, T116, Q119, E122, E123, and L124 *(see e.g.,* Aritomi et al., (1999) Nature 401: 713).

The commercially available forms ofrhG-CSF have a short-term pharmacological effect and must often be administered more once a day for the duration of the leukopenic state. A molecule with a longer circulation half-life would decrease the number of administrations necessary to alleviate the leukopenia and prevent consequent infections. Another problem with currently available rG-CSF products is the occurrence of dose-dependent bone pain. Since bone pain is experienced by patients as a significant side effect of treatment with rG-CSF, it would be desirable to provide a rG-CSF product that does not cause bone pain, either by means of a product that inherently does not have this effect or that is effective in a sufficiently small dose that no bone pain is caused. Thus, there is clearly a need for improved recombinant G-CSF molecules.

Protein-engineered variants of hG-CSF have been reported (U.S. Pat. No. 5,581,476, U.S. 5,214,132, U.S. 5,362,853, U.S. 4,904,584 and Riedhaar-Olson et al. Biochemistry 35: 9034-9041, 1996). Modification of hG-CSF and other polypeptides so as to introduce at least one additional carbohydrate chain as compared to the native polypeptide has also been reported (U.S. Pat. No. 5,218,092). In addition, polymer modifications of native hG-CSF, including attachment of PEG groups, have been reported and studied (*see e.g.,* Satake-Ishikawa et al., (1992) Cell Structure and Function 17: 157; Bowen et al. (1999) Experimental Hematology 27: 425; U.S. Pat. No. 5,824,778, U.S. 5,824,784, WO 96/11953, WO 95/21629, and WO 94/20069).

The attachment of synthetic polymers to the peptide backbone in an attempt to improve the pharmacokinetic properties of glycoprotein therapeutics is known in the art. An exemplary polymer that has been conjugated to peptides is poly(ethylene glycol) ("PEG"). The use of PEG to derivatize peptide therapeutics has been demonstrated to reduce the immunogenicity of the peptides. For example, U.S. Pat. No. 4,179,337 (Davis et al.) discloses non-immunogenic polypeptides such as enzymes and peptide hormones coupled to polyethylene glycol (PEG) or polypropylene glycol. In addition to reduced immunogenicity, the clearance time in circulation is prolonged due to the increased size of the PEG-conjugate of the polypeptides in question.

The principal mode of attachment of PEG, and its derivatives, to peptides is a non-specific bonding through a peptide amino acid residue *(see e.g.,* U.S. Patent No. 4,088,538 U.S. Patent No. 4,496,689, U.S. Patent No. 4,414,147, U.S. Patent No. 4,055,635, and PCT WO 87/00056). Another mode of attaching PEG to peptides is through the non-specific oxidation of glycosyl residues on a glycopeptide (*see e.g.,* WO 94/05332).

In these non-specific methods, poly(ethyleneglycol) is added in a random, non-specific manner to reactive residues on a peptide backbone. Of course, random addition of PEG molecules has its drawbacks, including a lack of homogeneity of the final product, and the possibility for reduction in the biological or enzymatic activity of the peptide. Therefore, for the production of therapeutic peptides, a dcrivitization strategy that results in the formation of a specifically labeled, readily characterizable, essentially homogeneous product is superior. Such methods have been developed.

Specifically labeled, homogeneous peptide therapeutics can be produced in *vitro* through the action of enzymes. Unlike the typical non-specific methods for attaching a synthetic polymer or other label to a peptide, enzyme-based syntheses have the advantages of regioselectivity and stereoselectivity. Two principal classes of enzymes for use in the synthesis of labeled peptides are glycosyltransferases (*e.g.,* sialyltransferases, oligosaccharyltransferases, N-acetylglucosaminyltransferases), and glycosidases. These enzymes can be used for the specific attachment of sugars which can be subsequently modified to comprise a therapeutic moiety. Alternatively, glycosyltransferases and modified glycosidases can be used to directly transfer modified sugars to a peptide backbone (*see e.g.,* U.S. Patent 6,399,336, and U.S. Patent Application Publications 20030040037, 20040132640, 20040137557, 20040126838, and 20040142856. Methods combining both chemical and enzymatic synthetic elements are also known (*see e.g.,* Yamamoto et al. Carbohydr. Res. 305: 415-422 (1998) and U.S. Patent Application Publication 20040137557.

In response to the need for improved therapeutic G-CSF, the present invention provides a glycopegylated G-CSF that is therapeutically active and which has pharmacokinetic parameters and properties that are improved relative to an identical, or closely analogous, G-CSF peptide that is not glycopegylated. Furthermore, the invention provides method for producing cost effectively and on an industrial scale the improved G-CSF peptides of the invention.

### SUMMARY OF THE INVENTION

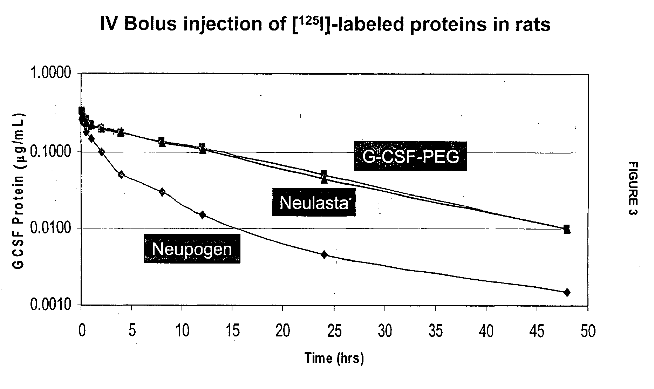

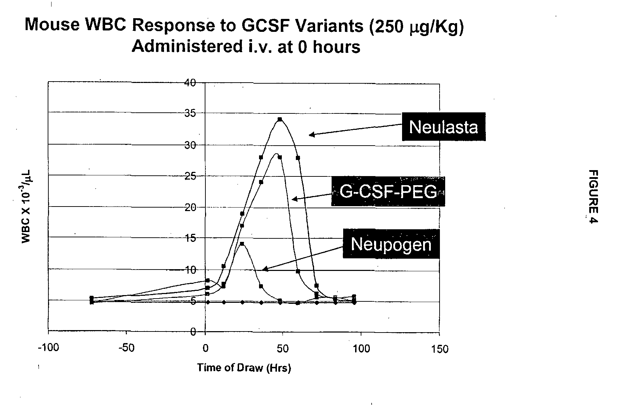

It has now been discovered that the controlled modification of Granulocyte colony stimulating factor (G-CSF) with one or more poly(ethylene glycol) moieties affords a novel G-CSF derivative with pharmacokinetic properties that are improved relative to the corresponding native (un-pegylated) G-CSF **(****FIG. 3****).** Moreover, the pharmacological activity of the glycopegylated G-CSF is approximately the same as the commercially available mono-pegylated filgrastim **(****FIG. 4****).**

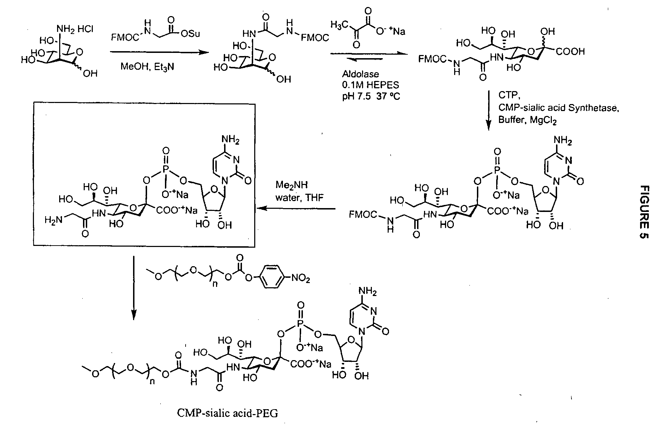

In an exemplary embodiment, "glycopeglyated" G-CSF molecules of the invention are produced by the enzyme mediated formation of a conjugate between a glycosylated or non-glycosylated G-CSF peptide and an enzymatically transferable saccharyl moiety that includes a poly(ethylene glycol) moiety within its structure The PEG moiety is attached to the saccharyl moiety directly (i.e., through a single group formed by the reaction of two reactive groups) or through a linker moiety, e.g., substituted or unsubstituted alkyl, substituted or unsubstituted heteroalkyl, etc. An exemplary transferable PEG-saccharyl structure is set forth in **FIG. 5****.**

Thus, in one aspect, the present invention provides a conjugate between a PEG moiety, *e.g.,* PEG and a peptide that has an *in vivo* activity similar or otherwise analogous to art-recognized G-CSF. In the conjugate of the invention, the PEG moiety is covalently attached to the peptide via an intact glycosyl linking group. Exemplary intact glycosyl linking groups include sialic acid moieties that are derivatized with PEG.

The present invention provides a G-CSF peptide that includes the moiety:

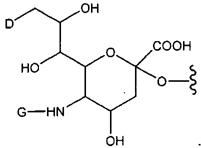

In the formula above, D is -OH or R¹-L-HN-. The symbol G represents R¹-L-or -C(O)(C₁-C₆)alkyl. R¹ is a moiety comprising a branched poly(ethylene glycol) residue as defined in the claims; and L is a linker which is a member selected from a bond, substituted or unsubstituted alkyl and substituted or unsubstituted heteroalkyl. Generally, when D is OH, G is R¹-L-, and when G is -C(O)(C₁-C₆)alkyl, D is R¹-L-NH-. In the modified sialic acid structures set forth herein, COOH also represents COO⁻ and/or a salt thereof.

In another aspect, the invention provides a method of making a PEG-ylated G-CSF comprising the moiety above. The method of the invention includes (a) contacting a substrate G-CSF peptide with a PEG-sialic acid donor and an enzyme that transfers the PEG-sialic acid onto an amino acid or glycosyl residue of the G-CSF, under conditions appropriate for the transfer. An exemplary PEG-sialic acid donor moiety has the formula:

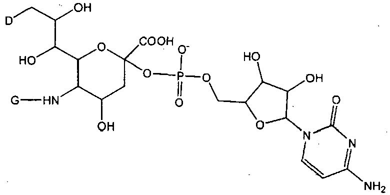

In one embodiment the host is mammalian cell. In other embodiments the host cell is an insect cell, plant cell, a bacteria or a fungi.

The pharmacokinetic properties of the compounds of the invention are readily varied by altering the structure, number or position of the glycosylation site(s) of the peptide. Thus, it is within the purview of the present application to add one or more mutation that inserts an O- or N-linked glycosylation site into the G-CSF peptide that is not present in the wild type. Antibodies to these mutants and their glycosylated final products and intermediates are also described.

In another aspect, the invention provides a G-CSF conjugate having a population of PEG moiety moieties, *e.g.,* PEG, covalently bound thereto through an intact glycosyl linking group. In the conjugate of the invention, essentially each member of the population is bound via the glycosyl linking group to a glycosyl residue of the peptide, and each glycosyl residue has the same structure.

In exemplary embodiment, the present invention provides a G-CSF conjugate having a population of PEG moiety moieties, e.g., PEG, covalently bound thereto through an intact glycosyl linking group. In the conjugate of the invention, essentially each member of the population is bound to an amino acid residue of the peptide, and each of the amino acid residues to which the polymer is bound has the same structure. For example, if one peptide includes an Thr linked glycosyl residue, at least about 70%, 80%, 90%, 95%, 97%, 99%, 99.2%, 99.4%, 99.6%, or more preferably 99.8% of the peptides in the population will have the same glycosyl residue covalently bound to the same Thr residue. The discussion above is equally relevant for both O-glycosylation and N-glycosylation sites.

Also provided is a pharmaceutical composition. The composition includes a pharmaceutically acceptable carrier and a covalent conjugate between a non-naturally-occurring, PEG moiety and a glycosylated or non-glycosylated G-CSF peptide.

Other objects and advantages of the invention will be apparent to those of skill in the art from the detailed description that follows.

### DESCRIPTION OF THE DRAWINGS

**FIG. 1** is the structure of G-CSF, showing the presence and location of a potential glycosylation at Thr 133 (Thr 134 if a methionine is present).

**FIG. 2** is a scheme showing an exemplary embodiment of the invention in which a carbohydrate residue on a G-CSF peptide is remodeled by enzymatically adding a GalNAc moiety to the glycosyl residue at Thr 133 (Thr 134 is methionine is present) prior to adding a saccharyl moiety derivatized with PEG.

**FIG. 3** is a plot comparing the in vivo residence lifetimes ofunglycosylated G-CSF, Neulasta™ and enzymatically glycopegylated G-CSF.

**FIG. 4** is a plot comparing the activities of the species shown in **FIG. 3****.**

**FIG. 5** is a synthetic scheme for producing an exemplary PEG-glycosyl linking group precursor (modified sugar) of us in preparing the conjugates of the invention.

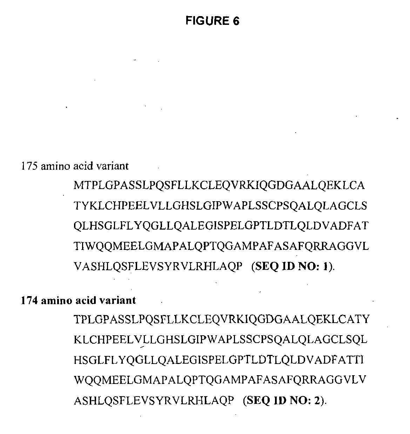

**FIG. 6** shows exemplary G-CSF amino acid sequences. SEQ ID NO:1 is the 175 amino acid variant, wherein the first amino acid is methionine and there is a threonine residue at Thr 134. SEQ ID NO:2 is a 174 amino acid variant which has the same sequence as the 175 amino acid variant except thet the leading methionine is missing, thus the sequence begins with T and there is a Threonine residue at position 133.

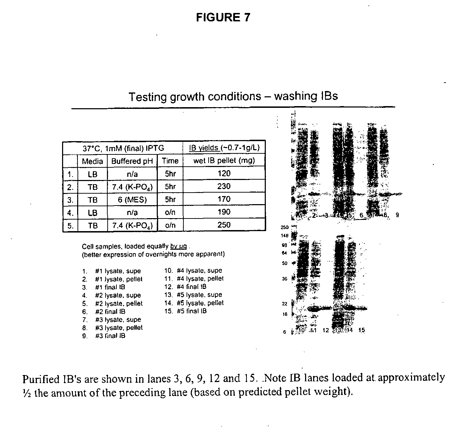

**FIG. 7** illustrates some exemplary modified sugar nucleotides useful in the practice of the invention.

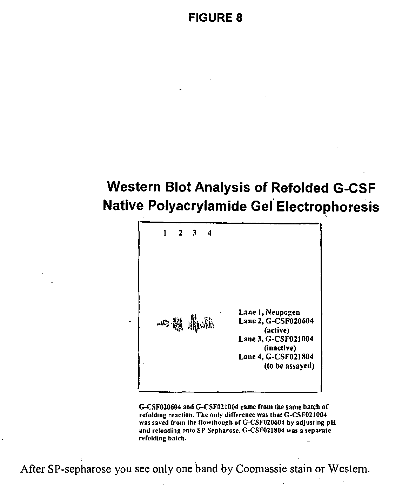

**FIG. 8** illustrates further exemplary modified sugar nucleotides useful in the practice of the invention.

**FIG. 9** demonstrates production of recombinant GCSF in bacteria grown in various media and induced with IPTG.

**FIG. 10** provides Western blot analysis of refolded GCSF after SP-sepharose chromatography.

**FIG. 11** is a table of sialyl transferases that are of use for transferring to an acceptor the modified sialic acid species set forth herein and unmodified sialic acid.

### DETAILED DESCRIPTION OF THE INVENTION AND THE PREFERRED EMBODIMENTS

### Abbreviations

PEG, poly(ethyleneglycol); PPG, poly(propyleneglycol); Ara, arabinosyl; Fru, fructosyl; Fuc, fucosyl; Gal, galactosyl; GalNAc, N-acetylgalactosaminyl; Glc, glucosyl; GlcNAc, N-acetylglucosaminyl; Man, mannosyl; ManAc, mannosaminyl acetate; Xyl, xylosyl; and NeuAc, sialyl (N-acetylneuraminyl); M6P, mannose-6-phosphate; Sia, sialic acid, N-acetylneuraminyl, and derivatives and analogues thereof.

### Definitions

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization are those well known and commonly employed in the art. Standard techniques are used for nucleic acid and peptide synthesis. The techniques and procedures are generally performed according to conventional methods in the art and various general references (*see generally,* Sambrook et al. MOLECULAR CLONING: A LABORATORY MANUAL, 2d ed. (1989) Cold Spring Harbor Laboratory Press, Cold Spring Harbor, N.Y. ), which are provided throughout this document. The nomenclature used herein and the laboratory procedures in analytical chemistry, and organic synthetic described below are those well known and commonly employed in the art. Standard techniques, or modifications thereof, are used for chemical syntheses and chemical analyses.

All oligosaccharides described herein are described with the name or abbreviation for the non-reducing saccharide (*i.e*., Gal), followed by the configuration of the glycosidic bond (α or β), the ring bond (1 or 2), the ring position of the reducing saccharide involved in the bond (2, 3, 4, 6 or 8), and then the name or abbreviation of the reducing saccharide (*i.e.*, GlcNAc). Each saccharide is preferably a pyranose. For a review of standard glycobiology nomenclature *see,* Essentials of Glycobiology Varki et al. eds. CSHL Press (1999).

Oligosaccharides are considered to have a reducing end and a non-reducing end, whether or not the saccharide at the reducing end is in fact a reducing sugar. In accordance with accepted nomenclature, oligosaccharides are depicted herein with the non-reducing end on the left and the reducing end on the right.

The term "sialic acid" refers to any member of a family of nine-carbon carboxylated sugars. The most common member of the sialic acid family is N-acetylneuraminic acid (2-keto-5-acetamido-3,5-dideoxy-D-glycero-D-- galactononulopyranos-1-onic acid (often abbreviated as Neu5Ac, NeuAc, or NANA). A second member of the family is N-glycolyl-neuraminic acid (Neu5Gc or NeuGc), in which the N-acetyl group ofNeuAc is hydroxylated. A third sialic acid family member is 2-keto-3-deoxy-nonulosonic acid (KDN) (Nadano et al. (1986) J. Biol. Chem. 261: 11550-11557; Kanamori et al., J Biol. Chem. 265: 21811-21819 (1990)). Also included are 9-substituted sialic acids such as a 9-O-C₁-C₆ acyl-Neu5Ac like 9-O-lactyl-Neu5Ac or 9-O-acetyl-Neu5Ac, 9-deoxy-9-fluoro-Neu5Ac and 9-azido-9-deoxy-Neu5Ac. For review of the sialic acid family, *see, e.g.,* Varki, Glycobiology 2: 25-40 (1992); Sialic Acids: Chemistry, Metabolism and Function, R. Schauer, Ed. (Springer-Verlag, New York (1992)). The synthesis and use of sialic acid compounds in a sialylation procedure is disclosed in international application WO 92/16640, published October 1, 1992.

The term "Granuloctye Colony Stimulating Factor" or "Granuloctye Colony Stimulating Factor peptide", or "G-CSF" or "G-CSF peptide" refers to any wild type or mutated peptide, recombinant, or native, or any fragment of G-CSF that has an activity that is or that mimics that of native GCSF. The term also generally encompasses non-peptide G-CSF mimetics. In an exemplary embodiment a G-CSF peptide has the amino acid sequence shown in SEQ ID NO:1. In other exemplary embodiments a G-CSF peptide has a sequence selected from SEQ ID NOs:3-11.

The term "Granuloctye Colony Stimulating Factor activity" refers to any activity including receptor binding and activation, inhibition of receptor binding, or any biochemical or physiological reaction that is normally affected by the action of wild-type Granuloctye Colony Stimulating Factor. Granuloctye Colony Stimulating Factor activity can arise from the action of any Granuloctye Colony Stimulating Factor peptide, as defined above.

"Peptide" refers to a polymer in which the monomers are amino acids and are joined together through amide bonds, alternatively referred to as a polypeptide. Additionally, unnatural amino acids, for example, β-alanine, phenylglycine and homoarginine are also included. Amino acids that are not gene-encoded may also be used in the present invention. Furthermore, amino acids that have been modified to include reactive groups, glycosylation sites, polymers, therapeutic moieties, biomolecules and the like may also be used in the invention. All of the amino acids used in the present invention may be either the D - or L-isomer. The L-isomer is generally preferred. In addition, other peptidomimetics are also useful in the present invention. As used herein, "peptide" refers to both glycosylated and unglycosylated peptides. Also included are peptides that are incompletely glycosylated by a system that expresses the peptide. For a general review, *see,* Spatola, A. F., in CHEMISTRY AND BIOCHEMISTRY OF AMINO ACIDS, PEPTIDES AND PROTEINS, B. Weinstein, eds., Marcel Dekker, New York, p. 267 (1983).

The term "peptide conjugate," refers to species of the invention in which a peptide is conjugated with a modified sugar as set forth herein.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g.,* hydroxyproline, γ-carboxyglutamate, and O-phosphoserine. Amino acid analogs refers to compounds that have the same basic chemical structure as a naturally occurring amino acid, *i.e.,* an α carbon that is bound to a hydrogen, a carboxyl group, an amino group, and an R group, *e.g.,* homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that function in a manner similar to a naturally occurring amino acid. As used herein, "amino acid," whether it is in a linker or a component of a peptide sequence refers to both the D- and L-isomer of the amino acid as well as mixtures of these two isomers.

As used herein, the term "modified sugar," refers to a naturally- or non-naturally-occurring carbohydrate that is enzymatically added onto an amino acid or a glycosyl residue of a peptide in a process of the invention. The modified sugar is selected from a number of enzyme substrates including, sugar nucleotides (mono-, di-, and tri-phosphates), activated sugars (*e.g*., glycosyl halides, glycosyl mesylates) and sugars that are neither activated nor nucleotides. The "modified sugar" is covalently functionalized with a "modifying group." Useful modifying groups include, but are not limited to, PEG moieties, therapeutic moieties, diagnostic moieties, biomolecules and the like. The modifying group is preferably not a naturally occurring, or an unmodified carbohydrate. The locus of functionalization with the modifying group is selected such that it does not prevent the "modified sugar" from being added enzymatically to a peptide.

The term "water-soluble" refers to moieties that have some detectable degree of solubility in water. Methods to detect and/or quantify water solubility are well known in the art. Exemplary PEG moieties include peptides, saccharides, poly(ethers), poly(amines), poly(carboxylic acids) and the like. Peptides can have mixed sequences or be composed of a single amino acid, *e.g.* poly(lysine). Similarly, saccharides can be of mixed sequence or composed of a single saccharide subunit, e.g, dextran, amylose, chitosan, and poly(sialic acid). An exemplary poly(ether) is poly(ethylene glycol). Poly(ethylene imine) is an exemplary polyamine, and poly(acrylic) acid is a representative poly(carboxylic acid)

The term, "glycosyl linking group," as used herein refers to a glycosyl residue to which an agent (*e.g.,* PEG moiety, therapeutic moiety, biomolecule) is covalently attached. In the methods of the invention, the "glycosyl linking group" becomes covalently attached to a glycosylated or unglycosylated peptide, thereby linking the agent to an amino acid and/or glycosyl residue on the peptide. A "glycosyl linking group" is generally derived from a "modified sugar" by the enzymatic attachment of the "modified sugar" to an amino acid and/or glycosyl residue of the peptide. An "intact glycosyl linking group" refers to a linking group that is derived from a glycosyl moiety in which the individual saccharide monomer that links the conjugate is not degraded, *e.g.,* oxidized, *e.g.,* by sodium metaperiodate. "Intact glycosyl linking groups" of the invention may be derived from a naturally occurring oligosaccharide by addition of glycosyl unit(s) or removal of one or more glycosyl unit from a parent saccharide structure.

The term "targeting moiety," as used herein, refers to species that will selectively localize in a particular tissue or region of the body. The localization is mediated by specific recognition of molecular determinants, molecular size of the targeting agent or conjugate, ionic interactions, hydrophobic interactions and the like. Other mechanisms of targeting an agent to a particular tissue or region are known to those of skill in the art. Exemplary targeting moieties include antibodies, antibody fragments, transferrin, HS-glycoprotein, coagulation factors, serum proteins, β-glycoprotein, G-CSF, GM-CSF, M-CSF, EPO and the like.

As used herein, "pharmaceutically acceptable carrier" includes any material, which when combined with the conjugate retains the conjugates' activity and is nonreactive with the subject's immune systems. Examples include, any of the standard pharmaceutical carriers such as a phosphate buffered saline solution, water, emulsions such as oil/water emulsion, and various types of wetting agents. Other carriers may also include sterile solutions, tablets including coated tablets and capsules. Typically such carriers contain excipients such as starch, milk, sugar, certain types of clay, gelatin, stearic acid or salts thereof, magnesium or calcium stearate, talc, vegetable fats or oils, gums, glycols, or other known excipients. Such carriers may also include flavor and color additives or other ingredients. Compositions comprising such carriers are formulated by well known conventional methods.

As used herein, "administering," means oral administration, administration as a suppository, topical contact, intravenous, intraperitoneal, intramuscular, intralesional, intranasal or subcutaneous administration, or the implantation of a slow-release device *e.g.,* a mini-osmotic pump, to the subject. Adminsitration is by any route including parenteral, and transmucosal (*e.g*., oral, nasal, vaginal, rectal, or transdermal). Parenteral administration includes, *e.g.,* intravenous, intramuscular, intra-arteriole, intradermal, subcutaneous, intraperitoneal, intraventricular, and intracranial. Moreover, where injection is to treat a tumor, *e.g.,* induce apoptosis, administration may be directly to the tumor and/or into tissues surrounding the tumor. Other modes of delivery include, but are not limited to, the use of liposomal formulations, intravenous infusion, transdermal patches, etc.
The term "ameliorating" or "ameliorate" refers to any indicia of success in the treatment of a pathology or condition, including any objective or subjective parameter such as abatement, remission or diminishing of symptoms or an improvement in a patient's physical or mental well-being. Amelioration of symptoms can be based on objective or subjective parameters; including the results of a physical examination and/or a psychiatric evaluation.

The term "therapy" refers to"treating" or "treatment" of a disease or condition including preventing the disease or condition from occurring in an animal that may be predisposed to the disease but does not yet experience or exhibit symptoms of the disease (prophylactic treatment), inhibiting the disease (slowing or arresting its development), providing relief from the symptoms or side-effects of the disease (including palliative treatment), and relieving the disease (causing regression of the disease).

The term "effective amount" or "an amount effective to"or a "therapeutically effective amount" or any gramatically equivalent term means the amount that, when administered to an animal for treating a disease, is sufficient to effect treatment for that disease.

The term "isolated" refers to a material that is substantially or essentially free from components, which are used to produce the material. For peptide conjugates of the invention, the term "isolated" refers to material that is substantially or essentially free from components, which normally accompany the material in the mixture used to prepare the peptide conjugate. "Isolated" and "pure" are used interchangeably. Typically, isolated peptide conjugates of the invention have a level of purity preferably expressed as a range. The lower end of the range of purity for the peptide conjugates is about 60%, about 70% or about 80% and the upper end of the range of purity is about 70%, about 80%, about 90% or more than about 90%.

When the peptide conjugates are more than about 90% pure, their purities are also preferably expressed as a range. The lower end of the range of purity is about 90%, about 92%, about 94%, about 96% or about 98%. The upper end of the range of purity is about 92%, about 94%, about 96%, about 98% or about 100% purity.

Purity is determined by any art-recognized method of analysis (e.g., band intensity on a silver stained gel, polyacrylamide gel electrophoresis, HPLC, or a similar means).

"Essentially each member of the population," as used herein, describes a characteristic of a population of peptide conjugates of the invention in which a selected percentage of the modified sugars added to a peptide are added to multiple, identical acceptor sites on the peptide. "Essentially each member of the population" speaks to the "homogeneity" of the sites on the peptide conjugated to a modified sugar and refers to conjugates of the invention, which are at least about 80%, preferably at least about 90% and more preferably at least about 95% homogenous.

"Homogeneity," refers to the structural consistency across a population of acceptor moieties to which the modified sugars are conjugated. Thus, in a peptide conjugate of the invention in which each modified sugar moiety is conjugated to an acceptor site having the same structure as the acceptor site to which every other modified sugar is conjugated, the peptide conjugate is said to be about 100% homogeneous. Homogeneity is typically expressed as a range. The lower end of the range of homogeneity for the peptide conjugates is about 60%, about 70% or about 80% and the upper end of the range of purity is about 70%, about 80%, about 90% or more than about 90%.

When the peptide conjugates are more than or equal to about 90% homogeneous, their homogeneity is also preferably expressed as a range. The lower end of the range of homogeneity is about 90%, about 92%, about 94%, about 96% or about 98%. The upper end of the range of purity is about 92%, about 94%, about 96%, about 98% or about 100% homogeneity. The purity of the peptide conjugates is typically determined by one or more methods known to those of skill in the art, *e.g.,* liquid chromatography-mass spectrometry (LC-MS), matrix assisted laser desorption mass time of flight spectrometry (MALDITOF), capillary electrophoresis, and the like.

"Substantially uniform glycoform" or a "substantially uniform glycosylation pattern," when referring to a glycopeptide species, refers to the percentage of acceptor moieties that are glycosylated by the glycosyltransferase of interest (e.g., fucosyltransferase). For example, in the case of a α1,2 fucosyltransferase, a substantially uniform fucosylation pattern exists if substantially all (as defined below) of the Galβ1,4-GlcNAc-R and sialylated analogues thereof are fucosylated in a peptide conjugate of the invention. It will be understood by one of skill in the art, that the starting material may contain glycosylated acceptor moieties (e.g., fucosylated Galβ1,4-GlcNAc-R moieties). Thus, the calculated percent glycosylation will include acceptor moieties that are glycosylated by the methods of the invention, as well as those acceptor moieties already glycosylated in the starting material.

The term "substantially" in the above definitions of "substantially uniform" generally means at least about 40%, at least about 70%, at least about 80%, or more preferably at least about 90%, and still more preferably at least about 95% of the acceptor moieties for a particular glycosyltransferase are glycosylated.

Where substituent groups are specified by their conventional chemical formulae, written from left to right, they equally encompass the chemically identical substituents, which would result from writing the structure from right to left, *e.g.,* -CH₂O- is intended to also recite -OCH₂-.

The term "alkyl," by itself or as part of another substituent means, unless otherwise stated, a straight or branched chain, or cyclic hydrocarbon radical, or combination thereof, which may be fully saturated, mono- or polyunsaturated and can include di- and multivalent radicals, having the number of carbon atoms designated *(i.e.* C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1-and 3-propynyl, 3-butynyl, and the higher homologs and isomers. The term "alkyl," unless otherwise noted, is also meant to include those derivatives of alkyl defined in more detail below, such as "heteroalkyl." Alkyl groups that are limited to hydrocarbon groups are termed "homoalkyl".

The term "alkylene" by itself or as part of another substituent means a divalent radical derived from an alkane, as exemplified, but not limited, by -CH₂CH₂CH₂CH₂-, and further includes those groups described below as "heteroalkylene." Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a stable straight or branched chain, or cyclic hydrocarbon radical, or combinations thereof, consisting of the stated number of carbon atoms and at least one heteroatom selected from the group consisting of 0, N, Si and S, and wherein the nitrogen and sulfur atoms may optionally be oxidized and the nitrogen heteroatom may optionally be quaternized. The heteroatom(s) O, N and S and Si may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃, -CH₂-CH₂-N(CH₃)-CH₃, - CH₂-S-CH₂-CH₃, -CH₂-CH₂,-S(O)-CH₃, -CH₂-CH₂-S(O)₂-CH₃, -CH=CH-O-CH₃, - Si(CH₃)₃, -CH₂-CH=N-OCH₃, and -CH=CH-N(CH₃)-CH₃. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (*e.g*., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-.

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is mean to include, but not be limited to, trifluoromethyl, 2,2,2-trifluoroethyl, 4-chlorobutyl, 3-bromopropyl, and the like.

The term "aryl" means, unless otherwise stated, a polyunsaturated, aromatic, substituent that can be a single ring or multiple rings (preferably from 1 to 3 rings), which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quatemized. A heteroaryl group can be attached to the remainder of the molecule through a heteroatom. Non-limiting examples of aryl and heteroaryl groups include phenyl, 1-naphthyl, 2-naphtliyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, tetrazolyl, benzo[b]furanyl, benzo[b]thienyl, 2,3-dihydrobenzo[1,4]dioxin-6-yl, benzo[1,3]dioxol-5-yl and 6-quinolyl. Substituents for each of the above noted aryl and heteroaryl ring systems are selected from the group of acceptable substituents described below.

For brevity, the term "aryl" when used in combination with other terms (e.g., aryloxy, arylthioxy, arylalkyl) includes both aryl and heteroaryl rings as defined above. Thus, the term "arylalkyl" is meant to include those radicals in which an aryl group is attached to an alkyl group (*e.g*., benzyl, phenethyl, pyridylmethyl and the like) including those alkyl groups in which a carbon atom (*e.g.,* a methylene group) has been replaced by, for example, an oxygen atom (*e.g.*, phenoxymethyl, 2-pyridyloxymethyl, 3-(1-naphthyloxy)propyl, and the like).

Each of the above terms (*e.g*., "alkyl," "heteroalkyl," "aryl" and "heteroaryl") is meant to include both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are generically referred to as "alkyl group substituents," and they can be one or more of a variety of groups selected from, but not limited to: -OR', =O, =NR', =N-OR', -NR'R", -SR', - halogen, -SiR'R"R"', -OC(O)R', -C(O)R', -CO₂R' -CONR'R", -OC(O)NR'R",-NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -CN and-NO₂ in a number ranging from zero to (2m'+1), where m' is the total number of carbon atoms in such radical. R', R", R"' and R"" each preferably independently refer to hydrogen, substituted or unsubstituted heteroalkyl, substituted or unsubstituted aryl, e.g., aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R"' and R"" groups when more than one of these groups is present. When R' and R" are attached to the same nitrogen atom, they can be combined with the nitrogen atom to form a 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl *(e.g.,* -CF₃ and -CH₂CF₃) and acyl (e.g., -C(O)CH₃, -C(O)CF₃, -C(O)CH₂OCH₃, and the like).

Similar to the substituents described for the alkyl radical, substituents for the aryl and heteroaryl groups are generically referred to as "aryl group substituents." The substituents are selected from, for example: halogen, -OR', =O, =NR', =N-OR', - NR'R", -SR', -halogen, -SiR'R"R"', -OC(O)R', -C(O)R', -CO₂R', -CONR'R", - OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)2R', -S(O)₂NR'R", -NRSO₂R', -CN and-NO₂, -R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R'" and R"" are preferably independently selected from hydrogen, substituted or unsubstituted alkyl, substituted or unsubstituted heteroalkyl, substituted or unsubstituted aryl and substituted or unsubstituted heteroaryl. When a compound of the invention includes more than one R group, for example, each of the R groups is independently selected as are each R', R", R'" and R"" groups when more than one of these groups is present. In the schemes that follow, the symbol X represents "R" as described above.

Two of the substituents on adjacent atoms of the aryl or heteroaryl ring may optionally be replaced with a substituent of the formula -T-C(O)-(CRR')_{q}-U-, wherein T and U are independently -NR-, -O-, -CRR'- or a single bond, and q is an integer of from 0 to 3. Alternatively, two of the substituents on adjacent atoms of the aryl or heteroaryl ring may optionally be replaced with a substituent of the formula - A-(CH₂)ᵣ-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula - (CRR')ₛ-X-(CR"R"')_{d}-, where s and d are independently integers of from 0 to 3, and X is -O-, -NR'-, -S-, -S(O)-, -S(O)₂-, or -S(O)₂NR'-. The substituents R, R', R" and R'" are preferably independently selected from hydrogen or substituted or unsubstituted (C₁-C₆)alkyl.

As used herein, the term "heteroatom" is meant to include oxygen (O), nitrogen (N), sulfur (S) and silicon (Si).

### Introduction

The present invention encompasses a method for the modification of the glycan structure on G-CSF. G-CSF is well known in the art as a cytokine produced by activated T-cells, macrophages, endothelial cells, and stromal fibroblasts. G-CSF primarily acts on the bone marrow to increase the production of inflammatory leukocytes, and further functions as an endocrine hormone to initiate the replenishment of neutrophils consumed during inflammatory functions. G-CSF also has clinical applications in bone marrow replacement following chemotherapy.

The present invention provides a conjugate of granulocyte colony stimulating factor (G-CSF). The invention provides conjugates of glycosylated and unglycosylated peptides having granulocyte colony stimulating activity. The conjugates may be additionally modified by further conjugation with diverse species such as therapeutic moieties, diagnostic moieties, targeting moieties and the like.

The present invention further includes a method for remodeling and/or modifying G-CSF. G-CSF is a valuable tool in treatment of numerous diseases, but as stated above, its clinical efficacy has been hampered by its relatively poor pharmacokinetics

AG-CSF peptide of the invention may be administered to patients for the purposed of preventing infection in cancer patients undergoing certain types of radiation therapy, chemotherapy, and bone marrow transplantations, to mobilize progenitor cells for collection in peripheral blood progenitor cell transplantations, for treatment of severe chronic or relative leukopenia, irrespective of cause, and to support treatment of patients with acute myeloid leukaemia. Additionally, the polypeptide conjugate or composition of the invention may be used for treatment of AIDS or other immunodeficiency diseases as well as bacterial infections.

G-CSF has been cloned and sequenced. In an exemplary embodiment, G-CSF has an the amino acid sequence according to SEQ ID NO:1.

Thus, the present invention further encompasses G-CSF variants, as well known in the art. As an example, a G-CSF variant has been described in U.S. Patent No. 6,166,183, in which a G-CSF comprising the natural complement of lysine residues and further linked to one or two polyethylene glycol molecules is described. Additionally, U.S. Patent Nos. 6,004,548, 5,580,755, 5,582,823, and 5,676,941 describe a G-CSF variant in which one or more of the cysteine residues at position 17, 36, 42, 64, and 74 are replaced by alanine or alternatively serine. U.S. Patent No. 5,416,195 describes a G-CSF molecule in which the cysteine at position 17, the aspartic acid at position 27, and the serines at positions 65 and 66 are substituted with serine, serine, proline, and proline, respectively. Other variants are well known in the art, and are described in, for example, U.S. Patent No. 5,399,345. Still further variants have an amino acid selected from SEQ ID Nos:3-11.

The expression and activity of a modified G-CSF molecule of the present invention can be assayed using methods well known in the art, and as described in, for example, U.S. Patent No. 4,810,643. As an example, activity can be measured using radio-labeled thymidine uptake assays. Briefly, human bone marrow from healthy donors is subjected to a density cut with Ficoll-Hypaque (1.077 g/ml, Pharmacia, Piscataway, NJ) and low density cells are suspended in Iscove's medium (GIBCO, La Jolla, CA) containing 10% fetal bovine serum, glutamine and antibiotics. About 2 x 10⁴ human bone marrow cells are incubated with either control medium or the G-CSF or the present invention in 96-well flat bottom plates at about 37° C in 5% CO₂ in air for about 2 days. Cultures are then pulsed for about 4 hours with 0.5 µCi/wel) of ³H-thymidine (New England Nuclear, Boston, Mass.) and uptake is measured as described in, for example, Ventua, et al.(1983, Blood 61:781). An increase in ³H-thymidine incorporation into human bone marrow cells as compared to bone marrow cells treated with a control compound is an indication of a active and viable G-CSF compound.

As discussed above, the conjugates of the invention are formed by the enzymatic attachment of a modified sugar to the glycosylated or unglycosylated G-CSF peptide. The modified sugar, when interposed between the G-CSF peptide and the modifying group on the sugar becomes what may be referred to herein e.g., as an "intact glycosyl linking group." Using the exquisite selectivity of enzymes, such as glycosyltransferases, the present method provides peptides that bear a desired group at one or more specific locations. Thus, according to the present invention, a modified sugar is attached directly to a selected locus on the G-CSF peptide chain or, alternatively, the modified sugar is appended onto a carbohydrate moiety of a glycopeptide. Peptides in which modified sugars are bound to both a glycopeptide carbohydrate and directly to an amino acid residue of the G-CSF peptide backbone are also within the scope of the present invention.

In contrast to known chemical and enzymatic peptide elaboration strategies, the methods of the invention, make it possible to assemble peptides and glycopeptides that have a substantially homogeneous derivatization pattern; the enzymes used in the invention are generally selective for a particular amino acid residue or combination of amino acid residues of the G-CSF peptide. The methods are also practical for large-scale production of modified peptides and glycopeptides. Thus, the methods of the invention provide a practical means for large-scale preparation of glycopeptides having preselected uniform derivatization patterns. The methods are particularly well suited for modification of therapeutic peptides, including but not limited to, glycopeptides that are incompletely glycosylated during production in cell culture cells (e.g., mammalian cells, insect cells, plant cells, fungal cells, yeast cells, or prokaryotic cells) or transgenic plants or animals.

The present invention also provides conjugates of glycosylated and unglycosylated G-CSF peptides with increased therapeutic half-life due to, for example, reduced clearance rate, or reduced rate of uptake by the immune or reticuloendothelial system (RES). Moreover, the methods of the invention provide a means for masking antigenic determinants on peptides, thus reducing or eliminating a host immune response against the peptide. Selective attachment of targeting agents can also be used to target a peptide to a particular tissue or cell surface receptor that is specific for the particular targeting agent.

### The Conjugates

In a first aspect, the present invention provides a conjugate between a selected modifying group and a G-CSF peptide.

The link between the G-CSF peptide and the selected moiety includes an intact glycosyl linking group interposed between the peptide and the selected moiety. As discussed herein, the selected moiety is essentially any species that can be attached to a saccharide unit, resulting in a "modified sugar" that is recognized by an appropriate transferase enzyme, which appends the modified sugar onto the G-CSF peptide. The saccharide component of the modified sugar, when interposed between the G-CSF peptide and a selected moiety, becomes an "intact glycosyl linking group." The glycosyl linking group is formed from any mono- or oligo-saccharide that, after modification with a selected moiety, is a substrate for an appropriate transferase.

The conjugates of the invention are described in the claims and will typically correspond to the general structure: 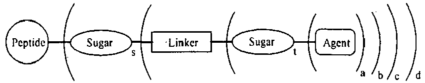 in which the symbols a, b, c, d and s represent a positive, non-zero integer; and t is either 0 or a positive integer. The "agent" is typically a water-soluble moiety, e.g., a PEG moiety. The linker can be any of a wide array of linking groups, *infra.* Alternatively, the linker may be a single bond or a "zero order linker."

In an exemplary embodiment, the selected modifying group is a water-soluble polymer, e.g., m-PEG. The water-soluble polymer is covalently attached to the G-CSF peptide via a glycosyl linking group, which is covalently attached to an amino acid residue or a glycosyl residue of the G-CSF peptide. The invention also provides conjugates in which an amino acid residue and a glycosyl residue are modified with a glycosyl linking group.

An exemplary water-soluble polymer is poly(ethylene glycol), e.g., methoxy-poly(ethylene glycol). The poly(ethylene glycol) used in the present invention is not restricted to any particular molecular weight range.

The poly(ethylene glycol) of the conjugates of the invention is a branched PEG having more than one PEG moiety attached. Examples of branched PEGs are described in U.S. Pat. No. 5,932,462; U.S. Pat. No. 5,342,940; U.S. Pat. No. 5,643,575; U.S. Pat. No. 5,919,455; U.S. Pat. No. 6,113,906; U.S. Pat. No. 5,183,660; WO 02/09766; Kodera Y., Bioconjugate Chemistry 5: 283-288 (1994); and Yamasaki et al., Agric. Biol. Chem., 52: 2125-2127, 1998. The branched PEG structures of the invention are disclosed herein.

In an exemplary embodiment the molecular weight of each poly(ethylene glycol) of the branched PEG is equal to or greater than about 2,000, 5,000, 10,000, 15,000, 20,000, 40,000 or 60,000 daltons.

The peptides of the present invention include at least one N- or O-linked glycosylation site. In addition to providing conjugates that are formed through an enzymatically added glycosyl linking group, the present invention provides conjugates that are highly homogenous in their substitution patterns. Using the methods of the invention, it is possible to form peptide conjugates in which essentially all of the modified sugar moieties across a population of conjugates of the invention are attached to multiple copies of a structurally identical amino acid or glycosyl residue. Thus, in a second aspect, the invention provides a peptide conjugate having a population of water-soluble polymer moieties, which are covalently bound to the G-CSF peptide through an intact glycosyl linking group. In a preferred conjugate of the invention, essentially each member of the population is bound via the glycosyl linking group to a glycosyl residue of the G-CSF peptide, and each glycosyl residue of the G-CSF peptide to which the glycosyl linking group is attached has the same structure.

Also provided is a peptide conjugate having a population of water-soluble polymer moieties covalently bound thereto through a glycosyl linking group. In a preferred embodiment, essentially every member of the population of water soluble polymer moieties is bound to an amino acid residue of the G-CSF peptide via a glycosyl linking group, and each amino acid residue having a glycosyl linking group attached thereto has the same structure.

Also described are conjugates analogous to those described above in which the G-CSF peptide is conjugated to a therapeutic moiety, diagnostic moiety, targeting moiety, toxin moiety or the like via an intact glycosyl linking group. Each of the above-recited moieties can be a small molecule, natural polymer (e.g., polypeptide) or synthetic polymer.

Essentially any Granulocyte Colony Stimulating Factor peptide or agent, having any sequence, is of use as the peptide component of the conjugates of the present invention. Granulocyte Colony Stimulating Factor has been cloned and sequenced. In an exemplary embodiment, the G-CSF peptide has the sequence presented in SEQ ID NO:1:

MTPLGPASSLPQSFLLKCLEQVRKIQGDGAALQEKL
CATYKLCHPEELVLLGHSLGIPWAPLSSCPSQALQLA
GCLSQLHSGLFLYQGLLQALEGISPELGPTLDTLQLD
VADFATTIWQQMEELGMAPALQPTQGAMPAFASAF
QRRAGGVLVASHLQSFLEVSYRVLRHLAQP (SEQ
ID NO: 1).

In another exemplary embodiment, the G-CSF peptide has the sequence presented in SEQ ID NO:2:

TPLGPASSLPQSFLLKCLEQVRKIQGDGAALQEKLCA
TYKLCHPEELVLLGHSLGIPWAPLSSCPSQALQLAGC
LSQLHSGLFLYQGLLQALEGISPELGPTLDTLQLDVA
DFATTIWQQMEELGMAPALQPTQGAMPAFASAFQR
RAGGVLVASHLQSFLEVSYRVLRHLAQP (SEQ ID
NO: 2).

In other exemplary embodiments, the G-CSF peptide has a sequence presented in SEQ ID Nos:3-11, below. 
MTPLGPASSLPQSFLLKCLEQVRKIQGDGAALQEKL
VSECATYKLCHPEELVLLGHSLGIPWAPLSSCPSQAL
QLAGCLSQLHSGLFLYQGLLQALEGISPELGPTLDTL
QLDVADFATTIWQQMEELGMAPALQPTQGAMPAFA
SAFQRRAGGVLVASHLQSFLEVSYRVLRHLAQP
(SEQ ID NO:3)
 
MAGPATQSPMKLMALQLLLWHSALWTVQEATPLGP
ASSLPQSFLLKCLEQVRKIQGDGAALQEKLCATYKL
CHPEELVLLGHSLGIPWAPLSSCPSQALQLAGCLSQL
HSGLFLYQGLLQALEGISPELGPTLDTLQLDVADFAT
 
TIWQQMEELGMAPALQPTQGAMPAFASAFQRRAGG
VLVASHLQSFLEVSYRVLRHLAQP (SEQ ID NO:4)
 
MAGPATQSPMKLMALQLLLWHSALWTVQEATPLGP
ASSLPQSFLLKCLEQVRKIQGDGAALQEKLVSECAT
YKLCHPEELVLLGHSLGIPWAPLSSCPSQALQLAGCL
SQLHSGLFLYQGLLQALEGISPELGPTLDTLQLDVAD
FATTIWQQMEELGMAPALQPTQGAMPAFASAFQRR
AGGVLVASHLQSFLEVSYRVLRHLAQP (SEQ ID
NO:5)
 
MVTPLGPASSLPQSFLLKCLEQVRKIQGDGAALQEK
LCATYKLCHPEELVLLGHTLGIPWAPLSSCPSQALQL
AGCLSQLHSGLFLYQGLLQALEGISPELGPTLDTLQL
DVADFATTIWQQMEELGMAPALQPTQGAMPAFASA
FQRRAGGVLVASHLQSFLEVSYRVLRHLAQP(SEQ
ID NO:6);
 
MTPLGPASSLPQSFLLKCLEQVRKIQGDGAALQEKL
CATYKLCHPEELVLLGHTLGIPWAPLSSCPSQALQLA
GCLSQLHSGLFLYQGLLQALEGISPELGPTLDTLQLD
VADFATTIWQQMEELGMAPALQPTQGAMPAFASAF
QRRAGGVLVASHLQSFLEVSYRVLRHLAQP(SEQ ID
NO:7);
 
MVTPLGPASSLPQSFLLKCLEQVRKIQGDGAALQEK
LCATYKLCHPEELVLLGSSLGIPWAPLSSCPSQALQL
AGCLSQLHSGLFLYQGLLQALEGISPELGPTLDTLQL
DVADFATTIWQQMEELGMAPALQPTQGAMPAFASA
FQRRAGGVLVASHLQSFLEVSYRVLRHLAQP(SEQ
ID NO:8);
 
MQTPLGPASSLPQSFLLKCLEQVRKIQGDGAALQEK
LCATYKLCHPEELVLLGHSLGIPWAPLSSCPSQALQL
AGCLSQLHSGLFLYQGLLQALEGISPELGPTLDTLQL
DVADFATTIWQQMEELGMAPALQPTQGAMPAFASA
FQRRAGGVLVASHLQSFLEVSYRVLRHLAQP(SEQ
ID NO:9);
 
MTPLGPASSLPQSFLLKCLEQVRKIQGDGAALQEKL
CATYKLCHPEELVLLGHSLGIPWAPLSSCPSQALQLA
GCLSQLHSGLFLYQGLLQALEGISPELGPTLDTLQLD
VADFATTIWQQMEELGMAPALQPTQGAMPAFASAF
QRRAGGVLVASHLQSFLEVSYRVLRHLAQPTQGAM
P; (SEQ ID NO:10)
 and

MTPLGPASSLPQSFLLKCLEQVRKIQGDGAALQEKL
CATYKLCHPEEL VLLGSSLGIPWAPLSSCPSQALQLA
GCLSQLHSGLFL YQGLLQALEGISPELGPTLDTLQLD
VADFATTIWQQMEELGMAPTTTPTQTAMPAFASAF
QRRAGGVLVASHLQSFLEVS YRVLRHLAQP(SEQ ID
NO:11)

In an exemplary embodiment, the G-CSF peptides of the invention include at least one O-linked glycosylation site, which is glycosylated with a glycosyl residue that includes a PEG moiety. The PEG is covalently attached to the G-CSF peptide via an intact glycosyl linking group. The glycosyl linking group is covalently attached to either an amino acid residue or a glycosyl residue of the G-CSF peptide. Alternatively, the glycosyl linking group is attached to one or more glycosyl units of a glycopeptide. The invention also provides conjugates in which the glycosyl linking group is attached to both an amino acid residue and a glycosyl residue.

The PEG moiety is attached to an intact glycosyl linker directly, or via a non-glycosyl linker, e.g., substituted or unsubstituted alkyl, substituted or unsubstituted heteroalkyl.

The G-CSF peptide comprises a moiety having the formula of Formula I. 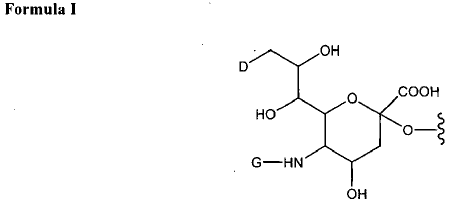 in which D is a member selected from -OH and R¹-L-HN-; G is a member selected from R¹-L- and-C(O)(C₁-C₆)alkyl; R¹ is a moiety as described in the claims and L is a linker which is a member selected from a bond, substituted or unsubstituted alkyl and substituted or unsubstituted heteroalkyl,such that when D is OH, G is R¹-L-, and when G is -C(O)(C₁-C₆)alkyl, D is R¹-L-NH-. In the modified sialic acid structures set forth herein, COOH also represents COO' and/or a salt thereof.

In one embodiment, a R¹-L has the formula: 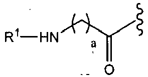 wherein a is an integer from 0 to 20.

R¹ has a structure that is a member selected from: 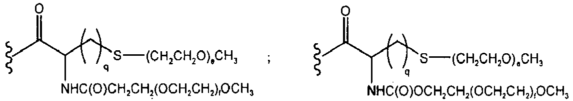 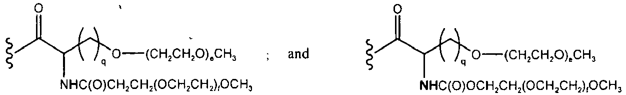 wherein e and fare integers independently selected from 1 to 2500; and q is an integer from 1 to 20. or R¹ has a structure that is a member selected from: 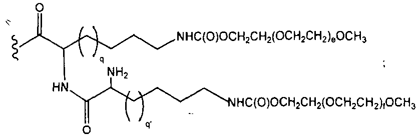 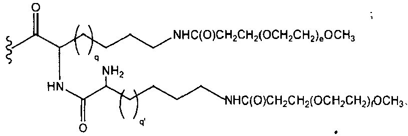 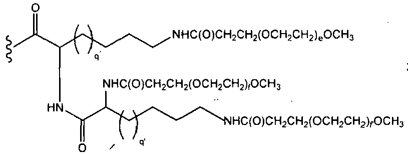 and 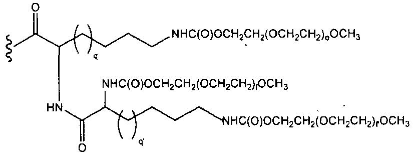 wherein e, f and f are integers independently selected from 1 to 2500; and q and q' are integers independently selected from 1 to 20.

or the invention provides a G-CSF peptide conjugate wherein R¹ has a structure that is a member selected from: 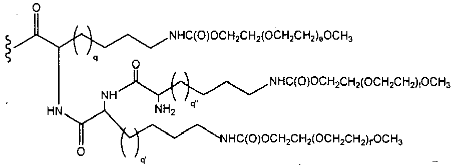 and 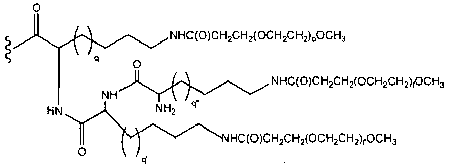 wherein e, f and f' are integers independently selected from 1 to 2500; and q, q' and q" are integers independently selected from 1 to 20.

In another exemplary embodiment, the invention provides a peptide comprising a moiety having the formula: 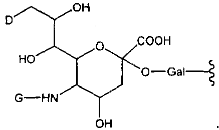 The Gal can be attached to an amino acid or to a glycosyl residue that is directly or indirectly (e.g., through a glycosyl residue) attached to an amino acid.

In other embodiments, the moiety has the formula: 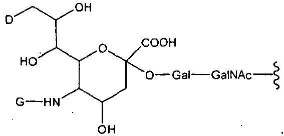 The GalNAc can be attached to an amino acid or to a glycosyl residue that is directly or indirectly (e.g., through a glycosyl residue) attached to an amino acid.

In a still further exemplary embodiment the peptide comprises a moiety according to the formula 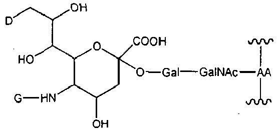 wherein AA is an amino acid residue of said peptide and, in each of the above structures, D and G are as described herein.

An exemplary amino acid residue of the G-CSF peptide at which one or more of the above species can be conjugated include serine and threonine, e.g., threonine 133 of SEQ. ID. NO.: 1.

In another exemplary embodiment, the invention provides a G-CSF conjugate that includes a glycosyl residue having the formula: 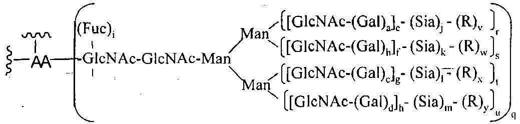 wherein a, b, c, d, i, r, s, t, and u are integers independently selected from 0 and 1. The index q is 1. The indices e, f, g, and h are independently selected from the integers from 0 to 6. The indices j, k, I, and m are independently selected from the integers from 0 and 100. The indices v, w, x, and y are independently selected from 0 and I, and at least one of v, w, x and y is 1. The symbol AA represents an amino acid residue of the G-CSF peptide.

The symbol Sia-(R) represents a group that has the formula: 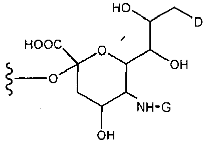 wherein D is selected from -OH and R¹-L-HN-. The symbol G is represents R¹-L- or -C(O)(C₁-C₆)alkyl. R¹ represents a moiety as described in the claims . L is a linker which is a member selected from a bond, substituted or unsubstituted alkyl and substituted or unsubstituted heteroalkyl. In general, when D is OH, G is R¹-L-, and when G is -C(O)(C₁-C₆)alkyl, D is R¹-L-NH-.

In another exemplary embodiment, the PEG-modified sialic acid moiety in the conjugate of the invention has the formula: 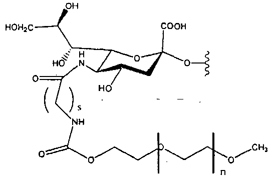 in which the index "s" represents an integer from 0 to 20, and n is an integer from-1 to 2500. In a preferred embodiment, s is equal to 1; and the m-PEG moiety has a molecular weight of about 20 kD.

In a preferred embodiment, at least two, more preferably three, more preferrably four of the above-named asparagine residues is functionalized with the N-linked glycan chain shown above.

The conjugates of the invention include intact glycosyl linking groups that are mono- or multi-valent (e.g., antennary structures). Thus, conjugates of the invention include both species in which a selected moiety is attached to a peptide via a monovalent glycosyl linking group and a multivalent linking group. Also included within the invention are conjugates in which more than one selected moiety is attached to a peptide via a multivalent linking group.

### Modified Sugars

The present invention provides modified sugars, modified sugar nucleotides and conjugates of the modified sugars. In modified sugar compounds of the invention, the sugar moiety is preferably a saccharide, a deoxy-saccharide, an amino-saccharide, or an N-acyl saccharide. The term "saccharide" and its equivalents, "saccharyl," "sugar," and "glycosyl" refer to monomers, dimers, oligomers and polymers. The sugar moiety is also functionalized with a modifying group. The modifying group is conjugated to the sugar moiety, typically, through conjugation with an amine, sulfhydryl or hydroxyl, e.g., primary hydroxyl, moiety on the sugar. In an exemplary embodiment, the modifying group is attached through an amine moiety on the sugar, e.g., through an amide, a urethane or a urea that is formed through the reaction of the amine with a reactive derivative of the modifying group.

Any sugar can be utilized as the sugar core of the conjugates of the invention. Exemplary sugar cores that are useful in forming the compositions of the invention include, but are not limited to, glucose, galactose, mannose, fucose, and sialic acid. Other useful sugars include amino sugars such as glucosamine, galactosamine, mannosamine, the 5-amine analogue of sialic acid and the like. The sugar core can be a structure found in nature or it can be modified to provide a site for conjugating the modifying group. For example, in one embodiment, the invention provides a peptide conjugate comprising a sialic acid derivative in which the 9-hydroxy moiety is replaced with an amine. The amine is readily derivatized with an activated analogue of a selected modifying group.

In the discussion that follows the invention is illustrated by reference to the use of selected derivatives of sialic acid. Those of skill in the art will recognize that the focus of the discussion is for clarity of illustration and that the structures and compositions set forth are generally applicable across the genus of saccharide groups, modified saccharide groups, activated modified saccharide groups and conjugates of modified saccharide groups.

In an exemplary embodiment, the invention provides a peptide conjugate comprising a modified sugar amine that has the formula: 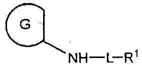 in which G is a glycosyl moiety, L is a bond or a linker and R¹ is the modifying group. Exemplary bonds are those that are formed between an NH₂ on the glycosyl moiety and a group of complementary reactivity on the modifying group. Thus, exemplary bonds include, but are not limited to NHR¹, OR¹, SR¹ and the like. For example, when R¹ includes a carboxylic acid moiety, this moiety may be activated and coupled with an NH₂ moiety on the glycosyl residue affording a bond having the structure NHC(O)R¹. Similarly, the OH and SH groups can be converted to the corresponding ether or thioether derivatives, respectively.

Exemplary linkers include alkyl and heteroalkyl moieties. The linkers include linking groups, for example acyl-based linking groups, *e.g.,* -C(O)NH-, -OC(O)NH-; and the like. The linking groups are bonds formed between components of the species of the invention, *e.g*., between the glycosyl moiety and the linker (L), or between the linker and the modifying group (R¹). Other linking groups are ethers, thioethers and amines. For example, in one embodiment, the linker is an amino acid residue, such as a glycine residue. The carboxylic acid moiety of the glycine is converted to the corresponding amide by reaction with an amine on the glycosyl residue, and the amine of the glycine is converted to the corresponding amide or urethane by reaction with an activated carboxylic acid or carbonate of the modifying group.

Another exemplary linker is a PEG moiety or a PEG moiety that is functionalized with an amino acid residue. The PEG is bound to the glycosyl group through the amino acid residue at one PEG terminus and bound to R¹ through the other PEG terminus. Alternatively, the amino acid residue is bound to R¹ and the PEG terminus not bound to the amino acid is bound to the glycosyl group.

An exemplary species for NH-L-R¹ has the formula:

-NH{C(O)(CH₂)ₐNH}ₛ{C(O)(CH₂)_{b}(OCH₂CH₂)_{c}O(CH₂)_{d}NH}ₜR¹,

in which the indices s and t are independently 0 or I. The indices a, b and d are independently integers from 0 to 20, and c is an integer from I to 2500. Other similar linkers are based on species in which the -NH moiety is replaced by, for example, -S, -O and - CH₂.

More particularly, the invention provides a peptide conjugate comprising compounds in which NH-L-R¹ is:
NHC(O)(CH₂)ₐNHC(O)(CH₂)_{b}(OCH₂CH₂)_{c}O(CH₂)_{d}NHR¹,
NHC(O)(CH₂)_{b}(OCH₂CH₂)_{c}O(CH₂)_{d}NHR¹,
NHC(O)O(CH₂)_{b}(OCH₂CH₂)_{c}O(CH₂)_{d}NHR¹,
NH(CH₂)ₐNHC(O)(CH₂)_{b}(OCH₂CH₂)_{c}O(CH₂)_{d}NHR¹, NHC(O)(CH₂)ₐNHR¹,
NH(CH₂)ₐNHR¹, and NHR¹. In these formulae, the indices a, b and d are independently selected from the integers from 0 to 20, preferably from 1 to 5. The index c is an integer from 1 to 2500.

In an illustrative embodiment, G is sialic acid and selected compounds of the invention have the formulae: 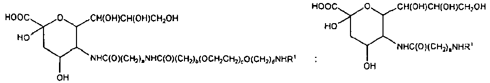 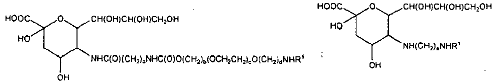 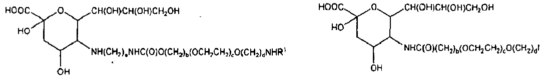 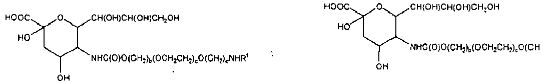 and

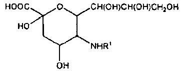

In another illustrative embodiment, a primary hydroxyl moiety of the sugar is functionalized with the modifying group. For example, the 9-hydroxyl of sialic acid can be converted to the corresponding amine and functionalized to provide a compound according to the invention. Formulae according to this embodiment include:

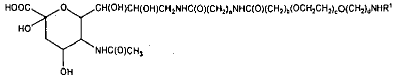

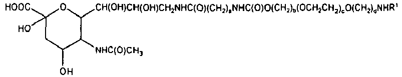

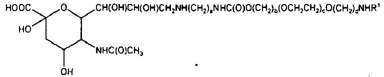

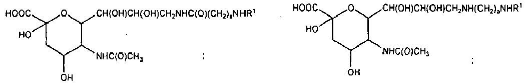

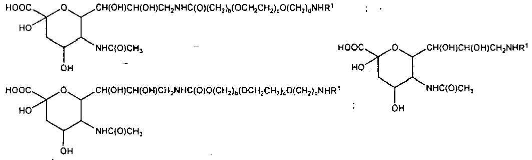

Also disclosed is a peptide conjugate comprising modified sugars in which the 6-hydroxyl position is converted to the corresponding amine moiety, which bears a linker-modifying group cassette such as those set forth above. Exemplary saccharyl groups that can be used as the core of these modified sugars include Gal, GalNAc, Glc, GlcNAc, Fuc, Xyl, Man, and the like. A representative modified sugar according to this structure has the formula: 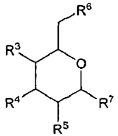 in which R³-R⁵ and R⁷ are members independently selected from H, OH, C(O)CH₃, NH, and NH C(O)CH₃. R⁶ is OR¹, NHR¹ or NH-L-R¹, which is as described above.

Also disclosed are conjugates based on mannose, galactose or glucose, or on species having the stereochemistry of mannose, galactose or glucose. The general formulae of these conjugates are:

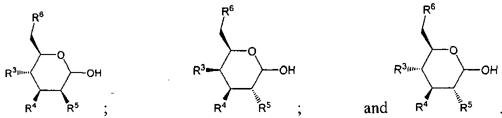

In another exemplary embodiment, the invention provides compounds as set forth above that are activated as the corresponding nucleotide sugars. Exemplary sugar nucleotides that are used in the present invention in their modified form include nucleotide mono-, di- or triphosphates or analogs thereof. In a preferred embodiment, the modified sugar nucleotide is selected from a UDP-glycoside, CMP-glycoside, or a GDP-glycoside. Even more preferably, the sugar nucleotide portion of the modified sugar nucleotide is CMP-sialic acid, In an exemplary embodiment, the nucleotide phosphate is attached to C-1.

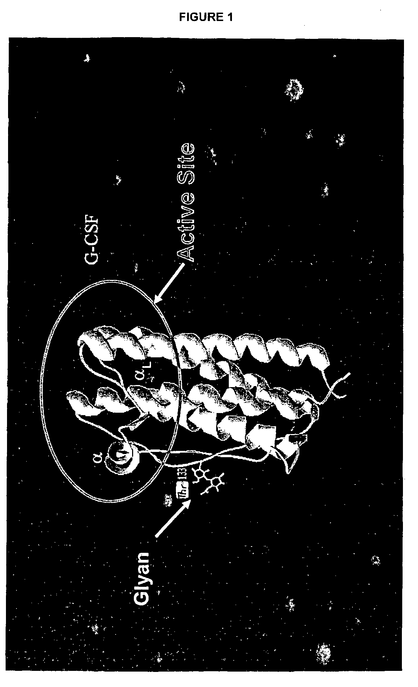

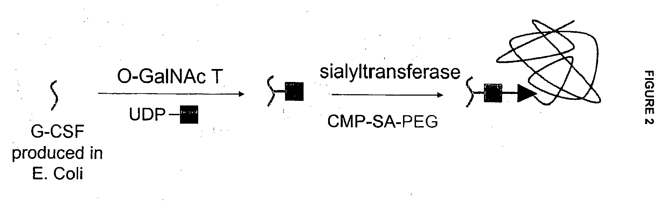

Thus, in an illustrative embodiment in which the glycosyl moiety is sialic acid, the invention provides peptide conjugates that are formed using compounds having the formulae: 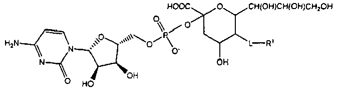 ; and 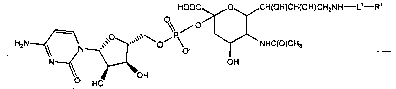 in which L-R¹ is as discussed above, and L¹-R¹ represents a linker bound to the modifying group. As with L, exemplary linker species according to L¹ include a bond, alkyl or heteroalkyl moieties. Exemplary modified sugar nucleotide compounds according to these embodiments are set forth in FIG. 1 and FIG. 2.

In another exemplary embodiment, the invention provides a conjugate formed between a modified sugar of the invention and a substrate, *e.g.,* a peptide, lipid, aglycone, etc., more particularly between a modified sugar and a glycosyl residue of a glycopeptide or a glycolipid. In this embodiment, the sugar moiety of the modified sugar becomes a glycosyl linking group interposed between the substrate and the modifying group. An exemplary glycosyl linking group is an intact glycosyl linking group, in which the glycosyl moiety or moieties forming the linking group are not degraded by chemical *(e.g.,* sodium metaperiodate) or enzymatic processes *(e.g.,* oxidase). Selected conjugates of the invention include a modifying group that is attached to the amine moiety of an amino-saccharide, *e.g.,* mannosamine, glucosamine, galactosamine, sialic acid etc. Exemplary modifying group-intact glycosyl linking group cassette according to this motif is based on a sialic acid structure, such as that having the formulae: 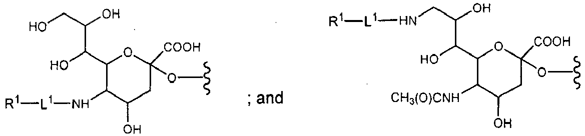 In the formulae above, R¹, L¹ and L² are as described above.

Also disclosed are the conjugates is formed between a substrate and the 1-position of a saccharyl moiety that in which the modifying group is attached through a linker at the 6-carbon position of the saccharyl moiety. conjugates according to this structure have the formulae: 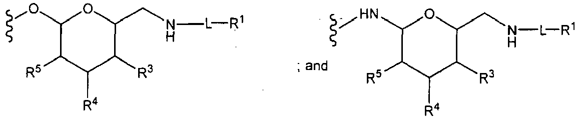 in which the radicals are as discussed above. Those of skill will appreciate that the modified saccharyl moieties set forth above can also be conjugated to a substrate at the 2, 3, 4, or 5 carbon atoms.

Illustrative compounds according to this structure include compounds having the formulae: 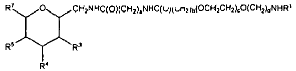 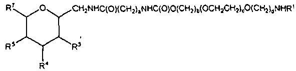 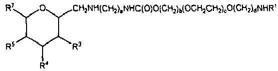 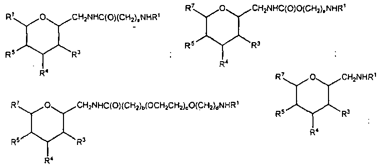 and 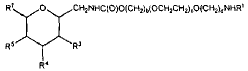 in which the R groups and the indices arc as described above.

Also described are sugar nucleotides modified with L-R¹ at the 6-carbon position. Exemplary species include: 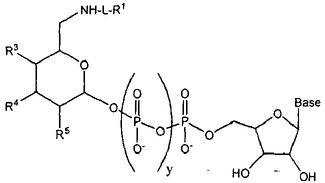 in which the R groups, and L, represent moieties as discussed above. The index "y" is 0, 1 or 2.

Also disclosed is a nucleotide sugare based on a species having the stereochemistry of GDP mannose. An exemplary species has the structure: 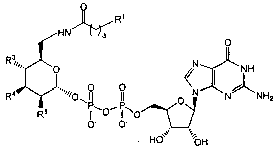 and

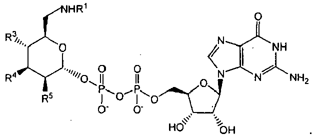

Further disclosed is a conjugate, based on the stereochemistry of UDP galactose. An exemplary compound has the structure: 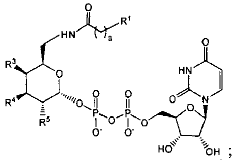 and

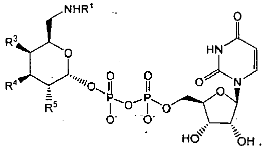

Further disclosed is a nucleotide sugar based on the stereochemistry of glucose. Exemplary species have the formulae: 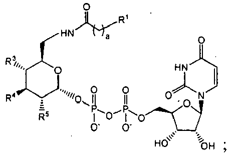 and

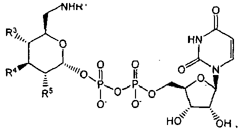

The modifying group, R¹, is any of a number of species including, but not limited to, water-soluble polymers, water-insoluble polymers, therapeutic agents, diagnostic agents and the like. The nature of exemplary modifying groups is discussed in greater detail hereinbelow.

### Modifying Groups

### Water-Soluble Polymers

Many water-soluble polymers are known to those of skill in the art and are useful in practicing the present invention. The term water-soluble polymer encompasses species such as saccharides (*e.g*., dextran, amylose, hyalouronic acid, poly(sialic acid), heparans, heparins, etc.); poly (amino acids), *e.g*., poly(aspartic acid) and poly(glutamic acid); nucleic acids; synthetic polymers (*e.g*., poly(acrylic acid), poly(ethers), *e.g*., poly(ethylene glycol); peptides, proteins, and the like.

Methods for activation of polymers can also be found in WO 94/17039, U.S. Pat. No. 5,324,844, WO 94/18247, WO 94/04193, U.S. Pat. No. 5,219,564, U.S. Pat. No. 5,122,614, WO 90/13540, U.S. Pat. No. 5,281,698, and more WO 93/15189, and for conjugation between activated polymers and peptides, *e.g.* Coagulation Factor VIII (WO 94/15625), hemoglobin (WO 94/09027), oxygen carrying molecule (U.S. Pat. No. 4,412,989), ribonuclease and superoxide dismutase (Veronese at al., App. Biochem. Biotech. 11: 141-45 (1985)).

Preferred water-soluble polymers are those in which a substantial proportion of the polymer molecules in a sample of the polymer are of approximately the same molecular weight; such polymers are "homodisperse."

The present invention is further illustrated by reference to a poly(ethylene glycol) conjugate. Several reviews and monographs on the functionalization and conjugation of PEG are available. *See*, for example, Harris, Macronol. Chem. Phys. C25: 325-373 (1985); Scouten, Methods in Enzymology 135: 30-65 (1987); Wong et al., Enzyme Microb. Technol. 14: 866-874 (1992); Delgado et al., Critical Reviews in Therapeutic Drug Carrier Systems 9: 249-304 (1992); Zalipsky, Bioconjugate Chem. 6: 150-165 (1995); and Bhadra, et al., Pharmazie, 57:5-29 (2002). Routes for preparing reactive PEG molecules and forming conjugates using the reactive molecules are known in the art. For example, U.S. Patent No. 5,672,662 discloses a water soluble and isolatable conjugate of an active ester of a polymer acid selected from linear or branched poly(alkylene oxides), poly(oxyethylated polyols), poly(olefinic alcohols), and poly(acrylomorpholine).

U.S. Patent No. 6,376,604 sets forth a method for preparing a water-soluble 1-benzotriazolylcarbonate ester of a water-soluble and non-peptidic polymer by reacting a terminal hydroxyl of the polymer with di(1-benzotriazoyl)carbonate in an organic solvent. The active ester is used to form conjugates with a biologically active agent such as a protein or peptide.

WO 99/45964 describes a conjugate comprising a biologically active agent and an activated water soluble polymer comprising a polymer backbone having at least one terminus linked to the polymer backbone through a stable linkage, wherein at least one terminus comprises a branching moiety having proximal reactive groups linked to the branching moiety, in which the biologically active agent is linked to at least one of the proximal reactive groups. Other branched poly(ethylene glycols) are described in WO 96/21469, U.S. Patent No. 5,932,462 describes a conjugate formed with a branched PEG molecule that includes a branched terminus that includes reactive functional groups. The free reactive groups are available to react with a biologically active species, such as a protein or peptide, forming conjugates between the poly(ethylene glycol) and the biologically active species. U.S. Patent No. 5,446,090 describes a bifunctional PEG linker and its use in forming conjugates having a peptide at each of the PEG linker termini.

Conjugates that include degradable PEG linkages are described in WO 99/34833; and WO 99/14259, as well as in U.S. Patent No. 6,348,558.

The art-recognized methods of polymer activation set forth above are of use in the context of the present invention in the formation of the branched polymers set forth herein and also for the conjugation of these branched polymers to other species, *e.g.,* sugars, sugar nucleotides and the like.

Exemplary poly(ethylene glycol) molecules described herein include those having the formula: 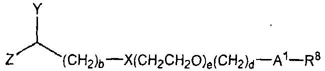 in which R⁸ is H, OH, NH₂, substituted or unsubstituted alkyl, substituted or unsubstituted aryl, substituted or unsubstituted heteroaryl, substituted or unsubstituted heterocycloalkyl, substituted or unsubstituted heteroalkyl, *e.g*., acetal, OHC-, H₂N-(CH₂)_{q}-, HS-(CH₂)_{q}, or -(CH₂)_{q}C(Y)Z¹. The index "e" represents an integer from I to 2500. The indices b, d, and q independently represent integers from 0 to 20. The symbols Z and Z¹ independently represent OH, NH₂, leaving groups, e.g., imidazole, p-nitrophenyl, HOBT, tetrazole, halide, S-R⁹, the alcohol portion of activated esters; -(CH₂)pC(Y¹)V, or -(CH₂)ₚU(CH₂)ₛC(Y¹)ᵥ. The symbol Y represents H(2), =O, =S, =N-R¹⁰. The symbols X, Y, Y¹, A¹, and U independently represent the moieties O, S, N-R¹¹. The symbol V represents OH, NH₂, halogen, S-R¹², the alcohol component of activated esters, the amine component of activated amides, sugar-nucleotides, and proteins. The indices p, q, s and v are members independently selected from the integers from 0 to 20. The symbols R⁹, R¹⁰, R¹¹ and R¹² independently represent H, substituted or unsubstituted alkyl, substituted or unsubstituted heteroalkyl, substituted or unsubstituted aryl, substituted or unsubstituted heterocycloalkyl and substituted or unsubstituted heteroaryl.

Also described are poly(ethylene glycol) molecule is selected from the following:

| | |
|---|---|
| 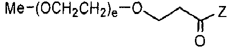 | 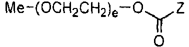 |
| 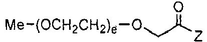 | 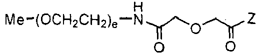 |
| 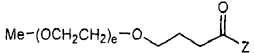 | 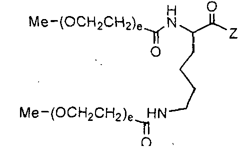 |
| Me-(OCH₂CH₂)ₑ-S-Z | |
| 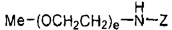 | |

Such that the peptide conjugates of the claims can be formed.

The poly(ethylene glycol) useful in forming the conjugate of the invention is branched. Branched poly(ethylene glycol) molecules suitable for use in the invention include those described by the following formula: 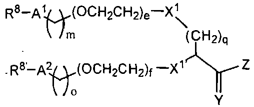 in which R⁸ and R^{8'} are members independently selected from the groups defined for R⁸, above. A¹ and A² are members independently selected from the groups defined for A¹, above. The indices e, f, o, and q are as described above. Z and Y are as described above. X¹ and X^{1'} are members independently selected from S, SC(O)NH, HNC(O)S, SC(O)O, O, NH, NHC(O), (O)CNH and NHC(O)O, OC(O)NH, such that on the compounds of the invention can be formed.

The branched PEG is based upon a cysteine, serine or di-lysine core. Thus exemplary branched PEGs include:

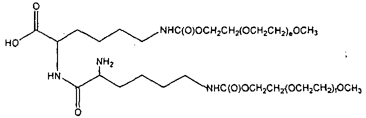

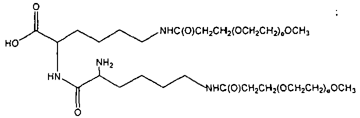

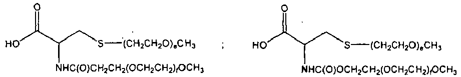

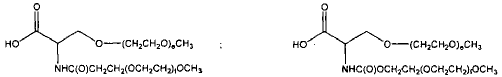

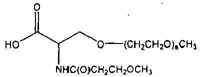

In yet another embodiment, the branched PEG moiety is based upon a tri-lysine peptide. The tri-lysine can be mono-, di-, tri-, or tetra-PEG-ylated. Exemplary species according to this embodiment have the formulae: 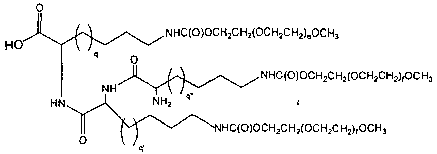 and 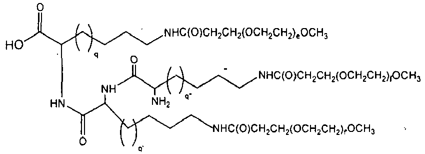 in which e, f and f are independently selected integers from 1 to 2500; and q, q' and q" are independently selected integers from 1 to 20.

In exemplary embodiments of the invention, the PEG is m-PEG (5 kD, 10 kD, or 20kD). An exemplary branched PEG species is a serine- or cysteine-(m-PEG)₂ in which the m-PEG is a 20 kD m-PEG.

As will be apparent to those of skill, the branched polymers of use in the invention include variations on the themes set forth above. For example the di-lysine-PEG conjugate shown above can include three polymeric subunits, the third bonded to the α-amine shown as unmodified in the structure above. Similarly, the use of a tri-lysine functionalized with three or four polymeric subunits is within the scope of the invention.
specific embodiments according to the invention include: 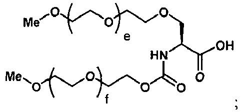 and carbonates and active esters of these species, such as: 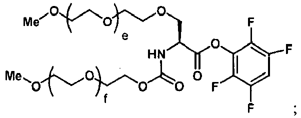 and

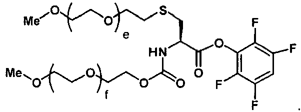

Other activating, or leaving groups, appropriate for activating linear PEGs of use in preparing the compounds set forth herein include, but are not limited to the species:

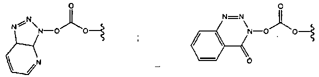

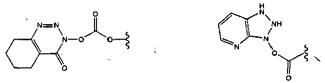

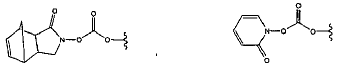

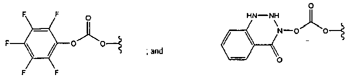

PEG molecules that are activated with these and other species and methods of making the activated PEGs are set forth in WO 04/083259.

Those of skill in the art will appreciate that one or more of the m-PEG arms of the branched polymer can be replaced by a PEG moiety with a different terminus, e.g., OH, COOH, NH₂, C₂-C₁₀-alkyl, etc. Moreover, the structures above are readily modified by inserting alkyl linkers (or removing carbon atoms) between the α-carbon atom and the functional group of the side chain. Thus, "homo" derivatives and higher homologues, as well as lower homologues are within the scope of cores for branched PEGs of use in the present invention.

The branched PEG species set forth herein are readily prepared by methods such as that set forth in the scheme below: 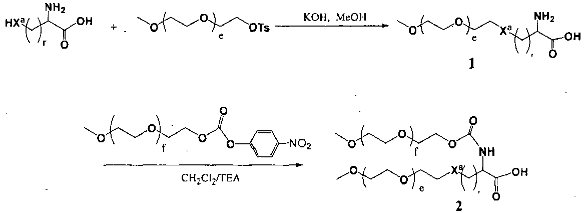 in which X^{a} is O or S and r is an integer from 1 to 5. The indices e and f are independently selected integers from 1 to 2500.

Thus, according to this scheme, a natural or unnatural amino acid is contacted with an activated m-PEG derivative, in this case the tosylate, forming 1 by alkylating the side-chain heteroatom X^{a}. The mono-functionalized m-PEG amino acid is submitted to N-acylation conditions with a reactive m-PEG derivative, thereby assembling branched m-PEG 2. As one of skill will appreciate, the tosylate leaving group can be replaced with any suitable leaving group, e.g., halogen, mesylate, triflate, etc. Similarly, the reactive carbonate utilized to acylate the amine can be replaced with an active ester, e.g., N-hydroxysuccinimide, etc., or the acid can be activated *in situ* using a dehydrating agent such as dicyclohexylcarbodiimide, carbonyldiimidazole, etc.

In this invention, the modifying group is a PEG moiety, however, any modifying group, *e.g.,* water-soluble polymer, water-insoluble polymer, therapeutic moiety, etc., can be incorporated in a glycosyl moiety through an appropriate linkage. The modified sugar is formed by enzymatic means, chemical means or a combination thereof, thereby producing a modified sugar. In an exemplary embodiment, the sugars are substituted with an active amine at any position that allows for the attachment of the modifying moiety, yet still allows the sugar to function as a substrate for an enzyme capable of coupling the modified sugar to the G-CSF peptide. For example, when galactosamine is the modified sugar, the amine moiety can be attached to the carbon atom at the 6-position.

### Water-soluble Polymer Modified Species

Water-soluble polymer modified nucleotide sugar species in which the sugar moiety is modified with a water-soluble polymer are of use in the present invention. An exemplary modified sugar nucleotide bears a sugar group that is modified through an amine moiety on the sugar. Modified sugar nucleotides, e.g., saccharyl-amine derivatives of a sugar nucleotide, are also of use in the methods of the invention. For example, a saccharyl amine (without the modifying group) can be enzymatically conjugated to a peptide (or other species) and the free saccharyl amine moiety subsequently conjugated to a desired modifying group. Alternatively, the modified sugar nucleotide can function as a substrate for an enzyme that transfers the modified sugar to a saccharyl acceptor on a substrate, e.g., a peptide or glycopeptide.

The present invention R¹ is a branched PEG, for example, one of those species set forth above. Illustrative compounds according to this embodiment include: 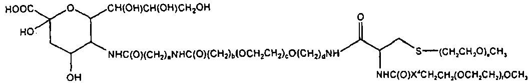 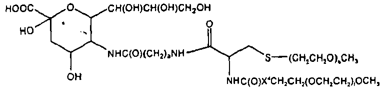 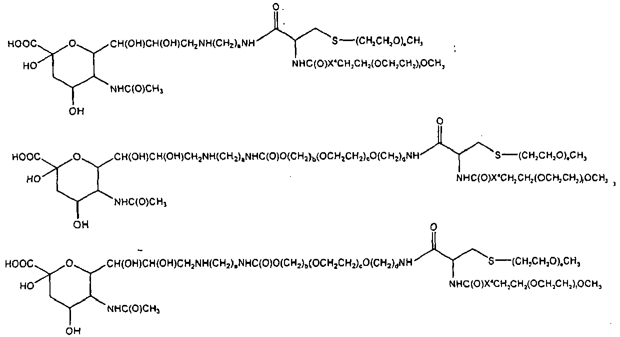 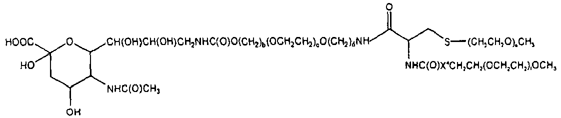 in which X⁴ is a bond or O.

Moreover, as discussed above, the present invention provides peptide conjugates that are formed using nucleotide sugars that are modified with a water-soluble polymer, which is either straight-chain or branched. For example, compounds having the formula shown below are within the scope of the present invention: 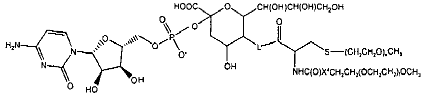 ; and 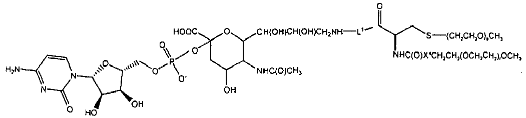 in which X⁴ is O or a bond.

Also provided are conjugates of peptides and glycopeptides, lipids and glycolipids that include the compositions of the invention. For example, the invention provides conjugates having the following formulae: 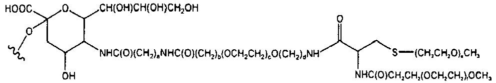 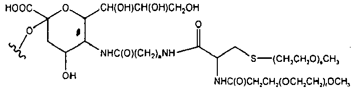 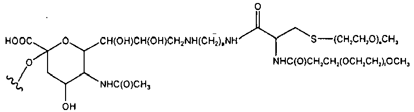 and

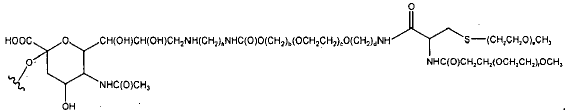

### Water-insoluble polymers

In yet another exemplary embodiment, the conjugate of the invention includes a component of a liposome. Liposomes can be prepared according to methods known to those skilled in the art, for example, as described in Eppstein et al., U.S. Patent No. 4,522,811, which issued on June 11, 1985. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its pharmaceutically acceptable salt is then introduced into the container. The container is then swirled by hand to free lipid material from the sides of the container and to disperse lipid aggregates, thereby forming the liposomal suspension.

The above-recited microparticles and methods of preparing the microparticles are offered by way of example and they are not intended to define the scope of microparticles of use in the present invention. It will be apparent to those of skill in the art that an array of microparticles, fabricated by different methods, are of use in the present invention.

The *in vivo* half-life of therapeutic glycopeptides can also be enhanced with PEG moieties such as polyethylene glycol (PEG). For example, chemical modification of proteins with PEG (PEGylation) increases their molecular size and decreases their surface- and functional group-accessibility, each of which are dependent on the size of the PEG attached to the protein. This results in an improvement of plasma half-lives and in proteolytic-stability, and a decrease in immunogenicity and hepatic uptake (Chaffee et al. J. Clin. Invest. 89: 1643-1651 (1992); Pyatak et al. Res. Commun. Chem. Pathol Pharmacol. 29: 113-127 (1980)). PEGylation of interleukin-2 has been reported to increase its antitumor potency in *vivo* (Katre et al. Proc. Natl. Acad. Sci. USA. 84: 1487-1491 (1987)) and PEGylation of a F(ab')2 derived from the monoclonal antibody A7 has improved its tumor localization (Kitamura et al. Biochem. Biophys. Res. Commun. 28: 1387-1394 (1990)). Thus, in another preferred embodiment, the in *vivo* half-life of a peptide derivatized with a PEG moiety by a method of the invention is increased relevant to the *in vivo* half life of the non-derivatized peptide.

The increase in peptide *in vivo* half-life is best expressed as a range of percent increase in this quantity. The lower end of the range of percent increase is about 40%, about 60%, about 80%, about 100%, about 150% or about 200%. The upper end of the range is about 60%, about 80%, about 100%, about 150%, or more than about 250%.

In an exemplary embodiment, the present invention provides a PEGylated G-CSF (FIG1, FIG2)

### The Methods

In addition to the conjugates discussed above, the present invention provides methods for preparing these and other conjugates. Thus, in a further aspect, the invention provides a method of forming a covalent conjugate between a selected moiety and an G-CSF peptide. Additionally, discussed are methods for targeting conjugates of the invention to a particular tissue or region of the body.

In exemplary embodiments, the conjugate is formed between a PEG moiety (or an enzymatically transferable glycosyl moiety comprising the PEG moiety), and a glycosylated or non-glycosylated peptide. The PEG is conjugated to the G-CSF peptide via an intact glycosyl linking group, which is interposed between, and covalently linked to both the G-CSF peptide and the PEG moiety, or to a PEG-non-glycosyl linker (e.g., substituted or unsubstituted alkyl, substituted or unsubstituted heteroalkyl) construct. The method includes contacting the G-CSF peptide with a mixture containing a modified sugar and a glycosyltransferase for which the modified sugar is a substrate. The reaction is conducted under conditions sufficient to form a covalent bond between the modified sugar and the G-CSF peptide. The sugar moiety of the modified sugar is preferably selected from nucleotide sugars, activated sugars and sugars, which are neither nucleotides nor activated.

The acceptor peptide (glycosylated or non-glycosylated) is typically synthesized *de novo,* or recombinantly expressed in a prokaryotic cell (*e.g*., bacterial cell, such as *E*. *coli)* or in a eukaryotic cell such as a mammalian, yeast, insect, fungal or plant cell. The G-CSF peptide can be either a full-length protein or a fragment. Moreover, the G-CSF peptide can be a wild type or mutated peptide. In an exemplary embodiment, the G-CSF peptide includes a mutation that adds one or more N- or O-linked glycosylation sites to the peptide sequence.

In an exemplary embodiment G-CSF is O-glycosylated and functionalized with a water-soluble polymer in the following manner. The peptide is either produced with an available amino acid glycosylation site or, if glycosylated, the glycosyl moiety is trimmed off to exposed the amino acid. For example, a serine or threonine is α-1 N-acetyl amino galactosylated (GalNAc) and the NAc-galactosylated peptide is sialylated with a sialic acid-modifying group cassette using ST6GalNAcT1. Alternatively, the NAc-galactosylated peptide is galactosylated using Core-1-GalT-1 and the product is sialylated with a sialic acid-modifying group cassette using ST3GalT1. An exemplary conjugate according to this method has the following linkages: Thr-α-1-GalNAc-β-1,3-Gal-α2,3-Sia*, in which Sia* is the sialic acid-modifying group cassette.

In the methods of the invention, such as that set forth above, using multiple enzymes and saccharyl donors, the individual glycosylation steps may be performed separately, or combined in a "single pot" reaction. For example, in the three enzyme reaction set forth above the GalNAc tranferase, GalT and SiaT and their donors may be combined in a single vessel. Alternatively, the GalNAc reaction can be performed alone and both the GalT and SiaT and the appropriate saccharyl donors added as a single step. Another mode of running the reactions involves adding each enzyme and an appropriate donor sequentially and conducting the reaction in a "single pot" motif. Combinations of each of the methods set forth above are of use in preparing the compounds of the invention.

In the conjugates of the invention, particularly the glycopegylated N-linked glycans, the Sia-modifying group cassette can be linked to the Gal in an α-2,6, or α-2,3 linkage.

The method of the invention also provides for modification of incompletely glycosylated peptides that are produced recombinantly. Employing a modified sugar in a method of the invention, the G-CSF peptide can be simultaneously further glycosylated and derivatized with, e.g., a PEG moiety, The sugar moiety of the modified sugar can be the residue that would properly be conjugated to the acceptor in a fully glycosylated peptide, or another sugar moiety with desirable properties.

G-CSF peptides modified by the methods of the invention can be synthetic or wild-type peptides or they can be mutated peptides, produced by methods known in the art, such as site-directed mutagenesis. Glycosylation of peptides is typically either N-linked or O-linked. An exemplary N-linkage is the attachment of the modified sugar to the side chain of an asparagine residue. The tripeptide sequences asparagine-X-serine and asparagine-X-threonine, where X is any amino acid except proline, are the recognition sequences for enzymatic attachment of a carbohydrate moiety to the asparagine side chain. Thus, the presence of either of these tripeptide sequences in a polypeptide creates a potential glycosylation site. O-linked glycosylation refers to the attachment of one sugar (e.g., N-aceylgalactosamine, galactose, mannose, GlcNAc, glucose, fucose or xylose) to a the hydroxy side chain of a hydroxyamino acid, preferably serine or threonine, although 5-hydroxyproline or 5-hydroxylysine may also be used.

For example, in one embodiment, G-CSF is expressed in a mammalian system and modified by treatment of sialidase to trim back terminal sialic acid residues, followed by PEGylation using ST3Gal3 and a donor of PEG-sialic acid.

In another exemplary embodiment, G-CSF expressed in mammalian cells is first treated with sialidase to trim back terminal sialic acid residues, then PEGylated using ST3Gal3 and a donor of PEG-sialic acid, and then sialylated using ST3Gal3 and a sialic acid donor.

G-CSF expressed in a mammalian system can also be treated with sialidase and galactosidase to trim back its sialic acid and galactose residues, then galactosylated using a galactose donor and a galactosyltransferase, and then PEGylated using ST3Gal3 and a donor of PEG-sialic acid.

In yet another examplary embodiment, the G-CSF is not first treated with sialidase, but is glycopegylated using a sialic acid transfer reaction with the modifying group-sialic acid cassette, and an enzyme such as ST3Gal3.

Also described is a method wherein G-CSF is expressed in insect cells and modified in the following procedure: N-acetylglucosamine is first added to G-CSF using an appropriate N-acetylglucosamine donor and one or more of GnT-I, II, IV, and V; G-CSF is then PEGylated using a donor of PEG-galactose and a galactosyltransferase.

G-CSF produced in yeast can also be glycopegylated. For example, G-CSF is first treated with endoglycanase to trim back the glycosyl groups, galactosylated using a galactose donor and a galactosyltransferase, and is then PEGylated with ST3Gal3 and a donor of PEG-sialic acid.

Addition of glycosylation sites to a peptide or other structure is conveniently accomplished by altering the amino acid.sequence such that it contains one or more glycosylation sites. The addition may also be made by the incorporation of one or more species presenting an -OH group, preferably serine or threonine residues, within the sequence of the G-CSF peptide (for O-linked glycosylation sites). The addition may be made by mutation or by full chemical synthesis of the G-CSF peptide. The G-CSF peptide amino acid sequence is preferably altered through changes at the DNA level, particularly by mutating the DNA encoding the peptide at preselected bases such that codons are generated that will translate into the desired amino acids. The DNA mutation(s) are preferably made using methods known in the art.

In an exemplary embodiment, the glycosylation site is added by shuffling polynucleotides. Polynucleotides encoding a candidate peptide can be modulated with DNA shuffling protocols. DNA shuffling is a process of recursive recombination and mutation, performed by random fragmentation of a pool of related genes, followed by reassembly of the fragments by a polymerase chain reaction-like process. *See, e.g.,* Stemmer, Proc. Natl. Acad. Sci. USA 91:10747-10751 (1994); Stemmer, Nature 370:389-391 (1994); and U.S. Patent Nos. 5,605,793, 5,837,458, 5,830,721 and 5,811,238.

The present invention also provides means of adding (or removing) one or more selected glycosyl residues to a peptide, after which a modified sugar is conjugated to at least one of the selected glycosyl residues of the peptide. The present embodiment is useful, for example, when it is desired to conjugate the modified sugar to a selected glycosyl residue that is either not present on a peptide or is not present in a desired amount. Thus, prior to coupling a modified sugar to a peptide, the selected glycosyl residue is conjugated to the G-CSF peptide by enzymatic or chemical coupling. In another embodiment, the glycosylation pattern of a glycopeptide is altered prior to the conjugation of the modified sugar by the removal of a carbohydrate residue from the glycopeptide. See, for example WO 98/31826.

Addition or removal of any carbohydrate moieties present on the glycopeptide is accomplished either chemically or enzymatically. Chemical deglycosylation is preferably brought about by exposure of the polypeptide variant to the compound trifluoromethanesulfonic acid, or an equivalent compound. This treatment results in the cleavage of most or all sugars except the linking sugar (N-acetylglucosamine or N-acetylgalactosamine), while leaving the peptide intact. Chemical deglycosylation is described by Hakimuddin et al., Arch. Biochem. Biophys. 259: 52 (1987) and by Edge et al., Anal. Biochem. 118: 131 (1981). Enzymatic cleavage of carbohydrate moieties on polypeptide variants can be achieved by the use of a variety of endo- and exo-glycosidases as described by Thotakura et al., Meth. Enzymol. 138: 350 (1987).

Chemical addition of glycosyl moieties is carried out by any art-recognized method. Enzymatic addition of sugar moieties is preferably achieved using a modification of the methods set forth herein, substituting native glycosyl units for the modified sugars used in the invention. Other methods of adding sugar moieties are disclosed in U.S. Patent No. 5,876,980, 6,030,815, 5,728,554, and 5,922,577.

Exemplary attachment points for selected glycosyl residue include, but are not limited to: (a) consensus sites for N- and O-glycosylation; (b) terminal glycosyl moieties that are acceptors for a glycosyltransferase; (c) arginine, asparagine and histidine; (d) free carboxyl groups; (e) free sulfhydryl groups such as those of cysteine; (f) free hydroxyl groups such as those of serine, threonine, or hydroxyproline; (g) aromatic residues such as those of phenylalanine, tyrosine, or tryptophan; or (h) the amide group of glutamine. Exemplary methods of use in the present invention are described in WO 87/05330 published Sep. 11, 1987, and in Aplin and Wriston, CRC CRIT. REV. BIOCHEM., pp. 259-306 (1981).

### The Methods

In addition to the conjugates discussed above, the present invention provides methods for preparing these and other conjugates. Moreover, the invention provides the conjugates for use in methods of preventing, curing or ameliorating a disease state by administering a conjugate of the invention to a subject at risk of developing the disease or a subject that has the disease.

Thus, the invention provides a method of forming a covalent conjugate between a selected moiety and a G-CSF peptide.

In exemplary embodiments, the conjugate is formed between a water-soluble polymer as described in the claims and a glycosylated or non-glycosylated G-CSF peptide. The polymer, therapeutic moiety or biomolecule is conjugated to the G-CSF peptide via a glycosyl linking group, which is interposed between, and covalently linked to both the peptide and the modifying group (e.g., water-soluble polymer). The method includes contacting the G-CSF peptide with a mixture containing a modified sugar and an enzyme, e.g., a glycosyltransferase, that conjugates the modified sugar to the substrate (e.g., peptide, aglycone, glycolipid). The reaction is conducted under conditions appropriate to form a covalent bond between the modified sugar and the G-CSF peptide.

The acceptor G-CSF peptide is typically synthesized *de novo,* or recombinantly expressed in a prokaryotic cell (*e.g*., bacterial cell, such as *E*. *coli*) or in a eukaryotic cell such as a mammalian, yeast, insect, fungal or plant cell. The G-CSF peptide can be either a full-length protein or a fragment. Moreover, the G-CSF peptide can be a wild type or mutated peptide. In an exemplary embodiment, the G-CSF peptide includes a mutation that adds one or more N- or O-linked glycosylation sites to the peptide sequence.

The method of the invention also provides for modification of incompletely glycosylated G-CSF peptides that are produced recombinantly. Many recombinantly produced glycoproteins are incompletely glycosylated, exposing carbohydrate residues that may have undesirable properties, e.g., immunogenicity, recognition by the RES. Employing a modified sugar in a method of the invention, the peptide can be simultaneously further glycosylated and derivatized with, e.g., a water-soluble polymer, therapeutic agent, or the like. The sugar moiety of the modified sugar can be the residue that would properly be conjugated to the acceptor in a fully glycosylated peptide, or another sugar moiety with desirable properties.

Exemplary methods of modifying peptides of use in the present invention are set forth in WO04/099231, WO 03/031464, and the references set forth therein.

In an exemplary embodiment, the invention provides a method of making a PEG-ylated G-CSF comprising the moiety: 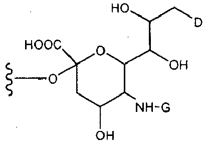 wherein D is -OH or R¹-L-HN-. The symbol G represents R¹-L- or -C(O)(C₁-C₆)alkyl. R¹ is a moiety comprising a a straight-chain or branched poly(ethylene glycol) residue. The symbol L represents a linker selected from a bond, substituted or unsubstituted alkyl and substituted or unsubstituted heteroalkyl. In general, when D is OH, G is R¹-L-, and when G is -C(O)(C₁-C₆)alkyl, D is R¹-L-NH-. The method of the invention includes, (a) contacting a substrate G-CSF peptide with a PEG-sialic acid donor and an enzyme that is capable of transferring the PEG-sialic acid moiety from the donor to the substrate G-CSF peptide.

An exemplary PEG-sialic acid donor is a nucleotide sugar such as that having the formula: 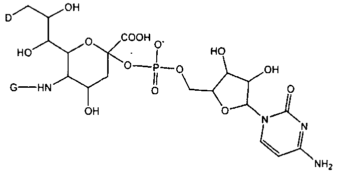 and an enzyme that transfers the PEG-sialic acid onto an amino acid or glycosyl residue of the G-CSF peptide, under conditions appropriate for the transfer.

In one embodiment the substrate G-CSF peptide is expressed in a host cell prior to the formation of the conjugate of the invention. An exemplary host cell is a mammalian cell. In other embodiments the host cell is an insect cell, plant cell, a bacteria or a fungi.

The method presented herein is applicable to each of the G-CSF conjugates set forth in the sections above.

G-CSF peptides modified by the methods of the invention can be synthetic or wild-type peptides or they can be mutated peptides, produced by methods known in the art, such as site-directed mutagenesis. Glycosylation of peptides is typically either N-linked or O-linked. An exemplary N-linkage is the attachment of the modified sugar to the side chain of an asparagine residue. The tripeptide sequences asparagine-X-serine and asparagine-X-threonine, where X is any amino acid except proline, are the recognition sequences for enzymatic attachment of a carbohydrate moiety to the asparagine side chain. Thus, the presence of either of these tripeptide sequences in a polypeptide creates a potential glycosylation site. O-linked glycosylation refers to the attachment of one sugar (*e.g.*, N-acetylgalactosamine, galactose, mannose, GlcNAc, glucose, fucose or xylose) to the hydroxy side chain of a hydroxyamino acid, preferably serine or threonine, although unusual or non-natural amino acids, e.g., 5-hydroxyproline or 5-hydroxylysine may also be used.

Addition of glycosylation sites to a peptide or other structure is conveniently accomplished by altering the amino acid sequence such that it contains one or more glycosylation sites. The addition may also be made by the incorporation of one or more species presenting an -OH group, preferably serine or threonine residues, within the sequence of the peptide (for O-linked glycosylation sites). The addition may be made by mutation or by full chemical synthesis of the peptide. The peptide amino acid sequence is preferably altered through changes at the DNA level, particularly by mutating the DNA encoding the peptide at preselected bases such that codons are generated that will translate into the desired amino acids. The DNA mutation(s) are preferably made using methods known in the art.

In an exemplary embodiment, the glycosylation site is added by shuffling polynucleotides. Polynucleotides encoding a candidate peptide can be modulated with DNA shuffling protocols. DNA shuffling is a process of recursive recombination and mutation, performed by random fragmentation of a pool of related genes, followed by reassembly of the fragments by a polymerase chain reaction-like process. *See, e.g.,* Stemmer, Proc. Natl. Acad. Sci. USA 91:10747-10751 (1994); Stemmer, Nature 370:389-391 (1994); and U.S. Patent Nos. 5,605,793, 5,837,458, 5,830,721 and 5,811,238.

Exemplary methods of adding or removing glycosylation sites, and adding or removing glycosyl structures or substructures are described in detail in WO04/099231, WO03/031464 and related U.S. and PCT applications.

The present invention also utilizes means of adding (or removing) one or more selected glycosyl residues to a G-CSF peptide, after which a modified sugar is conjugated to at least one of the selected glycosyl residues of the peptide. Such techniques are useful, for example, when it is desired to conjugate the modified sugar to a selected glycosyl residue that is either not present on a G-CSF peptide or is not present in a desired amount. Thus, prior to coupling a modified sugar to a peptide, the selected glycosyl residue is conjugated to the G-CSF peptide by enzymatic or chemical coupling. In another embodiment, the glycosylation pattern of a glycopeptide is altered prior to the conjugation of the modified sugar by the removal of a carbohydrate residue from the glycopeptide. *See,* for example WO 98/31826.

Exemplary attachment points for selected glycosyl residue include, but are not limited to: (a) consensus sites for N-linked glycosylation, and sites for O-linked glycosylation; (b) terminal glycosyl moieties that are acceptors for a glycosyltransferase; (c) arginine, asparagine and histidine; (d) free carboxyl groups; (e) free sulfhydryl groups such as those of cysteine; (f) free hydroxyl groups such as those of serine, threonine, or hydroxyproline; (g) aromatic residues such as those of phenylalanine, tyrosine, or tryptophan; or (h) the amide group of glutamine. Exemplary methods of use in the present invention are described in WO 87/05330 published Sep. 11, 1987, and in Aplin and Wriston, CRC CRIT. REV. BIOCHEM., pp. 259-306 (1981).

The PEG modified sugars are conjugated to a glycosylated or non-glycosylated peptide using an appropriate enzyme to mediate the conjugation. Preferably, the concentrations of the modified donor sugar(s), enzyme(s) and acceptor peptide(s) are selected such that glycosylation proceeds until the desired degree of modification of the acceptor is achieved. The considerations discussed below, while set forth in the context of a sialyltransferase, are generally applicable to other glycosyltransferase reactions.

A number of methods of using glycosyltransferases to synthesize desired oligosaccharide structures are known and are generally applicable to the instant invention. Exemplary methods are described, for instance, WO 96/32491, Ito et al., Pure Appl. Chem. 65: 753 (1993), U.S. Pat. Nos. 5,352,670, 5,374,541, 5,545,553, and commonly owned U.S. Pat. Nos. 6,399,336, and 6,440,703 which are incorporated herein by reference.

The present invention is practiced using a single glycosyltransferase or a combination of glycosyltransferases. For example, one can use a combination of a sialyltransferase and a galactosyltransferase. In those embodiments using more than one enzyme, the enzymes and substrates are preferably combined in an initial reaction mixture, or the enzymes and reagents for a second enzymatic reaction are added to the reaction medium once the first enzymatic reaction is complete or nearly complete. By conducting two enzymatic reactions in sequence in a single vessel, overall yields are improved over procedures in which an intermediate species is isolated. Moreover, cleanup and disposal of extra solvents and by-products is reduced.

In a preferred embodiment, each of the first and second enzyme is a glycosyltransferase. In another preferred embodiment, one enzyme is an endoglycosidase. In an additional preferred embodiment, more than two enzymes are used to assemble the modified glycoprotein of the invention. The enzymes are used to alter a saccharide structure on the G-CSF peptide at any point either before or after the addition of the modified sugar to the peptide.

In another embodiment, the method makes use of one or more exo- or endoglycosidase. The glycosidase is typically a mutant, which is engineered to form glycosyl bonds rather than rupture them. The mutant glycanase typically includes a substitution of an amino acid residue for an active site acidic amino acid residue. For example, when the endoglycanase is endo-H, the substituted active site residues will typically be Asp at position 130, Glu at position 132 or a combination thereof. The amino acids are generally replaced with serine, alanine, asparagine, or glutamine.

The mutant enzyme catalyzes the reaction, usually by a synthesis step that is analogous to the reverse reaction of the endoglycanase hydrolysis step. In these embodiments, the glycosyl donor molecule (*e.g.*, a desired oligo- or mono-saccharide structure) contains a leaving group and the reaction proceeds with the addition of the donor molecule to a GlcNAc residue on the protein. For example, the leaving group can be a halogen, such as fluoride. In other embodiments, the leaving group is a Asn, or a Asn-peptide moiety. In yet further embodiments, the GlcNAc residue on the glycosyl donor molecule is modified. For example, the GlcNAc residue may comprise a 1,2 oxazoline moiety.

In a preferred embodiment, each of the enzymes utilized to produce a conjugate of the invention are present in a catalytic amount. The catalytic amount of a particular enzyme varies according to the concentration of that enzyme's substrate as well as to reaction conditions such as temperature, time and pH value. Means for determining the catalytic amount for a given enzyme under preselected substrate concentrations and reaction conditions are well known to those of skill in the art.

The temperature at which an above process is carried out can range from just above freezing to the temperature at which the most sensitive enzyme denatures. Preferred temperature ranges are about 0 °C to about 55 °C, and more preferably about 20 °C to about 37 °C. In another exemplary embodiment, one or more components of the present method are conducted at an elevated temperature using a thermophilic enzyme.

The reaction mixture is maintained for a period of time sufficient for the acceptor to be glycosylated, thereby forming the desired conjugate. Some of the conjugate can often be detected after a few hours, with recoverable amounts usually being obtained within 24 hours or less. Those of skill in the art understand that the rate of reaction is dependent on a number of variable factors (*e.g,* enzyme concentration, donor concentration, acceptor concentration, temperature, solvent volume), which are optimized for a selected system.

The present invention also provides for the industrial-scale production of modified peptides. As used herein, an industrial scale generally produces at least one gram of finsihed, purified conjugate.

In the discussion that follows, the invention is exemplified by the conjugation of modified sialic acid moieties to a glycosylated peptide. The exemplary modified sialic acid is labeled with PEG. Moreover, the discussion is equally applicable to the modification of a glycosyl unit with agents other than PEG including other PEG moieties.

An enzymatic approach can be used for the selective introduction of PEGylated or PPGylated carbohydrates onto a peptide or glycopeptide. The method utilizes modified sugars containing PEG, PPG, or a masked reactive functional group, and is combined with the appropriate glycosyltransferase or glycosynthase. By selecting the glycosyltransferase that will make the desired carbohydrate linkage and utilizing the modified sugar as the donor substrate, the PEG or PPG can be introduced directly onto the G-CSF peptide backbone, onto existing sugar residues of a glycopeptide or onto sugar residues that have been added to a peptide.

An acceptor for the sialyltransferase is present on the G-CSF peptide to be modified by the methods of the present invention either as a naturally occurring structure or one placed there recombinantly, enzymatically or chemically. Suitable acceptors, include, for example, galactosyl acceptors such as Galβ1,4GlcNAc, Galβ1,4GalNAc, Galβ1,3GalNAc, lacto-N-tetraose, Galβ1,3GlcNAc, Galβ1,3Ara, Galβ1,6GlcNAc, Galβ1,4Glc (lactose), and other acceptors known to those of skill in the art (*see, e.g.,* Paulson et al., J Biol. Chem. 253: 5617-5624 (1978)).

In one embodiment, an acceptor for the sialyltransferase is present on the glycopeptide to be modified *upon in vivo* synthesis of the glycopeptide. Such glycopeptides can be sialylated using the claimed methods without prior modification of the glycosylation pattern of the glycopeptide. Alternatively, the methods of the invention can be used to sialylate a peptide that does not include a suitable acceptor; one first modifies the G-CSF peptide to include an acceptor by methods known to those of skill in the art: In an exemplary embodiment, a GalNAc residue is added by the action of a GalNAc transferase.

In an exemplary embodiment, the galactosyl acceptor is assembled by attaching a galactose residue to an appropriate acceptor linked to the G-CSF peptide, e.g., a GlcNAc. The method includes incubating the G-CSF peptide to be modified with a reaction mixture that contains a suitable amount of a galactosyltransferase *(e.g.,* galβ1,3 or galβ1,4), and a suitable galactosyl donor (*e.g.*, UDP-galactose). The reaction is allowed to proceed substantially to completion or, alternatively, the reaction is terminated when a preselected amount of the galactose residue is added. Other methods of assembling a selected saccharide acceptor will be apparent to those of skill in the art.

In yet another embodiment, glycopeptide-linked oligosaccharides are first "trimmed," either in whole or in part, to expose either an acceptor for the sialyltransferase or a moiety to which one or more appropriate residues can be added to obtain a suitable acceptor. Enzymes such as glycosyltransferases and endoglycosidases (*see,* for example U.S. Patent No. 5,716,812) are useful for the attaching and trimming reactions.

In the discussion that follows, the method of the invention is exemplified by the use of modified sugars having a PEG moiety attached thereto. The focus of the discussion is for clarity of illustration. Those of skill will appreciate that the discussion is equally relevant to other embodiments.

In an exemplary embodiment of the invention in which a carbohydrate residue is "trimmed" prior to the addition of the modified sugar high mannose is trimmed back to the first generation biantennary structure. A modified sugar bearing a PEG moiety is conjugated to one or more of the sugar residues exposed by the "trimming back." In one example, a PEG moiety is added via a GlcNAc moiety conjugated to the PEG moiety. The modified GlcNAc is attached to one or both of the terminal mannose residues of the biantennary structure. Alternatively, an unmodified GlcNAc can be added to one or both of the termini of the branched species.

In another exemplary embodiment, a PEG moiety is added to one or both of the terminal mannose residues of the biantennary structure via a modified sugar having a galactose residue, which is conjugated to a GlcNAc residue added onto the terminal mannose residues. Alternatively, an unmodified Gal can be added to one or both terminal GlcNAc residues.

In yet a further example, a PEG moiety is added onto a Gal residue using a modified sialic acid.

In another exemplary embodiment, a high mannose structure is "trimmed back" to the mannose from which the biantennary structure branches. In one example, a PEG moiety is added via a GlcNAc modified with the polymer. Alternatively, an unmodified GlcNAc is added to the mannose, followed by a Gal with an attached PEG moiety. In yet another embodiment, unmodified GlcNAc and Gal residues are sequentially added to the mannose, followed by a sialic acid moiety modified with a PEG moiety.

In a further exemplary embodiment, high mannose is "trimmed back" to the GlcNAc to which the first mannose is attached. The GlcNAc is conjugated to a Gal residue bearing a PEG moiety. Alternatively, an unmodified Gal is added to the GlcNAc, followed by the addition of a sialic acid modified with a water-soluble sugar. In yet a further example, the terminal GlcNAc is conjugated with Gal and the GlcNAc is subsequently fucosylated with a modified fucose bearing a PEG moiety.

High mannose may also be trimmed back to the first GlcNAc attached to the Asn of the peptide. In one example, the GlcNAc of the GlcNAc-(Fuc)ₐ residue is conjugated wit ha GlcNAc bearing a water soluble polymer. In another example, the GlcNAc of the GlcNAc-(Fuc)ₐ residue is modified with Gal, which bears a water soluble polymer. In a still further embodiment, the GlcNAc is modified with Gal, followed by conjugation to the Gal of a sialic acid modified with a PEG moiety.

Other exemplary embodiments are set forth in commonly owned U.S. Patent application Publications: 20040132640; 20040063911; 20040137557; U.S. Patent application Nos: 10/369,979; 10/410,913; 10/360,770; 10/410,945 and PCT/US02/32263.

The examples set forth above provide an illustration of the power of the methods set forth herein. Using the methods described herein, it is possible to "trim back" and build up a carbohydrate residue of substantially any desired structure. The modified sugar can be added to the termini of the carbohydrate moiety as set forth above, or it can be intermediate between the peptide core and the terminus of the carbohydrate.

In an exemplary embodiment, an existing sialic acid is removed from a G-CSF glycopeptide using a sialidase, thereby unmasking all or most of the underlying galactosyl residues. Alternatively, a peptide or glycopeptide is labeled with galactose residues, or an oligosaccharide residue that terminates in a galactose unit. Following the exposure of or addition of the galactose residues, an appropriate sialyltransferase is used to add a modified sialic acid. The approach is summarized in Scheme 1.

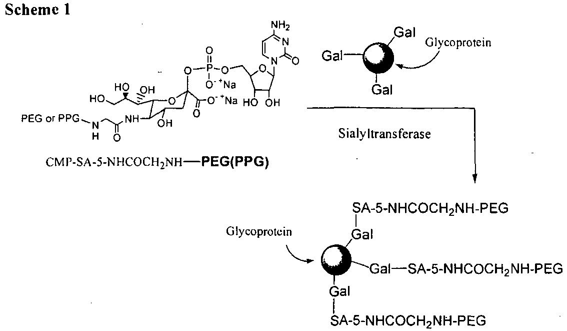

In yet a further approach, summarized in Scheme 2, a masked reactive functionality is present on the sialic acid. The masked reactive group is preferably unaffected by the conditions used to attach the modified sialic acid to the G-CSF. After the covalent attachment of the modified sialic acid to the G-CSF peptide, the mask is removed and the G-CSF peptide is conjugated with an agent such as PEG. The agent is conjugated to the peptide in a specific manner by its reaction with the unmasked reactive group on the modified sugar residue.

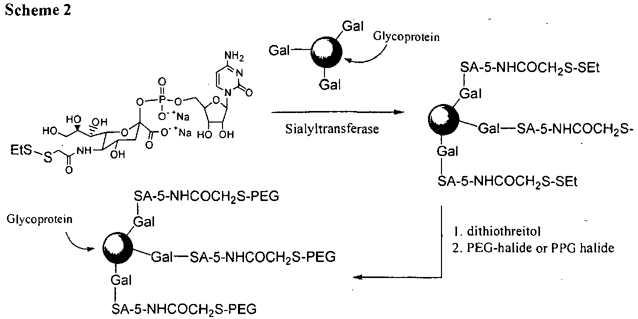

Any modified sugar set forth herein can be used with its appropriate glycosyltransferase, depending on the terminal sugars of the oligosaccharide side chains of the glycopeptide (Table 1). As discussed above, the terminal sugar of the glycopeptide required for introduction of the PEGylated structure can be introduced naturally during expression or it can be produced post expression using the appropriate glycosidase(s), glycosyltransferase(s) or mix of glycosidase(s) and glycosyltransferase(s).

**Table 1**

| | |
|---|---|
| 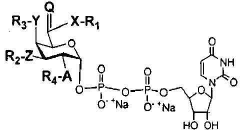 | 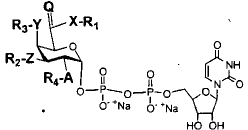 |
| UDP-galactose-derivatives | UDP-galactosamine-derivatives (when A = NH, R₄ may be acetyl) |
| 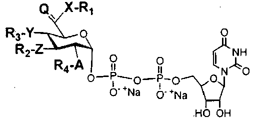 | 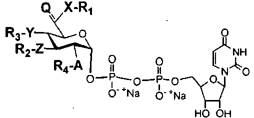 |
| UDP-Glucose-derivatives | UDP-Glucosamine-derivatives (when Δ = NH, R₄ may be acetyl) |
| 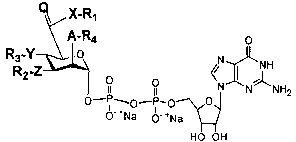 | 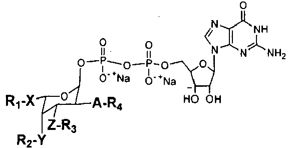 |
| GDP-Mannose-derivatives | GDP-fucose-derivatives |

| |
|---|
| X = O, NH, S, CH₂, N-(R₁₋₅)₂. |
| Y = X; Z = X; A = X; B = X. |
| Q = H₂, O, S, NH, N-R. |
| R, R₁₋₄ = H, Linker-M, M. |
| M = PEG, e.g., m-PEG |

Discussed is a method wherein UDP-galactose-PEG is reacted with bovine milk β1,4-galactosyltransferase, thereby transferring the modified galactose to the appropriate terminal N-acetylglucosamine structure. The terminal GlcNAc residues on the glycopeptide may be produced during expression, as may occur in such expression systems as mammalian, insect, plant or fungus, but also can be produced by treating the glycopeptide with a sialidase and/or glycosidase and/or glycosyltransferase, as required.

Also discussed is a method where in transferase, such as GNT1-5, is utilized to transfer PEGylated-GlcN to a terminal mannose residue on a glycopeptide. In a still further exemplary embodiment, an the N- and/or O-linked glycan structures are enzymatically removed from a glycopeptide to expose an amino acid or a terminal glycosyl residue that is subsequently conjugated with the modified sugar. For example, an endoglycanase is used to remove the N-linked structures of a glycopeptide to expose a terminal GlcNAc as a GlcNAc-linked-Asn on the glycopeptide. UDP-Gal-PEG and the appropriate galactosyltransferase is used to introduce the PEG-galactose functionality onto the exposed GlcNAc.

In an alternative embodiment, the modified sugar is added directly to the G-CSF peptide backbone using a glycosyltransferase known to transfer sugar residues to the peptide backbone. This exemplary embodiment is set forth in Scheme 3. Exemplary glycosyltransferases include, but are not limited to, GalNAc transferases (GalNAc T1-14), GlcNAc transferases, fucosyltransferases, glucosyltransferases, xylosyltransferases, mannosyltransferases and the like. Use of this approach allows the direct addition of modified sugars onto peptides that lack any carbohydrates or, alternatively, onto existing glycopeptides. In both cases, the addition of the modified sugar occurs at specific positions on the peptide backbone as defined by the substrate specificity of the glycosyltransferase and not in a random manner as occurs during modification of a protein's peptide backbone using chemical methods. An array of agents can be introduced into proteins or glycopeptides that lack the glycosyltransferase substrate peptide sequence by engineering the appropriate amino acid sequence into the polypeptide chain.

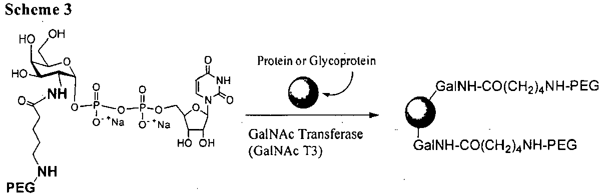

In each of the exemplary embodiments set forth above, one or more additional chemical or enzymatic modification steps can be utilized following the conjugation of the modified sugar to the peptide. In an exemplary embodiment, an enzyme (*e.g.*, fucosyltransferase) is used to append a glycosyl unit (*e.g.*, fucose) onto the terminal modified sugar attached to the G-CSF peptide. In another example, an enzymatic reaction is utilized to "cap" sites to which the modified sugar failed to conjugate. Alternatively, a chemical reaction is utilized to alter the structure of the conjugated modified sugar. For example, the conjugated modified sugar is reacted with agents that stabilize or destabilize its linkage with the peptide component to which the modified sugar is attached. In another example, a component of the modified sugar is deprotected following its conjugation to the peptide. One of skill will appreciate that there is an array of enzymatic and chemical procedures that are useful in the methods of the invention at a stage after the modified sugar is conjugated to the G-CSF peptide. Further elaboration of the modified sugar-peptide conjugate is within the scope of the invention.

### Enzymes

In addition to the enzymes discussed above in the context of forming the acyl-linked conjugate, the glycosylation pattern of the conjugate and the starting substrates (*e.g.*, peptides, lipids) can be elaborated, trimmed back or otherwise modified by methods utilizing other enzymes. The methods of remodeling peptides and lipids using enzymes that transfer a sugar donor to an acceptor are discussed in great detail in DeFrees, WO 03/031464 A2, published April 17, 2003. A brief summary of selected enzymes of use in the present method is set forth below.

### Glycosyltransferases

Glycosyltransferases catalyze the addition of activated sugars (donor NDP- or NMP-sugars), in a step-wise fashion, to a protein, glycopeptide, lipid or glycolipid or to the non-reducing end of a growing oligosaccharide. N-linked glycopeptides are synthesized via a transferase and a lipid-linked oligosaccharide donor Dol-PP-NAG₂Glc₃Man₉ in an en block transfer followed by trimming of the core. In this case the nature of the "core" saccharide is somewhat different from subsequent attachments. A very large number of glycosyltransferases are known in the art.

The glycosyltransferase to be used in the present invention may be any as long as it can utilize the modified sugar as a sugar donor. Examples of such enzymes include Leloir pathway glycosyltransferase, such as galactosyltransferase, N-acetylglucosaminyltransferase, N-acetylgalactosaminyltransferase, fucosyltransferase, sialyltransferase, mannosyltransferase, xylosyltransferase, glucurononyltransferase and the like.

For enzymatic saccharide syntheses that involve glycosyltransferase reactions, glycosyltransferase can be cloned, or isolated from any source. Many cloned glycosyltransferases are known, as are their polynucleotide sequences. *See, e.g.,* "The WWW Guide To Cloned Glycosyltransferases,"
(httt://www.vei.co.uk/TGN/gt_guide.htm). Glycosyltransferase amino acid sequences and nucleotide sequences encoding glycosyltransferases from which the amino acid sequences can be deduced are also found in various publicly available databases, including GenBank, Swiss-Prot, EMBL, and others.

Glycosyltransferases that can be employed in the methods of the invention include, but are not limited to, galactosyltransferases, fucosyltransferases, glucosyltransferases, N-acetylgalactosaminyltransferases, N-acetylglucosaminyltransferases, glucuronyltransferases, sialyltransferases, mannosyltransferases, glucuronic acid transferases, galacturonic acid transferases, and oligosaccharyltransferases. Suitable glycosyltransferases include those obtained from eukaryotes, as well as from prokaryotes.

DNA encoding glycosyltransferases may be obtained by chemical synthesis, by screening reverse transcripts of mRNA from appropriate cells or cell line cultures, by screening genomic libraries from appropriate cells, or by combinations of these procedures. Screening of mRNA or genomic DNA may be carried out with oligonucleotide probes generated from the glycosyltransferases gene sequence. Probes may be labeled with a detectable group such as a fluorescent group, a radioactive atom or a chemiluminescent group in accordance with known procedures and used in conventional hybridization assays. In the alternative, glycosyltransferases gene sequences may be obtained by use of the polymerase chain reaction (PCR) procedure, with the PCR oligonucleotide primers being produced from the glycosyltransferases gene sequence. See, U.S. Pat. No. 4,683,195 to Mullis *et al.* and U.S. Pat. No. 4,683,202 to Mullis.

The glycosyltransferase may be synthesized in host cells transformed with vectors containing DNA encoding the glycosyltransferases enzyme. Vectors are used either to amplify DNA encoding the glycosyltransferases enzyme and/or to express DNA which encodes the glycosyltransferases enzyme. An expression vector is a replicable DNA construct in which a DNA sequence encoding the glycosyltransferases enzyme is operably linked to suitable control sequences capable of effecting the expression of the glycosyltransferases enzyme in a suitable host. The need for such control sequences will vary depending upon the host selected and the transformation method chosen. Generally, control sequences include a transcriptional promoter, an optional operator sequence to control transcription, a sequence encoding suitable mRNA ribosomal binding sites, and sequences which control the termination of transcription and translation. Amplification vectors do not require expression control domains. All that is needed is the ability to replicate in a host, usually conferred by an origin of replication, and a selection gene to facilitate recognition of transformants.

In an exemplary embodiment, the invention utilizes a prokaryotic enzyme. Such glycosyltransferases include enzymes involved in synthesis of lipooligosaccharides (LOS), which are produced by many gram negative bacteria (Preston et al., Critical Reviews in Microbiology 23(3): 139-180 (1996)). Such enzymes include, but are not limited to, the proteins of the *rfa* operons of species such as *E. coli* and *Salmonella typhimurium,* which include a β1,6 galactosyltransferase and a β1,3 galactosyltransferase *(see, e.g.,* EMBL Accession Nos. M80599 and M86935 *(E. coli*); EMBL Accession No. S56361 *(S. typhimurium*)), a glucosyltransferase (Swiss-Prot Accession No. P25740 *(E. coli),* an β1,2-glucosyltransferase (*rfa*J)(Swiss-Prot Accession No. P27129 (*E. coli)* and Swiss-Prot Accession No. P19817 (*S. typhimurium*)), and an β1,2-N-acetylglucosaminyltransferase (*rfa*K)(EMBL Accession No. U00039 *(E. coli).* Other glycosyltransferases for which amino acid sequences are known include those that are encoded by operons such as *rfa*B*,* which have been characterized in organisms such as *Klebsiella pneumoniae, E. coli, Salmonella typhimurium, Salmonella enterica, Yersinia enterocolitica, Mycobacterium leprosum,* and the *rhl* operon of *Pseudomonas aeruginosa.*

Also suitable for use in the present invention are glycosyltransferases that are involved in producing structures containing lacto-N-neotetraose, D-galactosyl-β1,4-N-acetyl-D-glucosaminyl-β-1,3-D-galactosyl-β-1,4-D-glucose, and the P^{k} blood group trisaccharide sequence, D-galactosyl-α-1,4-D-galactosyl-β-1,4-D-glucose, which have been identified in the LOS of the mucosal pathogens *Neisseria gonnorhoeae* and *N. meningitidis* (Scholten et al., J. Med. Microbiol. 41: 236-243 (1994)). The genes from *N. meningitidis* and *N. gonorrhoeae* that encode the glycosyltransferases involved in the biosynthesis of these structures have been identified from *N. meningitidis* immunotypes L3 and L1 (Jennings et al., Mol. Microbiol. 18: 729-740 (1995)) and the *N. gonorrhoeae* mutant F62 (Gotshlich, J. Exp. Med. 180: 2181-2190 (1994)). In *N. meningitidis,* a locus consisting of three genes, *lgtA, lgtB* and *lg E,* encodes the glycosyltransferase enzymes required for addition of the last three of the sugars in the lacto-N-neotetraose chain (Wakarchuk et al., J Biol. Chem. 271: 19166-73 (1996)). Recently the enzymatic activity of the *lgtB* and *lgtA* gene product was demonstrated, providing the first direct evidence for their proposed glycosyltransferase function (Wakarchuk et al., J Biol. Chem. 271(45): 28271-276 (1996)). In *N. gonorrhoeae,* there are two additional genes, IgtD which adds β-D-GalNAc to the 3 position of the terminal galactose of the lacto-*N-*neotetraose structure and *IgtC* which adds a terminal α-D-Gal to the lactose element of a truncated LOS, thus creating the P^{k} blood group antigen structure (Gotshlich (1994), *supra*.). In *N. meningitidis,* a separate immunotype L1 also expresses the P^{k} blood group antigen and has been shown to carry an *IgtC* gene (Jennings *et al.,* (1995), *supra.). Neisseria* glycosyltransferases and associated genes are also described in USPN 5,545,553 (Gotschlich). Genes for α1,2-fucosyltransferase and α1,3-fucosyltransferase from *Helicobacter pylori* has also been characterized (Martin et al., J. Biol. Chem. 272: 21349-21356 (1997)). Also of use in the present invention are the glycosyltransferases *of Campylobacler jejuni (see,* for example, http://afmb.cnrs-mrs.fr/-pedro/CAZY/gtf_42.html).

### Fucosyltransferases

In some embodiments, a glycosyltransferase used in the method of the invention is a fucosyltransferase. Fucosyltransferases are known to those of skill in the art. Exemplary fucosyltransferases include enzymes, which transfer L-fucose from GDP-fucose to a hydroxy position of an acceptor sugar. Fucosyltransferases that transfer non-nucleotide sugars to an acceptor are also of use in the present invention.

In some embodiments, the acceptor sugar is, for example, the GlcNAc in a Galp(1→3,4)GlcNAcβ- group in an oligosaccharide glycoside. Suitable fucosyltransferases for this reaction include the Galβ(1→3,4)GlcNAcβ1-α(1→3,4)fucosyltransferase (FTIII E.C. No. 2.4.1.65), which was first characterized from human milk (*see,* Palcic, et al., Carbohydrate Res. 190: 1-11 (1989); Prieels, et al., J Biol. Chem. 256: 10456-10463 (1981); and Nunez, et al., Can. J. Chem. 59: 2086-2095 (1981)) and the Galβ(1→4)GlcNAcβ- afucosyltransferases (FTIV, FTV, FTVI) which are found in human serum. FTVII (E.C. No. 2.4.1.65), a-sialyl α(2→3)Galβ((1→3)GlcNAcβ fucosyltransferase, has also been characterized. A recombinant form of the Galβ(1→3,4) GlcNAcβ- α(1→3,4)fucosyltransferase has also been characterized (*see,* Dumas, et al., Bioorg. Med. Letters 1: 425-428 (1991) and Kukowska-Latallo, et al., Genes and Development 4: 1288-1303 (1990)). Other exemplary fucosyltransferases include, for example, α1,2 fucosyltransferase (E.C. No. 2.4.1.69). Enzymatic fucosylation can be carried out by the methods described in Mollicone, et al., Eur. J Biochem. 191: 169-176 (1990) or U.S. Patent No. 5,374,655. Cells that are used to produce a fucosyltransferase will also include an enzymatic system for synthesizing GDP-fucose.

### Galactasyltransferases

In another group of embodiments, the glycosyltransferase is a galactosyltransferase. Exemplary galactosyltransferases include α(1,3) galactosyltransferases (E.C. No. 2.4.1.151, *see, e.g.,* Dabkowski et al., Transplant Proc. 25:2921 (1993) and Yamamoto et al. Nature 345: 229-233 (1990), bovine (GenBank j04989, Joziasse et al., J. Biol. Chem. 264: 14290-14297 (1989)), murine (GenBank m26925; Larsen et al., Proc. Nat'l. Acad. Sci. USA 86: 8227-8231 (1989)), porcine (GenBank L36152; Strahan et al., Immunogenetics 41: 101-105 (1995)). Another suitable α1,3 galactosyltransferase is that which is involved in synthesis of the blood group B antigen (EC 2.4.1.37, Yamamoto et al., J. Biol. Chem. 265: 1146-1151 (1990) (human)). Yet a further exemplary galactosyltransferase is core Gal-T1.

Also suitable for use in the methods of the invention are β(1,4) galactosyltransferases, which include, for example, EC 2.4.1.90 (LacNAc synthetase) and EC 2.4.1.22 (lactose synthetase) (bovine (D'Agostaro et al., Eur. J Biochem. 183: 211-217 (1989)), human (Masri et al., Biochem. Biophys. Res. Commun. 157: 657-663 (1988)), murine (Nakazawa et al., J. Biochem. 104: 165-168 (1988)), as well as E.C. 2.4.1.38 and the ceramide galactosyltransferase (EC 2.4.1.45, Stahl et al., J Neurosci. Res. 38: 234-242 (1994)). Other suitable galactosyltransferases include, for example, α1,2 galactosyltransferases (from *e.g., Schizosaccharomyces pombe,* Chapell et al., Mol. Biol. Cell 5: 519-528 (1994)).

### Sialyltransferases

Sialyltransferases are another type of glycosyltransferase that is useful in the recombinant cells and reaction mixtures of the invention. Cells that produce recombinant sialyltransferases will also produce CMP-sialic acid, which is a sialic acid donor for sialyltransferases. Examples of sialyltransferases that are suitable for use in the present invention include ST3Gal III *(e.g.,* a rat or human ST3Gal III), ST3Gal IV, ST3Gal I, ST3GalII, ST6Gal I, ST3Gal V, ST6Gal II, ST6GalNAc I, ST6GaINAc II, and ST6GalNAc III (the sialyltransferase nomenclature used herein is as described in Tsuji et al., Glycobiology 6: v-xiv (1996)). An exemplary α(2,3)sialyltransferase referred to as α(2,3)sialyltransferase (EC 2.4.99.6) transfers sialic acid to the non-reducing terminal Gal of Galβ1→3Glc disaccharide or glycoside. *See,* Van den Eijnden et al., J Biol. Chem. 256: 3159 (1981), Weinstein et al., J. Biol, Chem. 257: 13845 (1982) and Wen et al., J Biol. Chem. 267: 21011 (1992). Another exemplary α2,3-sialyltransferase (EC 2.4.99.4) transfers sialic acid to the non-reducing terminal Gal of the disaccharide or glycoside. *see,* Rearick et al., J Biol. Chem. 254: 4444 (1979) and Gillespie et al., J Biol. Chem. 267: 21004 (1992). Further exemplary enzymes include Gal-β-1,4-GlcNAc α-2,6 sialyltransferase (*See,* Kurosawa et al. Eur. J. Biochem. 219: 375-381 (1994)).

Preferably, for glycosylation of carbohydrates of glycopeptides the sialyltransferase will be able to transfer sialic acid to the sequence Galβ1,4GlcNAc-, the most common penultimate sequence underlying the terminal sialic acid on fully sialylated carbohydrate structures (*see,* Table 2).

**Table 2: Sialyltransferases which use the Galβ1,4GlcNAc sequence as an acceptor substrate**

| **Sialyltransferase** | **Source** | **Sequence(s) formed** | **Ref.** |
|---|---|---|---|
| ST6Gal I | Mammalian | NeuAcα2,6Galβ1,4GlCNAC- | 1 |
| ST3Gal III | Mammalian | NeuAcα2,3Galβ1,4GlCNAc- | 1 |
| | | NeuAcα2,3Galβ1,3GlCNAc- | |
| ST3Gal IV | Mammalian | NeuAcα2,3Galβ1,4GlCNAc- | 1 |
| | | NeuAcα2,3Galβ1,3GlCNAc- | |
| ST6Gal II | Mammalian | NeuAcα2,6Galβ1,4GlCNA | |
| ST6Gal II | photobacterium | NeuAcα2,6Galβ,4GlCNAc- | 2 |
| ST3Gal V | *N. meningitides* | NeuAcα2,3Galβ1,4GlCNAc- | 3 |
| | *N. gonorrhoeae* | | |

| | | | |
|---|---|---|---|
| 1) Goochee et al., Bio/Technology 9: 1347-1355 (1991) 2) Yamamoto et al., J Biochem. 120: 104-110 (1996) 3) Gilbert et al., J Biol. Chem. 271: 28271-28276 (1996) | | | |

An example of a sialyltransferase that is useful in the claimed methods is ST3Gal III, which is also referred to as α(2,3)sialyltransferase (EC 2.4.99.6). This enzyme catalyzes the transfer of sialic acid to the Gal of a Galβ1,3GlcNAc or Galβ1,4GlcNAc glycoside *(see, e.g.,* Wen et al., J. Biol. Chem. 267: 21011 (1992); Van den Eijnden et al., J. Biol. Chem. 256: 3159 (1991)) and is responsible for sialylation of asparagine-linked oligosaccharides in glycopeptides. The sialic acid is linked to a Gal with the formation of an α-linkage between the two saccharides. Bonding (linkage) between the saccharides is between the 2-position of NeuAc and the 3-position of Gal. This particular enzyme can be isolated from rat liver (Weinstein et al., J Biol. Chem. 257: 13845 (1982)); the human cDNA (Sasaki et al. (1993) J. Biol. Chem. 268: 22782-22787; Kitagawa & Paulson (1994) J Biol. Chem. 269: 1394-1401) and genomic (Kitagawa et al. (1996) J. Biol. Chem. 271: 931-938) DNA sequences are known, facilitating production of this enzyme by recombinant expression. In a preferred embodiment, the claimed sialylation methods use a rat ST3Gal III.

Other exemplary sialyltransferases of use in the present invention include those isolated from Campylobacterjejuni, including CST-I and CST-II and those forming α (2,3) linkages. *See,* e.g, WO99/49051.

Sialyltransferases other those listed in Table 2, are also useful in an economic and efficient large-scale process for sialylation of commercially important glycopeptides. As a simple test to find out the utility of these other enzymes, various amounts of each enzyme (1-100 mU/mg protein) are reacted with asialo-α₁ AGP (at 1-10 mg/ml) to compare the ability of the sialyltransferase of interest to sialylate glycopeptides relative to either bovine ST6Gal I, ST3Gal III or both sialyltransferases. Alternatively, other glycopeptides or glycopeptides, or N-linked oligosaccharidcs enzymatically released from the peptide backbone can be used in place of asialo-α₁ AGP for this evaluation. Sialyltransferases with the ability to sialylate N-linked oligosaccharides of glycopeptides more efficiently than ST6Gal I are useful in a practical large-scale process for peptide sialylation.

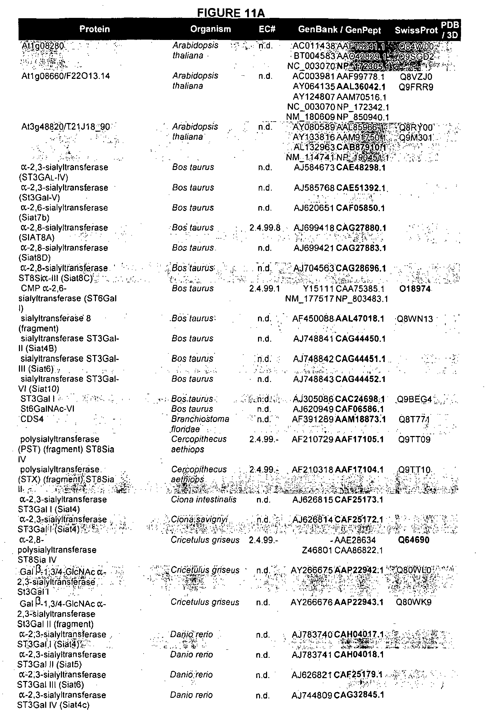

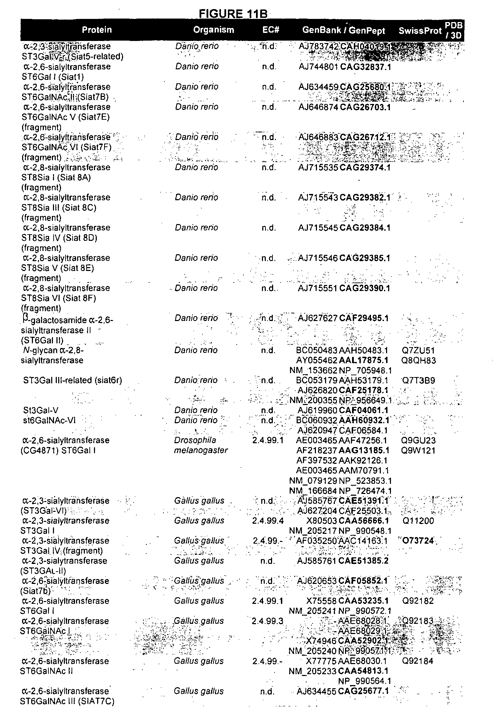

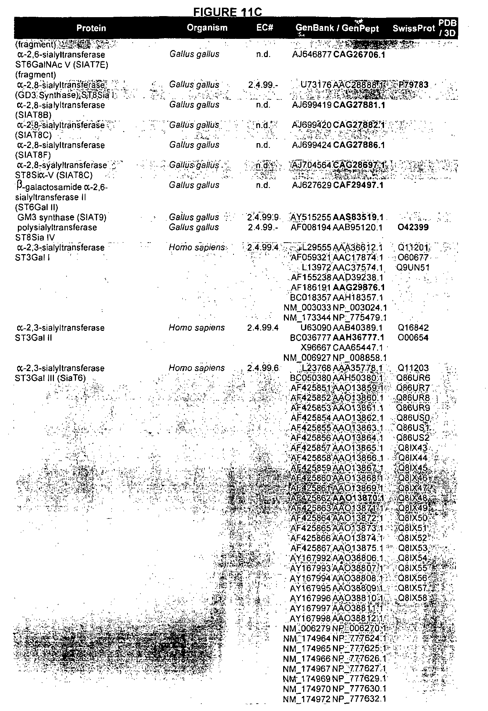

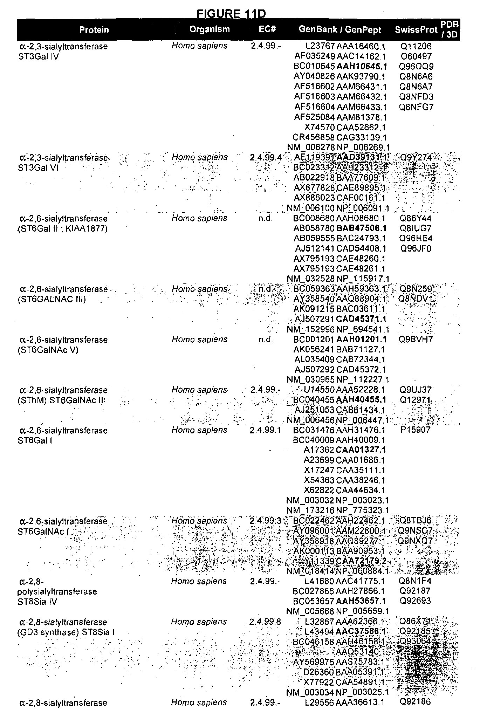

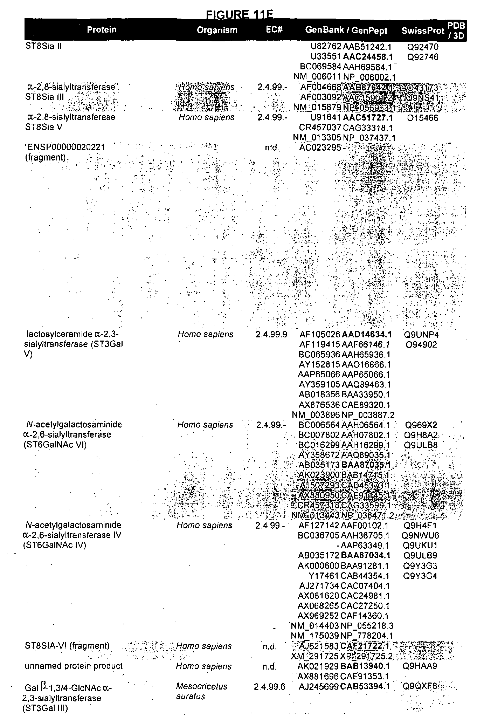

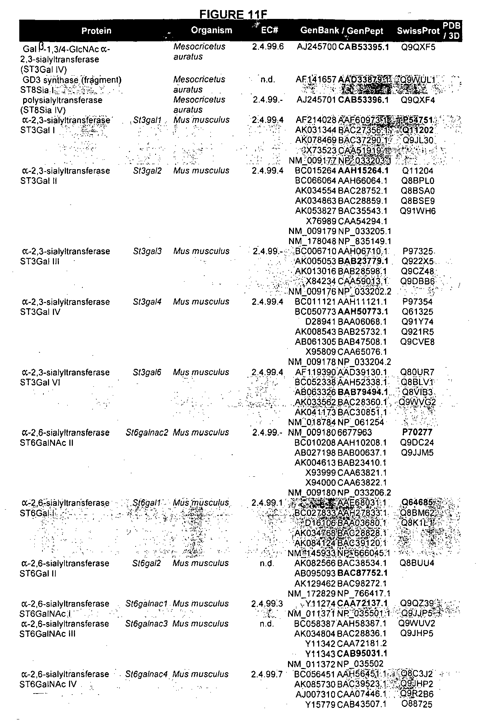

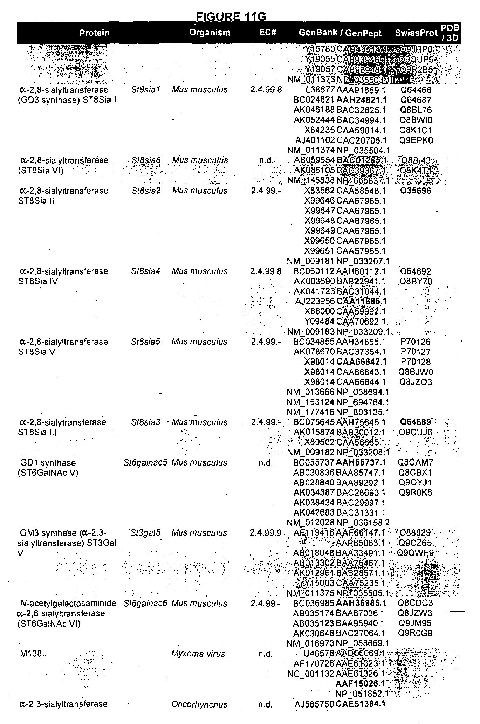

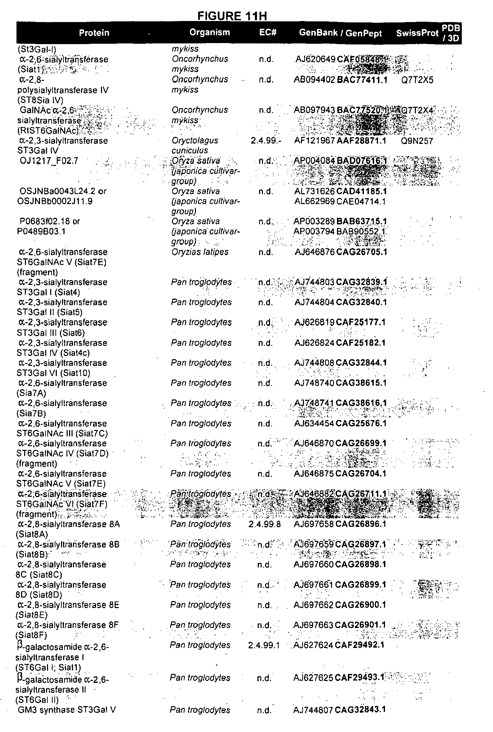

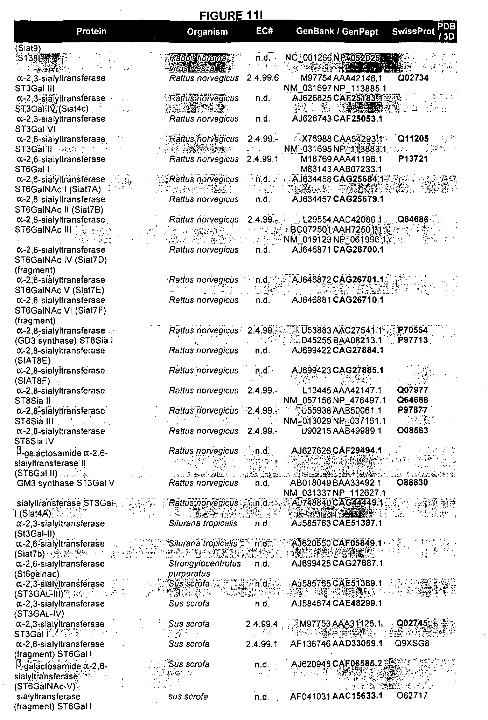

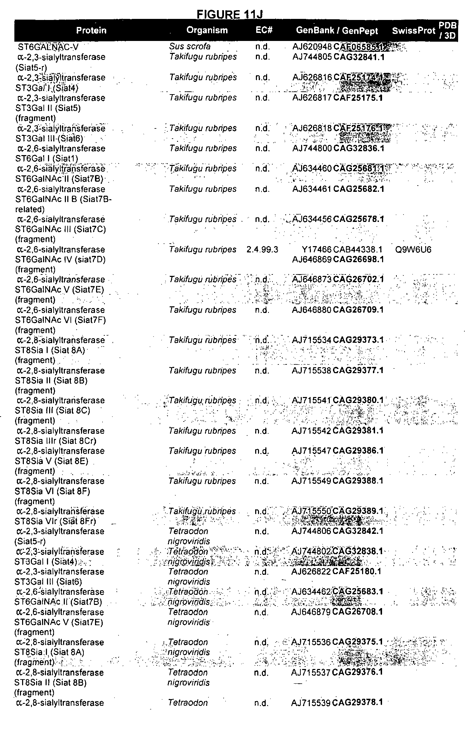

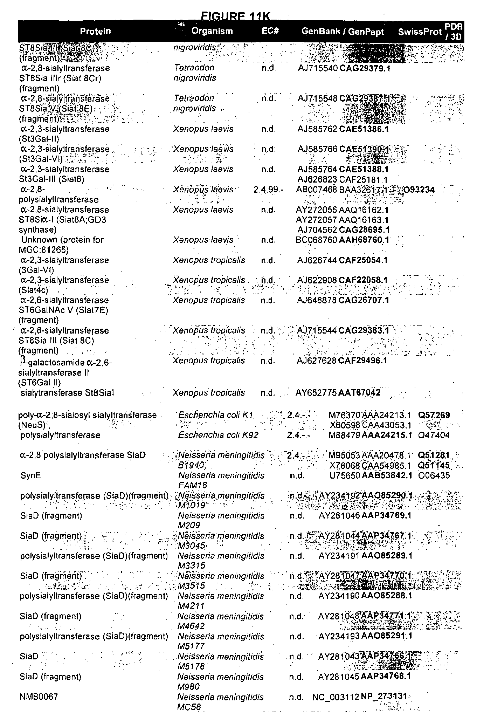

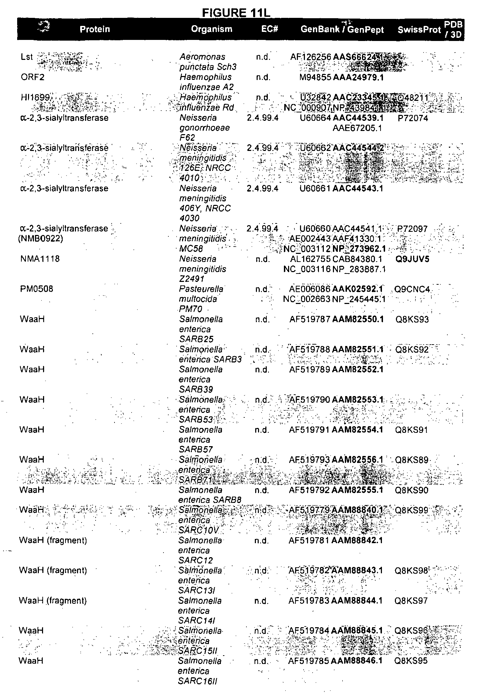

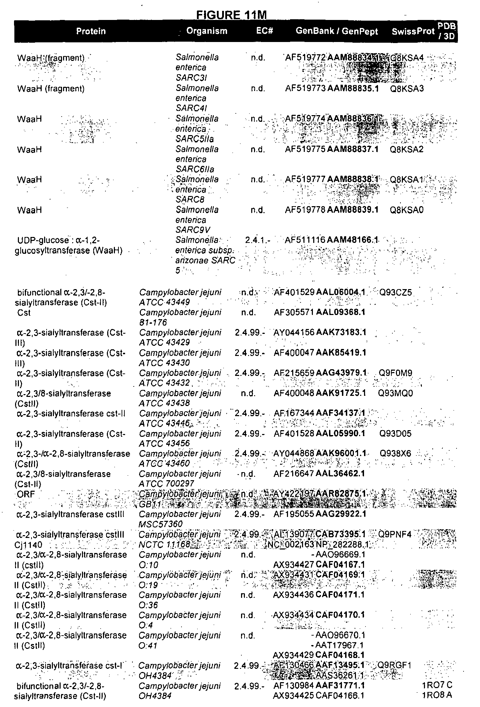

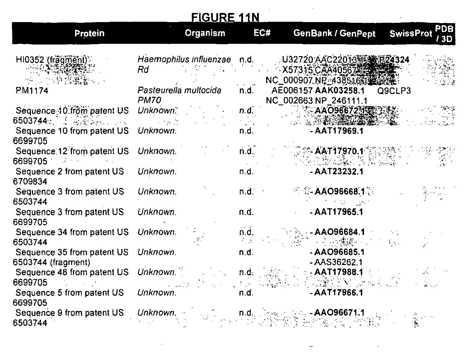

These and additional sialyltransferases are set forth in **FIG. 11****,** is a table of sialyl transferases that are of use for transferring to an acceptor the modified sialic acid species set forth herein and unmodified sialic acid.

### CalNAc transferases

N-acetylgalactosaminyltransferases are of use in practicing the present invention, particularly for binding a GalNAc moiety to an amino acid of the O-linked glycosylation site of the peptide. Suitable N-acetylgalactosaminyltransferases include, but are not limited to, α(1,3) N-acetylgalactosaminyltransferase, β(1,4) N-acetylgalactosaminyltransferases (Nagata et al., J. Biol. Chem. 267: 12082-12089 (1992) and Smith et al., J. Biol Chem. 269: 15162 (1994)) and polypeptide N-acetylgalactosaminyltransferase (Homa et al., J. Biol. Chem. 268: 12609 (1993)).

Production of proteins such as the enzyme GalNAc T₁₋ₓₓ from cloned genes by genetic engineering is well known. See, eg., U.S. Pat. No. 4,761,371. One method involves collection of sufficient samples, then the amino acid sequence of the enzyme is determined by N-terminal sequencing. This information is then used to isolate a cDNA clone encoding a full-length (membrane bound) transferase which upon expression in the insect cell line Sf9 resulted in the synthesis of a fully active enzyme. The acceptor specificity of the enzyme is then determined using a semiquantitative analysis of the amino acids surrounding known glycosylation sites in 16 different proteins followed by in vitro glycosylation studies of synthetic peptides. This work has demonstrated that certain amino acid residues are overrepresented in glycosylated peptide segments and that residues in specific positions surrounding glycosylated serine and threonine residues may have a more marked influence on acceptor efficiency than other amino acid moieties.

### Cell-Bound Glycosyltransferases

In another embodiment, the enzymes utilized in the method of the invention are cell-bound glycosyltransferases. Although many soluble glycosyltransferases are known (see, for example, U.S. Pat. No. 5,032,519), glycosyltransferases are generally in membrane-bound form when-associated with cells. Many of the membrane-bound enzymes studied thus far are considered to be intrinsic proteins; that is, they are not released from the membranes by sonication and require detergents for solubilization. Surface glycosyltransferases have been identified on the surfaces of vertebrate and invertebrate cells, and it has also been recognized that these surface transferases maintain catalytic activity under physiological conditions. However, the more recognized function of cell surface glycosyltransferases is for intercellular recognition (Roth, MOLECULAR APPROACHES to SUPRACELLULAR PHENOMENA, 1990).

Methods have been_developed to alter the glycosyltransferases expressed by cells. For example, Larsen et al., Proc. Natl. Acad. Sci. USA 86: 8227-8231 (1989), report a genetic approach to isolate cloned cDNA sequences that determine expression of cell surface oligosaccharide structures and their cognate glycosyltransferases. A cDNA library generated from mRNA isolated from a murine cell line known to express UDP-galactose:.β.-D-galactosyl-1,4-N-acetyl-D-glucosaminide α-1,3-galactosyltransferase was transfected into COS-1 cells. The transfected cells were then cultured and assayed for α 1-3 galactosyltransferase activity.

Francisco et al., Proc. Natl. Acad. Sci. USA 89: 2713-2717 (1992), disclose a method of anchoring β-lactamase to the external surface *of Escherichia coli.* A tripartite fusion consisting of (i) a signal sequence of an outer membrane protein, (ii) a membrane-spanning section of an outer membrane protein, and (iii) a complete mature β-lactamase sequence is produced resulting in an active surface bound β-lactamase molecule. However, the Francisco method is limited only to procaryotic cell systems and as recognized by the authors, requires the complete tripartite fusion for proper functioning.

### Sulfotransferases

The invention also provides methods for producing peptides that include sulfated molecules, including, for example sulfated polysaccharides such as heparin, heparan sulfate, carragenen, and related compounds. Suitable sulfotransferases include, for example, chondroitin-6-sulphotransferase (chicken cDNA described by Fukuta et al., J Biol. Chem. 270: 18575-18580 (1995); GenBank Accession No. D49915), glycosaminoglycan N-acetylglucosamine N-deacetylase/N-sulphotransferase 1 (Dixon et al., Genomics 26: 239-241 (1995); UL18918), and glycosaminoglycan N-acetylglucosamine N-deacetylase/N-sulphotransferase 2 (murine cDNA described in Orellana et al., J Biol. Chem. 269: 2270-2276 (1994) and Eriksson et al., J Biol. Chem. 269: 10438-10443 (1994); human cDNA described in GenBank Accession No. U2304).

### Glycosidases

This invention also encompasses the use of wild-type and mutant glycosidases. Mutant β-galactosidase enzymes have been demonstrated to catalyze the formation of disaccharides through the coupling of an α-glycosyl fluoride to a galactosyl acceptor molecule. (Withers, U.S. Pat. No. 6,284,494; issued Sept. 4, 2001). Other glycosidases of use in this invention include, for example, β-glucosidases, β-galactosidases, β-mannosidases, β-acetyl glucosaminidases, β-N-acetyl galactosaminidases, β-xylosidases, β-fucosidases, cellulases, xylanases, galactanases, mannanases, hemicellulases, amylases, glucoamylases, α-glucosidases, α-galactosidases, α-mannosidases, α-N-acetyl glucosaminidases, α-N-acetyl galactose-aminidases, α-xylosidases, α-fucosidases, and neuraminidases/sialidases.

### Immobilized Enzymes

The present invention also provides for the use of enzymes that are immobilized on a solid and/or soluble support. In an exemplary embodiment, there is provided a glycosyltransferase that is conjugated to a PEG via an intact glycosyl linker according to the methods of the invention. The PEG-linker-enzyme conjugate is optionally attached to solid support. The use of solid supported enzymes in the methods of the invention simplifies the work up of the reaction mixture and purification of the reaction product, and also enables the facile recovery of the enzyme. The glycosyltransferase conjugate is utilized in the methods of the invention. Other combinations of enzymes and supports will be apparent to those of skill in the art.

### Fusion Proteins

In other exemplary embodiments, the methods of the invention utilize fusion proteins that have more than one enzymatic activity that is involved in synthesis of a desired glycopeptide conjugate. The fusion polypeptides can be composed of, for example, a catalytically active domain of a glycosyltransferase that is joined to a catalytically active domain of an accessory enzyme. The accessory enzyme catalytic domain can, for example, catalyze a step in the formation of a nucleotide sugar that is a donor for the glycosyltransferase, or catalyze a reaction involved in a glycosyltransferase cycle. For example, a polynucleotide that encodes a glycosyltransferase can be joined, in-frame, to a polynucleotide that encodes an enzyme involved in nucleotide sugar synthesis. The resulting fusion protein can then catalyze not only the synthesis of the nucleotide sugar, but also the transfer of the sugar moiety to the acceptor molecule. The fusion protein can be two or more cycle enzymes linked into one expressible nucleotide sequence. In other embodiments the fusion protein includes the catalytically active domains of two or more glycosyltransferases. See, for example, 5,641,668. The modified glycopeptides of the present invention can be readily designed and manufactured utilizing various suitable fusion proteins (*see*, for example, PCT Patent Application PCT/CA98/01180, which was published as WO 99/31224 on June 24, 1999.)

### Preparation of Modified Sugars

In general, the sugar moiety or sugar moiety-linker cassette and the PEG or PEG-linker cassette groups are linked together through the use of reactive groups, which are typically transformed by the linking process into a new organic functional group or unreactive species. The sugar reactive functional group(s), is located at any position on the sugar moiety. Reactive groups and classes of reactions useful in practicing the present invention are generally those that are well known in the art of bioconjugate chemistry. Currently favored classes of reactions available with reactive sugar moieties are those, which proceed under relatively mild conditions. These include, but are not limited to nucleophilic substitutions (*e.g.*, reactions of amines and alcohols with acyl halides, active esters), electrophilic substitutions (*e.g.*, enamine reactions) and additions to carbon-carbon and carbon-heteroatom multiple bonds (*e.g.*, Michael reaction, Diels-Alder addition). These and other useful reactions are discussed in, for example, March, ADVANCED ORGANIC CHEMISTRY, 3rd Ed., John Wiley & Sons, New York, 1985; Hermanson, BIOCONJUGATE TECHNIQUES, Academic Press, San Diego, 1996; and Feeney et al., MODIFICATION OF PROTEINS; Advances in Chemistry Series, Vol. 198, American Chemical Society, Washington, D.C., 1982.

Useful reactive functional groups pendent from a sugar nucleus or modifying group include, but are not limited to:
(a) carboxyl groups and various derivatives thereof including, but not limited to, N-hydroxysuccinimide esters, N-hydroxybenztriazole esters, acid halides, acyl imidazoles, thioesters, p-nitrophenyl esters, alkyl, alkenyl, alkynyl and aromatic esters;
(b) hydroxyl groups, which can be converted to, *e.g.,* esters, ethers, aldehydes, *etc.*
(c) haloalkyl groups, wherein the halide can be later displaced with a nucleophilic group such as, for example, an amine, a carboxylate anion, thiol anion, carbanion, or an alkoxide ion, thereby resulting in the covalent attachment of a new group at the functional group of the halogen atom;
(d) dienophile groups, which are capable of participating in Diets-Alder reactions such as, for example, maleimido groups;
(e) aldehyde or ketone groups, such that subsequent derivatization is possible via formation of carbonyl derivatives such as, for example, imines, hydrazones, semicarbazones or oximes, or via such mechanisms as Grignard addition or alkyllithium addition;
(f) sulfonyl halide groups for subsequent reaction with amines, for example, to form sulfonamides;
(g) thiol groups, which can be, for example, converted to disulfides or reacted with acyl halides;
(h) amine or sulfhydryl groups, which can be, for example, acylated, alkylated or oxidized;
(i) alkenes, which can undergo, for example, cycloadditions, acylation, Michael addition, *etc;* and
(j) epoxides, which can react with, for example, amines and hydroxyl compounds.

The reactive functional groups can be chosen such that they do not participate in, or interfere with, the reactions necessary to assemble the reactive sugar nucleus or modifying group. Alternatively, a reactive functional group can be protected from participating in the reaction by the presence of a protecting group. Those of skill in the art understand how to protect a particular functional group such that it does not interfere with a chosen set of reaction conditions. For examples of useful protecting groups, *see,* for example, Greene et al., PROTECTIVE GROUPS IN ORGANIC SYNTHESIS, John Wiley & Sons, New York, 1991.

In the discussion that follows, a number of specific examples of modified sugars that are useful in practicing the present invention are set forth. In the exemplary embodiments, a sialic acid derivative is utilized as the sugar nucleus to which the modifying group is attached. The focus of the discussion on sialic acid derivatives is for clarity of illustration only and should not be construed to limit the scope of the invention. Those of skill in the art will appreciate that a variety of other sugar moieties can be activated and derivatized in a manner analogous to that set forth using sialic acid as an example. For example, numerous methods are available for modifying galactose, glucose, N-acetylgalactosamine and fucose to name a few sugar substrates, which are readily modified by art recognized methods. *See,* for example, Elhalabi et al., Curr. Med. Chem. 6: 93 (1999); and Schafer et al., J. Org. Chem. 65: 24 (2000)).

In an exemplary embodiment, the G-CSF peptide that is modified by a method of the invention is a glycopeptide that is produced in mammalian cells (e.g., CHO cells) or in a transgenic animal and thus, contains N- and/or O-linked oligosaccharide chains, which are incompletely sialylated. The oligosaccharide chains of the glycopeptide lacking a sialic acid and containing a terminal galactose residue can be PEGylated, PPGylated or otherwise modified with a modified sialic acid.

In Scheme 4, the amino glycoside **1,** is treated with the active ester of a protected amino acid (e.g., glycine) derivative, converting the sugar amine residue into the corresponding protected amino acid amide adduct. The adduct is treated with an aldolase to form α-hydroxy carboxylate **2.** Compound **2** is converted to the corresponding CMP derivative by the action of CMP-SA synthetase, followed by catalytic hydrogenation of the CMP derivative to produce compound 3. The amine introduced via formation of the glycine adduct is utilized as a locus of PEG attachment by reacting compound 3 with an activated PEG or PPG derivative (*e.g.,* PEG-C(O)NHS, PEG-OC(O)O-p-nitrophenyl), producing species such as **4** or **5,** respectively.

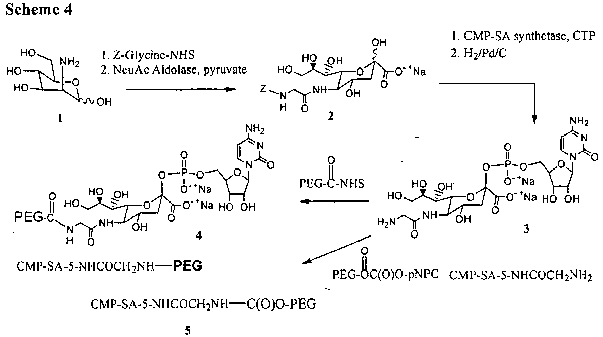

Table 3 sets forth representative examples of sugar monophosphates that are derivatized with a PEG moiety. Certain of the compounds of Table 3 are prepared by the method of Scheme 4. Other derivatives are prepared by art-recognized methods. *See,* for example, Keppler et al., Glycobiology 11: 11R (2001); and Charter et al., Glycobiology 10: 1049 (2000)). Other amine reactive PEG and PPG analogues are commercially available, or they can be prepared by methods readily accessible to those of skill in the art.

**Table 3**

| | |
|---|---|
| 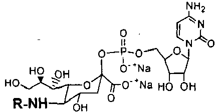 | 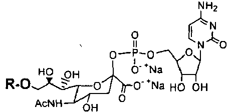 |
| CMP-SA-5-NH-R | CMP-NeuAc-9-O-R |
| 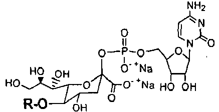 | 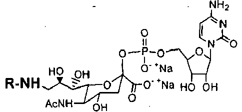 |
| rMP-KnN-5-O-R | CMP-NeuAc-9-NH-R |
| 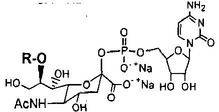 | 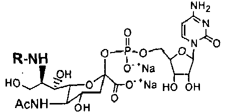 |
| CMP-NeuAc-8-O-R | CMP-NeuAc-8-NH-R |
| 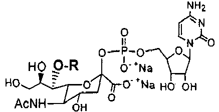 | 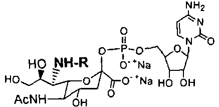 |
| CMP-NeuAc-7-O-R | CMP-NeuAc-7-NH-R |
| 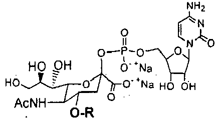 | 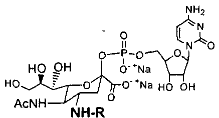 |
| CMP-NeuAc-4-O-R | CMP-NeuAc-4-NH-R |

The modified sugar phosphates of use in practicing the present invention can be substituted in other positions as well as those set forth above. Presently preferred substitutions of sialic acid are set forth in the formula below: 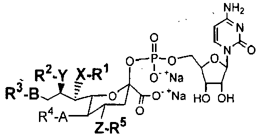 in which X is a linking group, which is preferably selected from -O-, -N(H)-, -S, - CH₂-, and N(R)₂, in which each R is a member independently selected from R¹-R⁵. The symbols Y, Z, A and B each represent a group that is selected from the group set forth above for the identity of X. X, Y, Z, A and B are each independently selected and, therefore, they can be the same or different. The symbols R¹, R², R³, R⁴ and R⁵ represent H, a PEG moiety, therapeutic moiety, biomolecule or other moiety. Alternatively, these symbols represent a linker that is bound to a PEG moiety, therapeutic moiety, biomolecule or other moiety.

Exemplary moieties attached to the conjugates disclosed herein include PEG derivatives as described in the claims. (e.g., acyl-PEG, acyl-alkyl-PEG, alkyl-acyl-PEG carbamoyl-PEG, aryl-PEG). Methods of conjugating the various modifying groups to a saccharide moiety are readily accessible to those of skill in the art (POLY (ETHYLENE GLYCOL CHEMISTRY : BIOTECHNICAL AND BIOMEDICAL APPLICATIONS, J. Milton Harris, Ed., Plenum Pub. Corp., 1992; POLY (ETHYLENE GLYCOL) CHEMICAL AND BIOLOGICAL APPLICATIONS, J. Milton Harris, Ed., ACS Symposium Series No. 680, American Chemical Society, 1997; Hermanson, BIOCONJUGATE TECHNIQUES, Academic Press, San Diego, 1996; and Dunn et al., Eds. POLYMERIC DRUGS AND DRUG DELIVERY SYSTEMS, ACS Symposium Series Vol. 469, American Chemical Society, Washington, D.C. 1991).

### Linker Groups (Cross-linking Groups)

Preparation of the modified sugar for use in the methods of the present invention includes attachment of a PEG moiety to a sugar residue and preferably, forming a stable adduct, which is a substrate for a glycosyltransferase. Thus, it is often preferred to use a linker, e.g., one formed by reaction of the PEG and sugar moiety with a cross-linking agent to conjugate the PEG and the sugar. Exemplary bifunctional compounds which can be used for attaching modifying groups to carbohydrate moieties include, but are not limited to, bifunctional poly(ethyleneglycols), polyamides, polyethers, polyesters and the like. General approaches for linking carbohydrates to other molecules are known in the literature. *See,* for example, Lee et al., Biochemistry 28: 1856 (1989); Bhatia et al., Anal. Biochem. 178: 408 (1989); Janda et al., J. Am. Chem. Soc. 112: 8886 (1990) and Bednarski et al., WO 92/18135. In the discussion that follows, the reactive groups are treated as benign on the sugar moiety of the nascent modified sugar. The focus of the discussion is for clarity of illustration. Those of skill in the art will appreciate that the discussion is relevant to reactive groups on the modifying group as well.

A variety of reagents are used to modify the components of the modified sugar with intramolecular chemical crosslinks (for reviews of crosslinking reagents and crosslinking procedures see: Wold, F., Meth. Enzymol. 25: 623-651, 1972; Weetall, H. H., and Cooney, D. A., In: ENZYMES AS DRUGS. (Holcenberg, and Roberts, eds.) pp. 395-442, Wiley, New York, 1981; Ji, T. H., Meth. Enzymol. 91: 580-609, 1983; Mattson et al., Mol. Biol. Rep. 17: 167-183, 1993, all of which are incorporated herein by reference). Preferred crosslinking reagents are derived from various zero-length, homo-bifunctional, and hetero-bifunctional crosslinking reagents. Zero-length crosslinking reagents include direct conjugation of two intrinsic chemical groups with no introduction of extrinsic material. Agents that catalyze formation of a disulfide bond belong to this category. Another example is reagents that induce condensation of a carboxyl and a primary amino group to form an amide bond such as carbodiimides, ethylchloroformate, Woodward's reagent K (2-ethyl-5-phenylisoxazolium-3'-sulfonate), and carbonyldiimidazole. In addition to these chemical reagents, the enzyme transglutaminase (glutamyl-peptide γ-glutamyltransferase; EC 2.3.2.13) may be used as zero-length crosslinking reagent. This enzyme catalyzes acyl transfer reactions at carboxamide groups of protein-bound glutaminyl residues, usually with a primary amino group as substrate. Preferred homo- and hetero-bifunctional reagents contain two identical or two dissimilar sites, respectively, which may be reactive for amino, sulfhydryl, guanidino, indole, or nonspecific groups.

### Purification of G-CSF Conjugates

### Refolding insoluble G-CSF

Many recombinant proteins expressed in bacteria are expressed as insoluble aggregates in bacterial inclusion bodies. Inclusion bodies are protein deposits found in both the cytoplasmic and periplasmic space of bacteria. (See, *e.g.,* Clark, Cur. Op. Biotech. 12:202-207 (2001)). Recombinant G-CSF proteins are expressed in bacterial inclusion bodies, and methods for refolding these proteins to produce active G-CSF proteins are provided herein.

### A. Conditions for refolding active G-CSF

To produce active G-CSF proteins from bacterial cells, G-CSF proteins are expressed in bacterial inclusion bodies, the bacteria are harvested, disrupted and the inclusion bodies are isolated and washed. In one embodiment, three washes are performed: a first wash in a buffer at a pH between 6.0 and 9.0; a monovalent salt, *e.g.,* sodium chloride; a nonionic detergent, *e.g.,* Triton X-100; an ionic detergent, *e.g.,* sodium deoxycholate; and EDTA; a second w ash in a detergent free buffer, and a third wash in H₂0. The proteins within the inclusion bodies are then solubilized. Solubilization can be performed using denaturants, guanidiniunl chloride or urea; extremes of pH; or detergents or any combination of these. In one embodiment of 5-6M guanidine HCl or urea are used to solubilize GCSF. In ..mother embodiment, DTT is added.

After solubilization, denaturants are removed from the GCSF protein mixture. Denaturant removal can be done by a variety of methods, including dilution into a refolding buffet- or buffer exchange methods. Buffer exchange methods include dialysis, diafiltration, g.el filtration, and immobilization of the protein onto a solid support. *(See, e.g.,* Clark, Cur. Op. Biotech. 12:202-207 (2001)). Any of the above methods can be combined to remove denaturants.

Disulfide bond formation in the GCSF proteins is promoted by addition of a refolding buffer comprising a redox couple. Redox couples include reduced and oxidized glutathionc ((-JSF-I/GSSG), cysteine/cystine, cysteamine/cystamine, DTT/GSSG, and DTE/GSSG. *(See, e.g.,* Clark, Cur. Op. Biotech. 12:202-207 (2001)). In one embodiment the redox couple is GSH/GSSG at a ratio of 10:1.

Refolding can be performed in buffers at pH's ranging from, for example, 6.0 to 10.0. Refolding buffers can include other additives to enhance refolding, *e.g.,* L-arginine (0.4-1 M); PEG; low concentrations of denaturants, such as urea (1-2M) and guanidinium chloride (0.5-1.5 M); and detergents (e.g., Chaps, SDS, CTAB, lauryl maltoside, Tween 80, and Triton X-100).

Alter refolding, the GCSF protein can be dialyzed to remove the redox couple or other unwanted buffer components. In one embodiment, dialysis is performed using a buffer including sodium acetae, glycerol, and a non-ionic detergent, e.g., Tween-80. After dialysis the GCSF protein can be further purified, and/or concentrated by ion exchange chromatography. In one embodiment, an SP-sepharose cation exchange resin is used.

Those of skill will recognize that a protein has been refolded correctly when the refolded protein has detectable biological activity. For a GCSF protein, biological activity can be measured using a variety of methods. For example, biologically active GCSF proteins are substrates for the O-linked glycosylation described in U.S. Patent Applications 60/535 284, filed January 8, 2004; 60/544411, filed February 12, 2004; and Attorney Docket Number 019957-018820US, filed February 20, 2004; each of which is herein incorporated by reference for all purposes. GCSF protein activity can also be measured using cell proliferation assays or white blood cell (WBC) assays in rats. (Also described in U.S. Patent Applications 60/535284, filed January 8, 2004; 60/544411, filed February 12, 2004; and Attorney Docket Number 019957-018820US, filed February 20, 2004. The proliferation assays and the WBC assays can be done before or after O-linked glycosylation of the refolded GCSF proteins.

### Other Methods for Isolating Conjugates of the Invention

Alternatively, the products produced by the above processes can be used without purification. However, it is usually preferred to recover the product. Standard, well-known techniques for recovery of glycosylated saccharides such as thin or thick layer chromatography, column chromatography, ion exchange chromatography, or membrane filtration can be used. It is preferred to use membrane filtration, more preferably utilizing a reverse osmotic membrane, or one or more column chromatographic techniques for the recovery as is discussed hereinafter and in the literature cited herein. For instance, membrane filtration wherein the membranes have molecular weight cutoff of about 3000 to about 10,000 can be used to remove proteins such as glycosyl transferases. Nanofiltration or reverse osmosis can then be used to remove salts and/or purify the product saccharides (*see, e.g.,* WO 98/15581). Nanofilter membranes are a class of reverse osmosis membranes that pass monovalent salts but retain polyvalent salts and uncharged solutes larger than about 100 to about 2,000 Daltons, depending upon the membrane used. Thus, in a typical application, saccharides prepared by the methods of the present invention will be retained in the membrane and contaminating salts will pass through.

If the modified glycoprotein is produced intracellularly, as a first step, the particulate debris, either host cells or lysed fragments, is removed, for example, by centrifugation or ultrafiltration; optionally, the protein may be concentrated with a commercially available protein concentration filter, followed by separating the polypeptide variant from other impurities by one or more steps selected from immunoaffinity chromatography, ion-exchange column fractionation (e.g., on diethylaminoethyl (DEAE) or matrices containing carboxymethyl or sulfopropyl groups), chromatography on Blue-Sepharose, CM Blue-Sepharose, MONO-Q, MONO-S, lentil lectin-Sepharose, WGA-Sepharose, Con A-Sepharose, Ether Toyopearl, Butyl Toyopearl, Phenyl Toyopearl, or protein A Sepharose, SDS-PAGE chromatography, silica chromatography, chromatofocusing, reverse phase HPLC (e.g., silica gel with appended aliphatic groups), gel filtration using, *e.g.,* Sephadex molecular sieve or size-exclusion chromatography, chromatography on columns that selectively bind the polypeptide, and ethanol or ammonium sulfate precipitation.

Modified glycopeptides produced in culture are usually isolated by initial extraction from cells, enzymes, etc., followed by one or more concentration, salting-out, aqueous ion-exchange, or size-exclusion chromatography steps. Additionally, the modified glycoprotein may be purified by affinity chromatography. Finally, HPLC may be employed for final purification steps.

A protease inhibitor, *e.g.,* methylsulfonylfluoride (PMSF) may be included in any of the foregoing steps to inhibit proteolysis and antibiotics may be included to prevent the growth of adventitious contaminants.

Within another embodiment, supernatants from systems which produce the modified glycopeptide of the invention are first concentrated using a commercially available protein concentration filter, for example, an Amicon or Millipore Pellicon ultrafiltration unit. Following the concentration step, the concentrate may be applied to a suitable purification matrix. For example, a suitable affinity matrix may comprise a ligand for the peptide, a lectin or antibody molecule bound to a suitable support. Alternatively, an anion-exchange resin may be employed, for example, a matrix or substrate having pendant DEAE groups. Suitable matrices include acrylamide, agarose, dextran, cellulose, or other types commonly employed in protein purification. Alternatively, a cation-exchange step may be employed. Suitable cation exchangers include various insoluble matrices comprising sulfopropyl or carboxymethyl groups. Sulfopropyl groups are particularly preferred.

Finally, one or more RP-HPLC steps employing hydrophobic RP-HPLC media, e.g., silica gel having pendant methyl or other aliphatic groups, may be employed to further purify a polypeptide variant composition. Some or all of the foregoing purification steps, in various combinations, can also be employed to provide a homogeneous modified glycoprotein.

The modified glycopeptide of the invention resulting from a large-scale fermentation may be purified by methods analogous to those disclosed by Urdal et al., J. Chromatog. 296: 171 (1984). This reference describes two sequential, RP-HPLC steps for purification of recombinant human IL-2 on a preparative HPLC column. Alternatively, techniques such as affinity chromatography may be utilized to purify the modified glycoprotein.

### Pharmaceutical Compositions

In another aspect, the invention provides a pharmaceutical composition. The pharmaceutical composition includes a pharmaceutically acceptable diluent and a covalent conjugate between a non-naturally-occurring, PEG moiety, therapeutic moiety or biomolecule and a glycosylated or non-glycosylated peptide. The polymer, therapeutic moiety or biomolecule is conjugated to the G-CSF peptide via an intact glycosyl linking group interposed between and covalently linked to both the G-CSF peptide and the polymer, therapeutic moiety or biomolecule.

Pharmaceutical compositions of the invention are suitable for use in a variety of drug delivery systems. Suitable formulations for use in the present invention are found in Remington's Pharmaceutical Sciences, Mace Publishing Company, Philadelphia, PA, 17th ed. (1985). For a brief review of methods for drug delivery, *see,* Langer, Science 249:1527-1533 (1990).

The pharmaceutical compositions may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g*., polylactate polyglycolate) may also be employed as carriers for the pharmaceutical compositions of this invention. Suitable biodegradable microspheres are disclosed, for example, in U.S. Patent Nos. 4,897,268 and 5,075,109.

Commonly, the pharmaceutical compositions are administered parenterally, *e.g.,* intravenously. Thus, the invention provides compositions for parenteral administration which comprise the compound dissolved or suspended in an acceptable carrier, preferably an aqueous carrier, *e.g.,* water, buffered water, saline, PBS and the like. The compositions may contain pharmaceutically acceptable auxiliary substances as required to approximate physiological conditions, such as pH adjusting and buffering agents, tonicity adjusting agents, wetting agents, detergents and the like.

These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably from 5 to 9 and most preferably from 7 and 8.

In some embodiments the glycopeptides of the invention can be incorporated into liposomes formed from standard vesicle-forming lipids. A variety of methods are available for preparing liposomes, as described in, *e.g.,* Szoka et al., Ann. Rev. Biophys. Bioeng. 9: 467 (1980), U.S. Pat. Nos. 4,235,871, 4,501,728 and 4,837,028. The targeting of liposomes using a variety of targeting agents (*e.g*., the sialyl galactosides of the invention) is well known in the art (*see, e.g.,* U.S. Patent Nos. 4,957,773 and 4,603,044).

Standard methods for coupling targeting agents to liposomes can be used. These methods generally involve incorporation into liposomes of lipid components, such as phosphatidylethanolamine, which can be activated for attachment of targeting agents, or derivatized lipophilic compounds, such as lipid-derivatized glycopeptides of the invention.

Targeting mechanisms generally require that the targeting agents be positioned on the surface of the liposome in such a manner that the target moieties are available for interaction with the target, for example, a cell surface receptor. The carbohydrates of the invention may be attached to a lipid molecule before the liposome is formed using methods known to those of skill in the art (e.g., alkylation or acylation of a hydroxyl group present on the carbohydrate with a long chain alkyl halide or with a fatty acid, respectively). Alternatively, the liposome may be fashioned in such a way that a connector portion is first incorporated into the membrane at the time of forming the membrane. The connector portion must have a lipophilic portion, which is firmly embedded and anchored in the membrane. It must also have a reactive portion, which is chemically available on the aqueous surface of the liposome. The reactive portion is selected so that it will be chemically suitable to form a stable chemical bond with the targeting agent or carbohydrate, which is added later. In some cases it is possible to attach the target agent to the connector molecule directly, but in most instances it is more suitable to use a third molecule to act as a chemical bridge, thus linking the connector molecule which is in the membrane with the target agent or carbohydrate which is extended, three dimensionally, off of the vesicle surface.

The compounds prepared by the methods of the invention may also find use as diagnostic reagents. For example, labeled compounds can be used to locate areas of inflammation or tumor metastasis in a patient suspected of having an inflammation. For this use, the compounds can be labeled with ¹²⁵I, ¹⁴C, or tritium.

The active ingredient used in the pharmaceutical compositions of the present invention is glycopegylated G-CSF and its derivatives having the biological properties of Follicle Stimulating Hormone to increase e.g., ovulation. Preferably, the G-CSF composition of the present invention is administered parenterally (e.g. IV, IM, SC or IP). Effective dosages are expected to vary considerably depending on the condition being treated and the route of administration but are expected to be in the range of about 0.1 (∼7U) to 100 (∼7000U) µg/kg body weight of the active material. Preferable doses for treatment of anemic conditions are about 50 to about 300 Units/kg three times a week. Because the present invention provides an G-CSF with an enhanced *in vivo* residence time, the stated dosages are optionally lowered when a composition of the invention is administered.

The following examples are provided to illustrate the conjugates, and methods and of the present invention.

### EXAMPLES

### EXAMPLE 1

### GlycoPEGylation of G-CSF produced in CHO cells

### a. Preparation of Asialo-Granulocyte-Colony Stimulation Factor (G-CSF)

G-CSF produced in CHO cells is dissolved at 2.5 mg/mL in 50 mM Tris 50 mM Tris-HCl pH 7.4, 0.15 M NaCl, 5 mM CaCl₂ and concentrated to 500 µL in a Centricon Plus 20 centrifugal filter. The solution is incubated with 300 mU/mL Neuraminidase II *(Vibrio cholerae)* for 16 hours at 32 °C. To monitor the reaction a small aliquot of the reaction is diluted with the appropriate buffer and a IEF gel performed. The reaction mixture is then added to prewashed N-(p-aminophenyl)oxamic acid-agarose conjugate (800 µL/mL reaction volume) and the washed beads gently rotated for 24 hours at 4 °C. The mixture is centrifuged at 10,000 rpm and the supernatant was collected. The beads are washed 3 times with Tris-EDTA buffer, once with 0.4 mL Tris-EDTA buffer and once with 0.2 mL of the Tris-EDTA buffer and all supernatants are pooled. The supernatant is dialyzed at 4 °C against 50 mM Tris -HCl pH 7.4, 1 M NaCl, 0.05% NaN₃ and then twice more against 50 mM Tris -HCl pH 7.4, 1 M NaCl, 0.05% NaN₃. The dialyzed solution is then concentrated using a Centricon Plus 20 centrifugal filter and stored at -20 °C. The conditions for the IEF gel were run according to the procedures and reagents provided by Invitrogen. Samples of native and desialylated G-CSF are dialyzed against water and analyzed by MALDI-TOF MS.

### b. Preparation of G-CSF-(alpha2,3)-Sialyl-PEG

Desialylated G-CSF was dissolved at 2.5 mg/mL in 50 mM Tris-HCl, 0.15 M NaCl, 0.05% NaN₃, pH 7.2. The solution is incubated with 1 mM CMP-sialic acid-PEG and 0.1 U/mL of ST3Gall at 32°C for 2 days. To monitor the incorporation of sialic acid-PEG, a small aliquot of the reaction had CMP-SA-PEG-fluorescent ligand added; the label incorporated into the peptide is separated from the free label by gel filtration on a Toso Haas G3000SW analytical column using PBS buffer (pH 7.1). The fluorescent label incorporation into the peptide is quantitated using an in-line fluorescent detector. After 2 days, the reaction mixture is purified using a Toso Haas G3000SW preparative column using PBS buffer (pH 7.1) and collecting fractions based on UV absorption. The product of the reaction is analyzed using SDS-PAGE and IEF analysis according to the procedures and reagents supplied by Invitrogen. Samples of native and PEGylated G-CSF are dialyzed against water and analyzed by MALDI-TOF MS.

### c. Preparation of G-CSF-(alpha2,8)-Sialyl-PEG

G-CSF produced in CHO cells, which contains an alpha2,3-sialylated O-linked glycan, is dissolved at 2.5 mg/mL in 50 mM Tris-HCl, 0.15 M NaCl, 0.05% NaN₃, pH 7.2. The solution is incubated with 1 mM CMP-sialic acid-PEG and 0.1 U/mL of CST-II at 32°C for 2 days. To monitor the incorporation of sialic acid-PEG, a small aliquot of the reaction has CMP-SA-PEG-fluorescent ligand added; the label incorporated into the peptide is separated from the free label by gel filtration on a Toso Haas G3000SW analytical column using PBS buffer (pH 7.1). The fluorescent label incorporation into the peptide is quantitated using an in-line fluorescent detector. After 2 days, the reaction mixture is purified using a Toso Haas G3000SW preparative column using PBS buffer (pH 7.1) and collecting fractions based on UV absorption. The product of the reaction is analyzed using SDS-PAGE and IEF analysis according to the procedures and reagents supplied by Invitrogen. Samples of native and PEGylated G-CSF are dialyzed against water and analyzed by MALDI-TOF MS.

### d. Preparation of G-CSF-(alpha2,6)-Sialyl-PEG

G-CSF, containing only O-linked GalNAc, is dissolved at 2.5 mg/mL in 50 mM Tris-HCl, 0.15 M NaCl, 0.05% NaN₃, pH 7.2. The solution is incubated with 1 mM CMP-sialic acid-PEG and 0.1 U/mL of ST6GalNAcI or II at 32°C for 2 days. To monitor the incorporation of sialic acid-PEG, a small aliquot of the reaction has CMP-SA-PEG-fluorescent ligand added; the label incorporated into the peptide is separated from the free label by gel filtration on a Toso Haas G3000SW analytical column using PBS buffer (pH 7.1). The fluorescent label incorporation into the peptide is quantitated using an in-line fluorescent detector. After 2 days, the reaction mixture is purified using a Toso Haas G3000SW preparative column using PBS buffer (pH 7.1) and collecting fractions based on UV absorption. The product of the reaction is analyzed using SDS-PAGE and IEF analysis according to the procedures and reagents supplied by Invitrogen. Samples of native and PEGylated G-CSF are dialyzed against water and analyzed by MALDI-TOF MS.

G-CSF produced in CHO cells was treated with Arthrobacter sialidase and was then purified by size exclusion on Superdex 75 and was treated with ST3Gal1 or ST3 Gal2 and then with CMP-SA-PEG 20Kda. The resulting molecule was purified by ion exchange and gel filtration and analysis by SDS PAGE demonstrated that the PEGylation was complete. This is the first demonstration of glycoPEGylation of an O-linked glycan.

### EXAMPLE 2

### Recombinant GCSF - Expression, refolding and purification

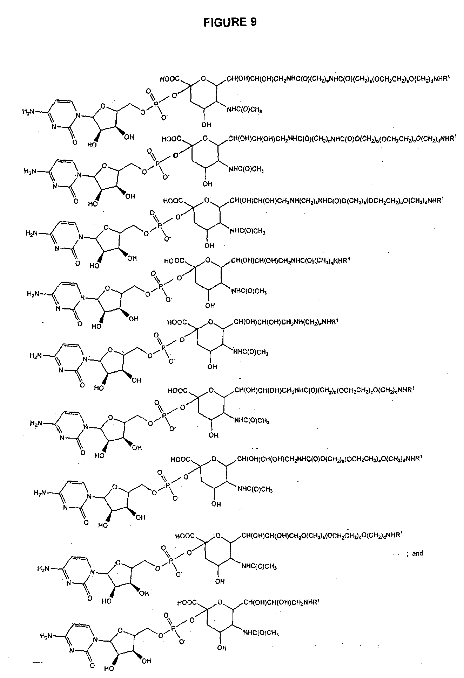

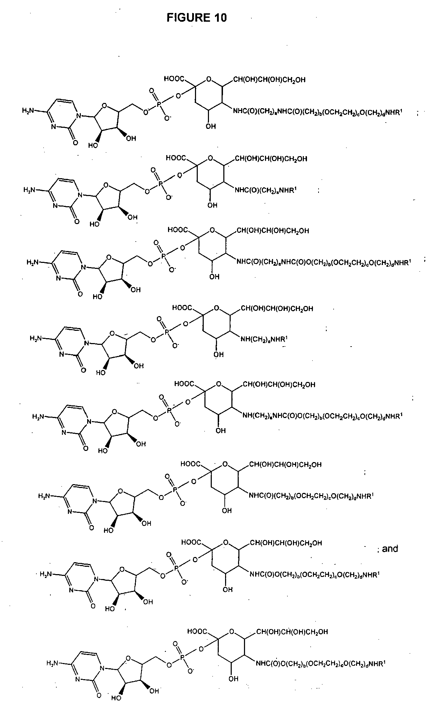

● Harvest cells by centrifugation, discard supernatant. Results of growth on various media are shown in Figure 9.
● Resuspend cell pellet in 10mM Tris pH7.4, 75mM NaCl, 5mM EDTA - use 10ml/g (lysis buffer)
● Microlluidize cells (French press works as well)
● Centrifuge 30min, 4°C at 5,000RPM-discard supernatant
● Resuspend pellet in lysis buffer and centrifuge as above
● Wash IB's in 25mM Tris pH8, 100mM NaCl, 1%TX-100, 1% NaDOC, 5mM EDTA. Pellets are resuspended by pipetting and vortexing. Centrifuge 15min 4°C 5,000RPM. Repeat this step once more (total of two washes)
● Wash pellets two times in 25mM Tris pH8, 100mM NaCl, 5mM EDTA to remove detergents, centrifuge as above.
● Resuspend pellets in dH2O to aliquot and centrifuge as above. Pellets are frozen at - 20C
● IB's are resuspended at 20mg/ml in 6M guanidineHCl, 5mM EDTA, 100mM NaCl, 100mM Tris pH8, 10mM DTT using a pipettor, followed by rotation for 2-4h at room temperature.
● Centrifuge solubilized IB's for 1min at room temperature at 14,000RPM. Save supernatant.
● Dilute supernatant 1:20 with refold buffer 50mM MES pH6, 240mM NaCl, 10mM
● KCl, 0.3mM lauryl maltoside, 0.055% PEG3350, 1mM GSH, 0.1M GSSG, 0.5M arginine and refold on rotator overnight at 4°C.
● Transfer refold to Pierce snakeskin 7kDa MWCO for dialysis. Dialysis buffer 20mM NaOAc pH4, 50mM NaCl, 0.005% Tween-80, 0.1mM EDTA. Dialyze a total of 3 times versus at least a 200 fold excess at 4°C.
● After dialysis pass material through a 0.45µM filter.
● Equlibrate SP-sepharose column with the dialysis buffer and apply sample. Wash column with dialysis buffer and elute with dialysis buffer containing a salt gradient up to 1M NaCl. Protein typically is eluted at 300-400mM NaCl.
● Check material on SDS-PAGE (see e.g., Figure 10).

### EXAMPLE 3

### The Two Enzyme Method in Two Pots

The following example illustrates the preparation of G-CSF-GalNAc-SA-PEG in two sequential steps wherein each intermediate product is purified before it is used in the next step.

### a. Preparation of G-CSF-GalNAc (pH 6.2) from G-CSF and UDP-GalNAc using GalNAc-T2.

G-CSF (960 mcg) in 3.2 mL of packaged buffer was concentrated by utrafiltration using an UF filter (MWCO 5K) and then reconstituted with 1 mL of 25 mM MES buffer (pH 6.2, 0.005% NaN₃). UDP-GalNAc (6 mg, 9.24 mM), GaINAc-T2 (40 µL, 0.04 U), and 100 mM MnCl₂ (40 µL, 4 mM) were then added and the resulting solution was incubated at room temperature.

After 24 hrs, MALDI indicated the reaction was complete. The reaction mixture was directly subjected to HPLC purification using SEC (Superdex 75 and Superdex 200) and an elution buffer comprising of PBS (phosphate buffered saline, pH 4.9 and 0.005% Tween 80). The collected peak of G-CSF-GalNAc was concentrated using a Centricon 5 KDa MWCO filter to about 150 µL and the volume adjusted to Iml using PBS (phosphate buffered saline, pH 4.9 and 0.005% Tween 80). Final protein concentration 1 mg/mL (A₂₈₀), yield 100%. The sample was stored at 4 °C.

### b. Preparation of G-CSF-GalNAc-SA-PEG using purified G-CSF-GalNAc, CMP-SA-PEG (20KDa) and mouse ST6GalNAc-TI (pH 6.2).

The G-CSF-GalNAc solution containing 1 mg of protein was buffer exchanged into 25 mM MES buffer (pH 6.2, 0.005% NaN₃) and CMP-SA-PEG (20KDa) (5 mg, 0.25 umol) was added. After dissolving, Munch (100 mcL, 100 mM solution) and ST6GalNAc-I (100 mcL, mouse enzyme) was added and the reaction mixture rocked slowly at 32 °C for three days. The reaction mixture was concentrated by ultrifiltration (MWCO 5K) and buffer exchanged with 25 mM NaOAc (pH 4.9) one time and then concentrated to 1 mL of total volume. The product was then purified using SP-sepharose (A: 25 mM NaOAc+0.005% tween-80 pH 4.5; B: 25 mM NaOAc+0.005% tween-80 pH 4.5+2M NaCl) at retention time 13-18 mins and SEC (Superdex 75; PBS-pH 7.2, 0.005% Tween 80) at retention time 8.6 mins (superdex 75, flow I ml/min) The desired fractions were collected, concentrated to 0.5 mL and stored at 4 °C.

### EXAMPLE 4

### One Pot Method to Make G-CSF-GalNAc-SA-PEG with Simultaneous Addition of Enzymes

The following example illustrates the preparation of G-CSF-GalNAc -SA-PEG in one pot using simultaneous addition of enzymes

### 1. One Pot process using mouse ST6GalNAc-I (pH 6.0).

G-CSF (960 µg of protein dissolved in 3.2 mL of the product formulation buffer) was concentrated by ultrafiltration (MWCO 5K) to 0.5 ml and reconstituted with 25 mM MES buffer (pH 6.0, 0.005% NaN₃) to a total volume of about 1 mL or a protein concentration of 1 mg/mL. UDP-GalNAc (6 mg, 9.21 µmol), GalNAc-T2 (80 µL, 80 mU), CMP-SA-PEG (20KDa) (6 mg, 0,3 µmol) and mouse enzyme ST6GalNAc-I (120 µL) and 100 mM MnCl₂(50 µL) were then added. The solution was rocked at 32°C for 48 hrs and purified using standard chromatography conditions on SP-sepharose. A total of 0.5 mg of protein (A₂₈₀) was obtained or about a 50% overall yield. The product structure was confirmed by analysis with both MALDI and SDS-PAGE.

### 2. One pot process using chicken ST6GalNAc-I (pH 6.0).

14.4 mg of G-CSF; was concentrated to 3 mL final volume, buffer exchanged with 25 mM MES buffer (pH 6.0, 0.05% NaN₃, 0.004% Tween 80) and the volume was adjusted to 13 mL. The UDP-GalNAc (90 mg, 150 µmole), GalNAc-T2 (0.59 U), CMP-SA-PEG-20KDa (90 mg), chicken ST6GalNAc-I (0.44 U), and 100 mM MnCl₂ (600 mcL) were then added. The resulting mixture stood at room temperature for 60 hrs. The reaction mixture was then concentrated using a UF (MWCO 5K) and centrifugation. The residue (about 2 mL) was dissolved in 25 mM NaOAc buffer (pH 4.5) and concentrated again to 5 mL final volume. This sample was purified using SP-sepharose for about 10-23 min, SEC (Superdex 75, 17 min, flow rate 0.5 ml/min) and an additional SEC (Superdex 200, 23 min, flow rate 0.5 ml/min), to yield 3.6 mg (25% overall yield) of G-CSF-GalNAc-SA-PEG-20 KDa (A₂₈₀ and BCA method).

### EXAMPLE 5

### One Pot Method to Make G-CSF-GalNAc-Gal-SA-PEG with Sequential Addition of Enzymes

The following example illustrates a method for making G-CSF-GalNAc-Gal-SA-PEG in one pot with sequential addition of enzymes. '

### 1. Starting from GalNAc-G-CSF

### a. Preparation of G-CSF-GalNAc (pH 6.2) from G-CSF and UDP-GalNAc using GalNAc-T2.

G-CSF (960 mcg) in 3.2 mL of packaged buffer was concentrated by utrafiltration using an UF filter (MWCO 5K) and then reconstituted with 1 mL of 25 mM MES buffer (pH 6.2, 0.005% NaN₃). UDP-GalNAc (6 mg, 9.24 mM), GalNAc-T2 (40 µL, 0.04 U), and 100 mM MnCl₂ (40 µL, 4 mM) were then added and the resulting solution was incubated at room temperature.

### b. Preparation of G-CSF-GalNAc-Gal-SA-PEG from G-CSF-GalNAc; UDP-Galactose, SA-PEG-20Kdalton, and the Appropriate Enzymes

The UDP-Galactose (4 mg, 6.5 µmoles), core-1-Gal-T (320 µL, 160 mU), CMP-SA-PEG-20KDa (8 mg, 0.4 µmole), ST3Gal2 (80 µL, 0.07 mU) and 100 mM MnCl₂( 80 µL) were directly added to the crude reaction mixture of the G-CSF-GalNAc (1.5 mg) in 1.5 ml 25 mM MES buffer (pH 6.0) from step a, above. The resulting mixture was incubated at 32°C for 60 hrs. The reaction mixture was centrifuged and the solution was concentrated using ultrafiltration (MWCO 5K) to 0.2 mL, and then redissolved with 25 mM NaOAc (pH 4.5) to a final volume of 1 mL. The product was purified using SP-sepharose (retention time of between 10-15 min), the peak fraction were concentrated using a spin filter (MWCO 5K) and the residue purified further using SEC (Superdex 75, retention time of 10.2 min). After concentration using a spin filter (MWCO 5K), the protein was diluted to 1 mL using formulation buffer with PBS, 2.5% mannitol, 0.005% polysorbate, pH 6.5 and formulated at a protein concentration of 850 mcg protein per mL (A₂₈₀). The overall yield was 55%.

### EXAMPLE 6

### One Pot Method to Make G-CSF-GalNAc-Gal-SA-PEG with Simultaneous Addition of Enzymes

### a. Sfarting from G-CSF.

G-CSF (960 mcg, 3.2 ml) was concentrated by ultrafiltration (MWCO 5K) and reconstituted with 25 mM Mes buffer (pH 6.0, 0.005% NaN₃). The total volume of the G-CSF solution was about 1 mg/ml. UDP-GalNAc (6 mg), GalNAc-T2 (80 µL, ∼80 µU), UDP-Gal (6 mg), Corel GalT (160 µL, 80 µU), CMP-SA-PEG(20K) (6 mg) and a 2,3-(O)-sialyltransferase (160 µL, 120 µU 100 mM MnCl₂ (40 µL) were added. The resulting mixture was incubated at 32°C for 48 h. Purification was performed as described below using IEX and SEC. The resulting fraction containing the product were concentrated using ultrafiltration (MWCO 5K) and the volume was adjusted to about 1 mL with buffer. The protein concentration was determined to be 0.392 mg/ml by A280, giving an overall yield of 40% from G-CSF.

## Claims

1. A Granulocyte Colony Stimulating Factor peptide comprising the moiety: 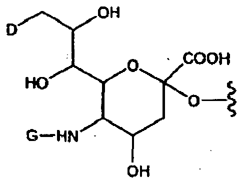 wherein
D is a member selected from -OH and R¹-L-HN-;
G is a member selected from R¹-L- and -C(O)(C₁-C₆)alkyl;
R¹ has a structure that is a member selected from: 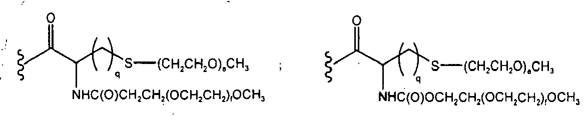 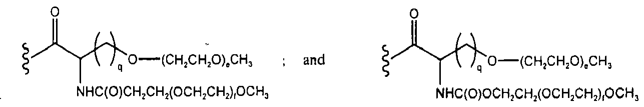 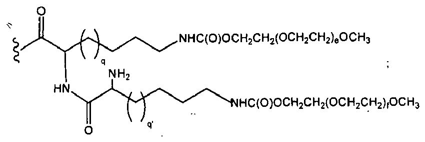 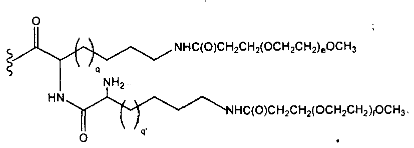 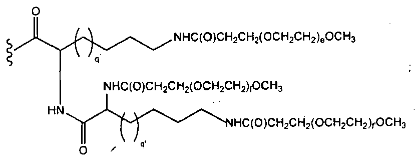 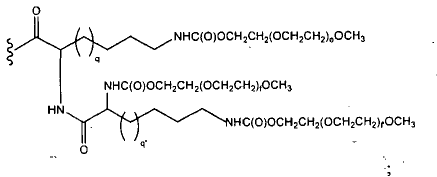 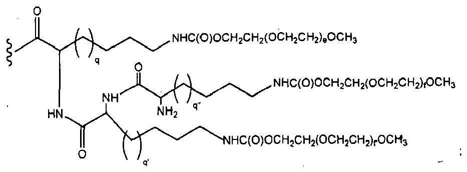 and 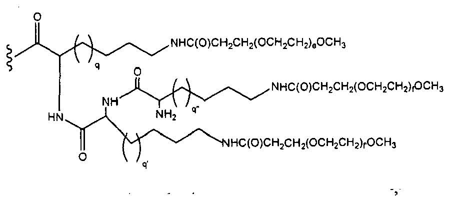 wherein
e, fand f are integers independently selected from 1 to 2500; and
q, q' and q" are integers independently selected from 1 to 20; and
L is a linker which is a member selected from a bond, substituted or unsubstituted alkyl and substituted or unsubstituted heteroalkyl,
such that when D is OH, G is R¹-L-, and when G is -C(O)(C₁-C₆)alkyl, D is R¹-L-NH-.

2. The peptide according to claim 1, wherein L-R¹ has the formula: 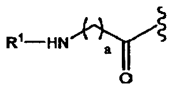 wherein
a is an integer from 0 to 20.

3. The peptide according to any preceding claim, wherein said moiety has the formula:

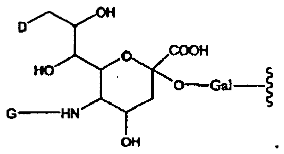

4. The G-CSF peptide according to any of claims 1 to 3, wherein said moiety has the formula:

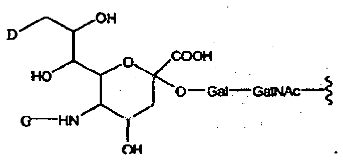

5. The G-CSF peptide according to any of claims 1 to 4, wherein said moiety has the formula: 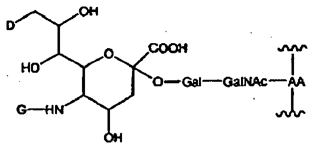 wherein
AA is an amino acid residue of said peptide.

6. The peptide according to claim 5, wherein said amino acid residue is serine or threonine.

7. The peptide according to claim 5 or claim 6, which comprises the amino acid sequence of SEQ. ID. NO: 1.

8. The peptide according to claim 7, wherein said amino acid residue is threonine at position 133 of SEQ. ID. NO: 1.

9. The peptide according any preceding claim, which comprises an amino acid sequence selected from SEQ. ID. NO: 1 and SEQ ID NO: 2.

10. The peptide according to any preceding claim, which comprises a moiety of the formula: 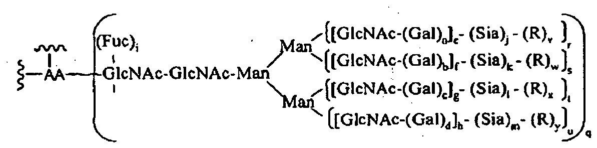 wherein
a, b, c, d, i, r, s, t, and u are integers independently selected from 0 and 1; q is 1;
e, f, g, and h are members independently selected from the integers from 0 to 6;
j, k, 1, and m are members independently selected from the integers from 0 and 100;
v, w, x, and y are independently selected from 0 and 1, and least one of v, w, x and y is 1;
AA is an amino acid residue of said peptide;
Sia-(R) is a moiety of the formula:

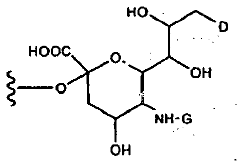

11. The peptide according to claim 10, wherein said amino acid residue is an asparagine residue.

12. The peptide according to any.preceding claim, wherein said peptide is a bioactive Granulocyte Colony Stimulating Factor peptide.

13. A method of making a G-CSF peptide conjugate comprising the moiety: 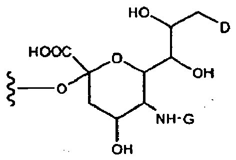 wherein
D is a member selected from -OH and R¹-L-HN-;
G is a member selected from R¹-L- and -C(O)(C₁-C₆)alkyl;
R¹ has a structure that is a member selected from: 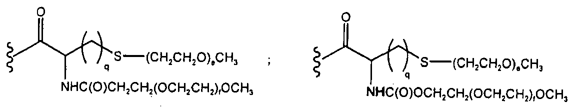 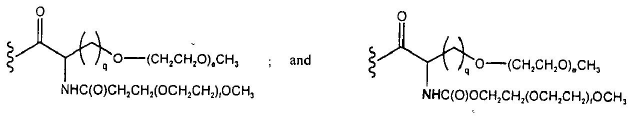 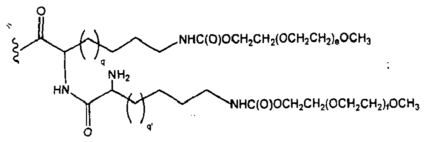 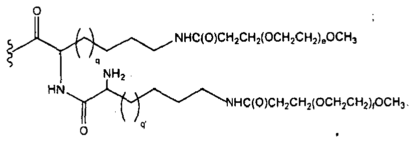 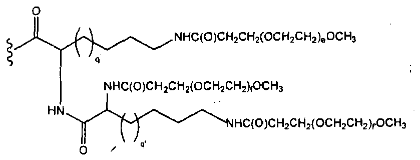 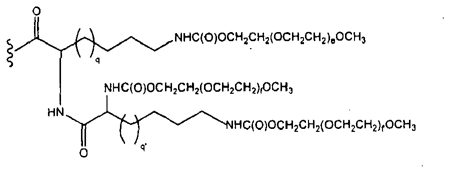 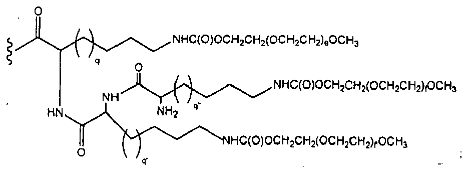 and 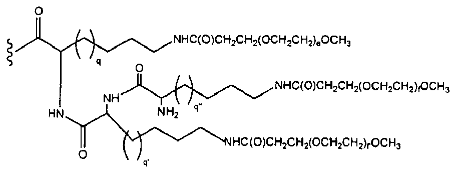 wherein
e, f and f' are integers independently selected from 1 to 2500; and
q, q' and q" are integers independently selected from 1 to 20; and
L is a linker which is a member selected from a bond, substituted or unsubstituted alkyl and substituted or unsubstituted heteroalkyl,
such that when D is OH, G is R¹-L-, and when G is -C(O)(C₁-C₆)alkyl, D is R¹-L-NH-, said method comprising:
(a) contacting a substrate G-CSF peptide with a PEG-sialic acid donor moiety having the formula: 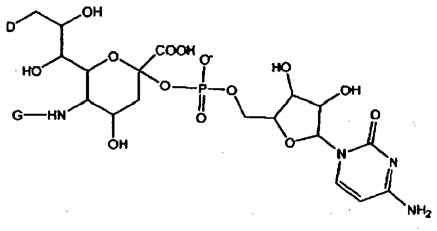
and an enzyme that transfers said PEG-sialic acid onto an amino acid or glycosyl residue of said G-CSF peptide, under conditions appropriate for the transfer.

14. The method according to claim 13, wherein L-R¹ has the formula: 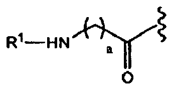 wherein
a is an integer from 0 to 20.

15. The method of claim 13 or 14, further comprising, prior to step (a):
(b) expressing said substrate Granulocyte Colony Stimulating Factor peptide in a suitable host.

16. The method of claim 15, wherein said host is selected from an insect cell and a mammalian cell.

17. The method of any of claims 13 to 16, which further comprises formulating the peptide into a pharmaceutical formulation.

18. Use of a peptide according to any of claims 1 to 12, for the manufacture of a medicament for stimulating inflammatory leukocyte production in a mammal.

19. Use of a peptide according to any of claims 1 to 12, for the manufacture of a medicament for treating infection.

20. A pharmaceutical formulation comprising a peptide according to any of claims 1 to 12 and a pharmaceutically acceptable carrier.

21. A peptide according to any of claims 1 to 12, for therapeutic use.

## Patentansprüche

1. Granulozyten-Kolonie stimulierender Faktor-Peptid umfassend die Gruppe: 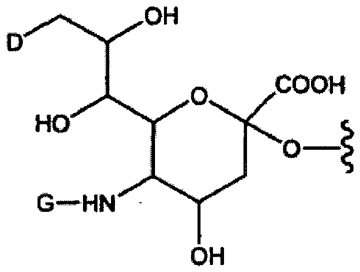 wobei
D ein Element ausgewählt aus -OH und R¹-L-HN- ist;
G ein Element ausgewählt aus R¹-L- und -C(O)(C₁-C₆)Alkyl ist;
R¹ eine Struktur aufweist, die ein Element ist ausgewählt aus: 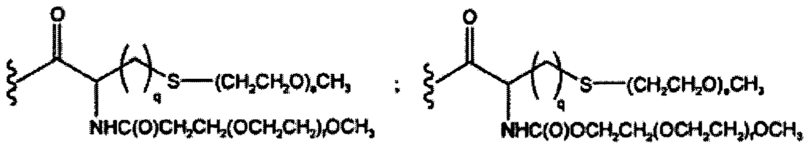 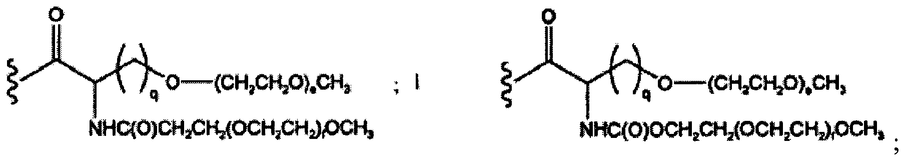 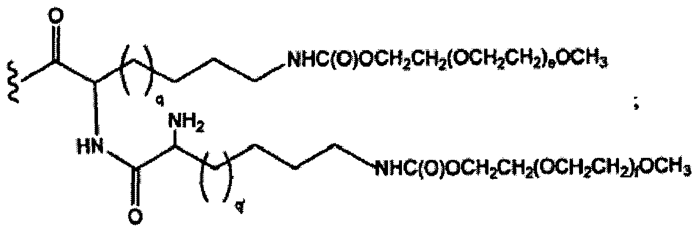 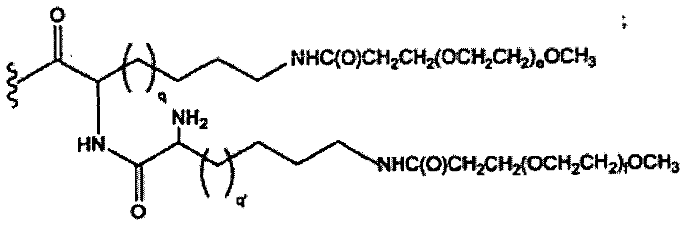 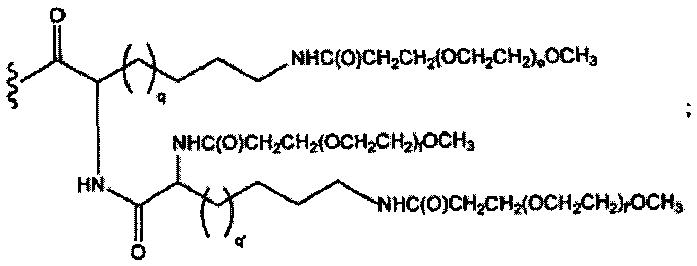 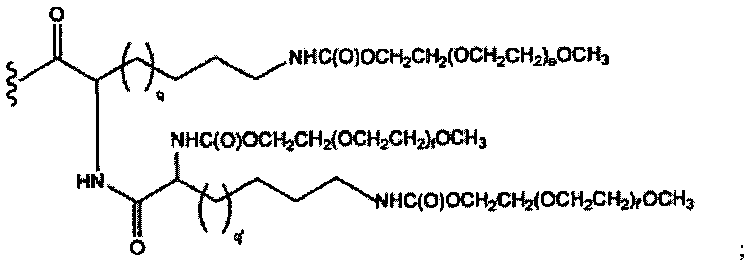 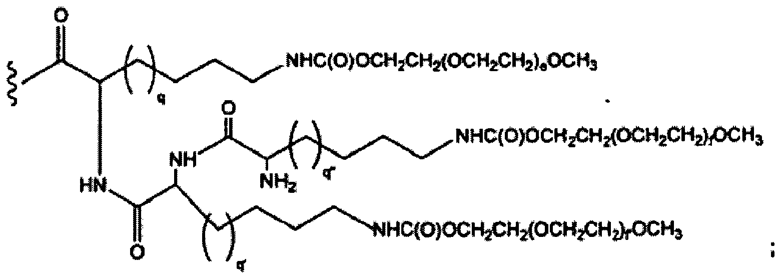 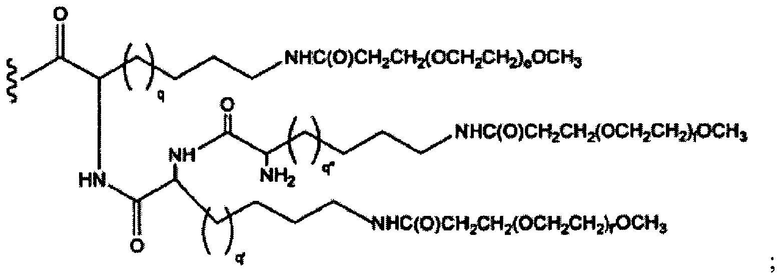 wobei
e, f und f' Ganzzahlen unabhängig ausgewählt aus 1 bis 2500 sind; und
q, q' und q" Ganzzahlen unabhängig ausgewählt aus 1 bis 20 sind; und
L ein Linker ist, der ein Element ausgewählt aus einer Bindung, substituiertem oder unsubstituiertem Alkyl und substituiertem oder unsubstituiertem Heteroalkyl ist,
sodass wenn D gleich OH dann G gleich R¹-L- ist, und wenn G gleich -C(O)(C₁-C₆)Alkyl dann D gleich R¹-L-NH- ist.

2. Peptid gemäß Anspruch 1, wobei L-R¹ die Formel aufweist: 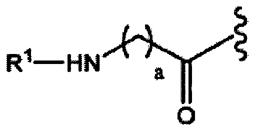 wobei
a eine Ganzzahl von 0 bis 20 ist.

3. Peptid gemäß einem der vorangehenden Ansprüche, wobei genannte Gruppe die Formel hat:

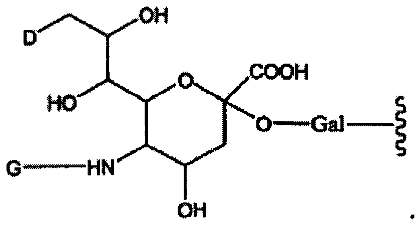

4. G-CSF Peptid gemäß einem der Ansprüche 1 bis 3, wobei genannte Gruppe die Formel aufweist:

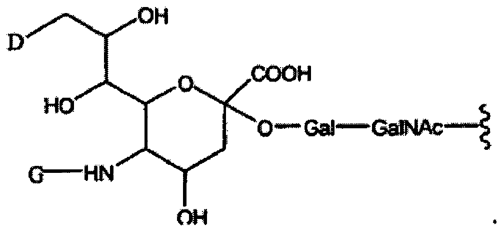

5. G-CSF Peptid gemäß einem der Ansprüche 1 bis 4, wobei genannte Gruppe die Formel aufweist: 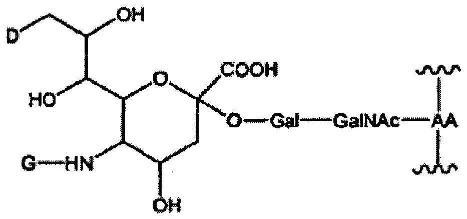 wobei
AA ein Aminosäurerest des genannten Peptids ist.

6. Peptid gemäß Anspruch 5, wobei genannter Aminosäurerest Serin oder Threonin ist.

7. Peptid gemäß Anspruch 5 oder Anspruch 6, das die Aminosäuresequenz von SEQ. ID. NR: 1 umfasst.

8. Peptid gemäß Anspruch 7, wobei genannter Aminosäurerest Threonin an Position 133 3 von SEQ. ID. NR: 1 ist.

9. Peptid gemäß einem der vorangehenden Ansprüche, das eine Aminosäuresequenz ausgewählt von SEQ. ID. NR: 1 und SEQ ID NR: 2 umfasst.

10. Peptid gemäß einem der vorangehenden Ansprüche, das eine Gruppe der Formel umfasst: 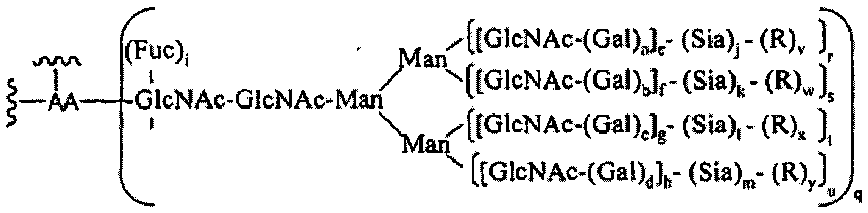 wobei
a, b, c, d, i, r, s, t, und u Ganzzahlen unabhängig ausgewählt aus 0 und 1 sind; q gleich 1 ist;
e, f, g, und h Elemente unabhängig ausgewählt aus den Ganzzahlen aus 0 bis 6 sind;
j, k, l, und m Elemente unabhängig ausgewählt aus den Ganzzahlen aus 0 und 100 sind;
v, w, x, und y unabhängig ausgewählt sind aus 0 und 1, und mindestens einer von v, w, x und y gleich 1 ist;
AA ein Aminosäurerest des genannten Peptids ist;
Sia-(R) eine Gruppe der Formel ist:

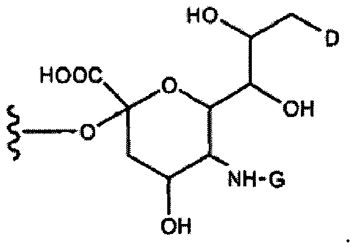

11. Peptid gemäß Anspruch 10, wobei genannter Aminosäurerest ein Asparaginrest ist.

12. Peptid gemäß einem der vorangehenden Ansprüche wobei genanntes Peptid ein bioaktives Granulozyten-Kolonie stimulierender Faktor-Peptid ist.

13. Verfahren zur Herstellung eines G-CSF Peptid-Konjugates umfassend die Gruppe: 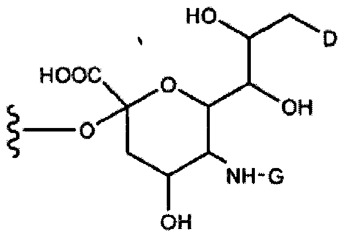 wobei
D ein Element ausgewählt aus -OH und R¹-L-HN- ist;
G ein Element ausgewählt aus R¹-L- und -C(O)(C₁-C₆)Alkyl ist;
R¹ eine Struktur aufweist, die ein Element ist ausgewählt aus: 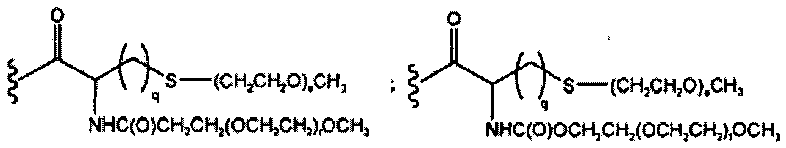 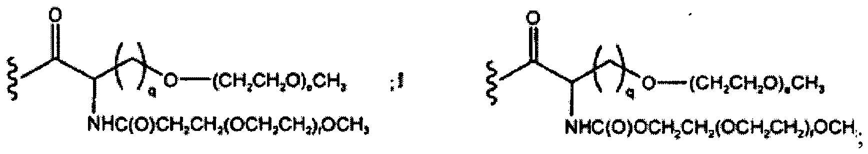 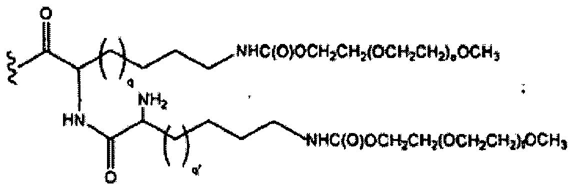 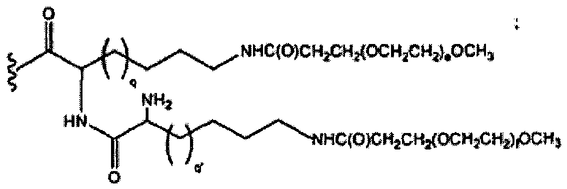 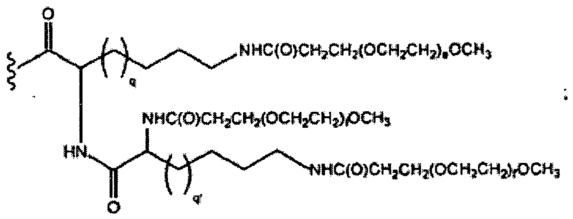 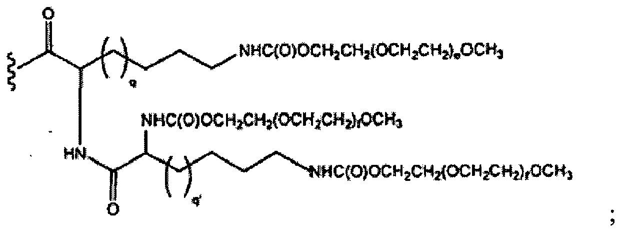 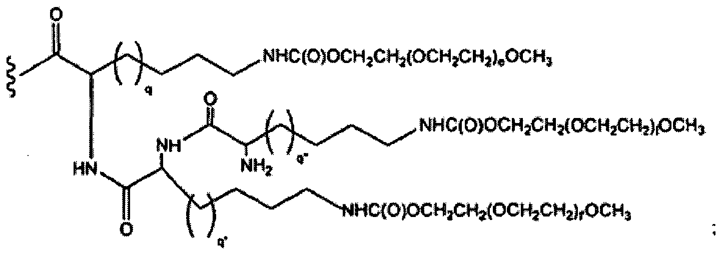 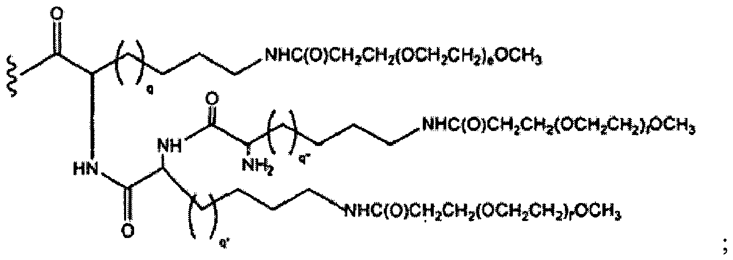 wobei
e, f und f Ganzzahlen unabhängig ausgewählt aus 1 bis 2500 sind; und
q, q' und q" Ganzzahlen unabhängig ausgewählt aus 1 bis 20 sind; und
L ein Linker ist, der ein Element ausgewählt aus einer Verbindung, substituiertem und unsubstituiertem Alkyl und substituiertem oder unsubstituiertem Heteroalkyl ist,
sodass wenn D gleich OH ist, G gleich R¹-L- ist, und wenn G gleich -C(O)(C₁-C₆)Alkyl ist, D gleich R¹-L-NH- ist,
genanntes Verfahren umfassend:
(a) Kontaktieren eines Substrates eines G-CSF Peptids mit einer PEG-Sialinsäure Donor Gruppe, die Formel aufweisend: 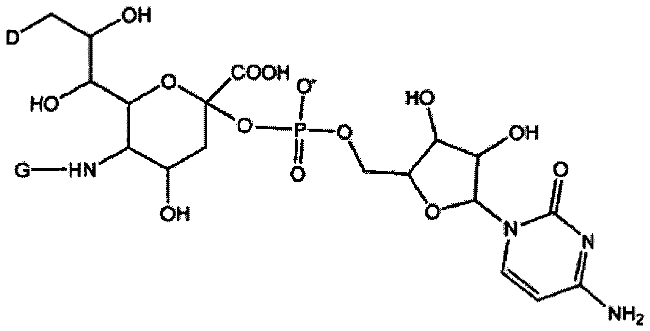 mit einem Enzym, das genannte PEG-Sialinsäure auf eine Aminosäure oder einen Glycosylrest des genannten G-CSF-Peptids überträgt, unter Bedingungen, die geeignet für diese Übertragung sind.

14. Verfahren gemäß Anspruch 13, wobei L-R¹ die Formel hat: 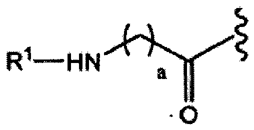 wobei
a eine Ganzzahl aus 0 bis 20 ist.

15. Verfahren gemäß Anspruch 13 oder 14, ferner vor Schritt (a) umfassend:
(b) Exprimieren des genannten Substrat Granulozyten-Kolonie stimulierender Faktor-Peptides in einem geeigneten Wirt.

16. Verfahren gemäß Anspruch 15, wobei genannter Wirt ausgewählt ist aus einer Insektenzelle und einer Säugerzelle.

17. Verfahren gemäß einem der Ansprüche 13 bis 16, das ferner Formulieren des Peptids in eine pharmazeutische Formulierung umfasst.

18. Verwendung eines Peptids gemäß einem der Ansprüche1 bis 12, zur Herstellung von einem Medikament zur Stimulierung der entzündlichen Leukozytenproduktion in einem Säugetier.

19. Verwendung von einem Peptid gemäß einem der Ansprüche 1 bis 12, zur Herstellung von einem Medikament zur Behandlung von Infektionen.

20. Pharmazeutische Formulierung umfassend ein Peptid gemäß einem der Ansprüche 1 bis 12 und einen pharmazeutisch annehmbaren Träger.

21. Peptid gemäß einem der Ansprüche 1 bis 12 zur therapeutischen Verwendung.

## Revendications

1. Peptide de Facteur de Stimulation de Colonies de Granulocytes comprenant le groupe fonctionnel : 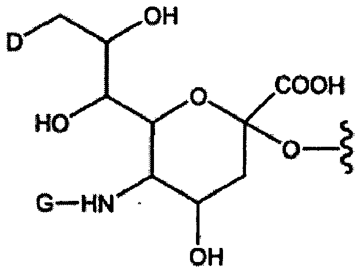 où
D est un élément sélectionné parmi -OH et R¹-L-HN- ;
G est un élément sélectionné parmi R¹-L- et -C(O)(C₁-C₆)alkyle ;
R¹ a une structure qui est un élément sélectionné parmi : 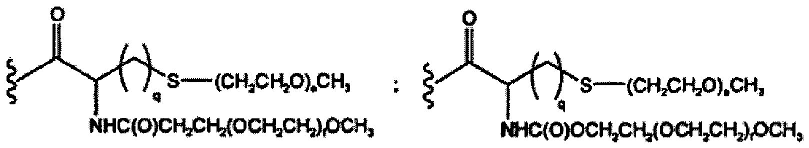 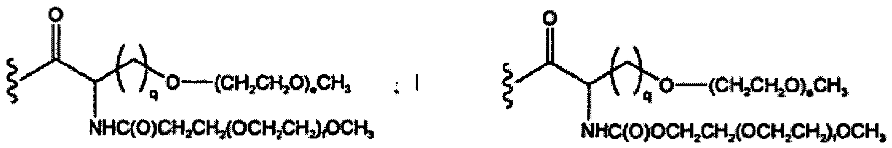 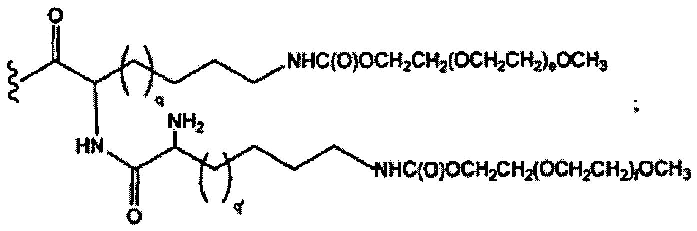 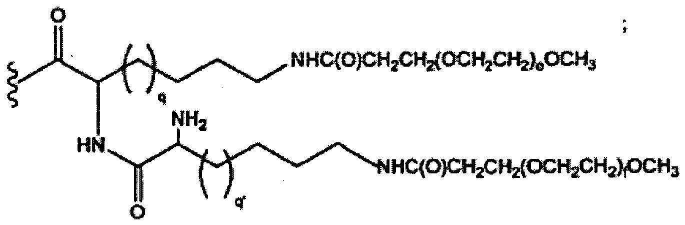 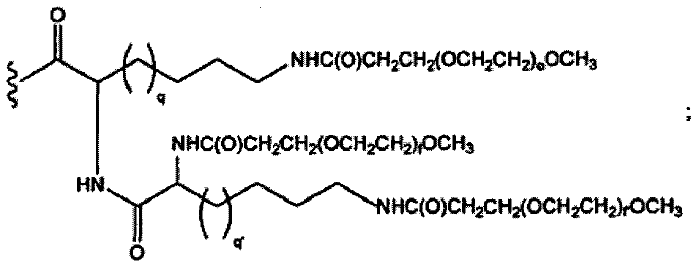 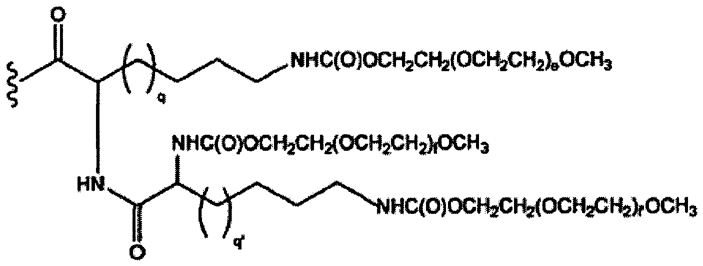 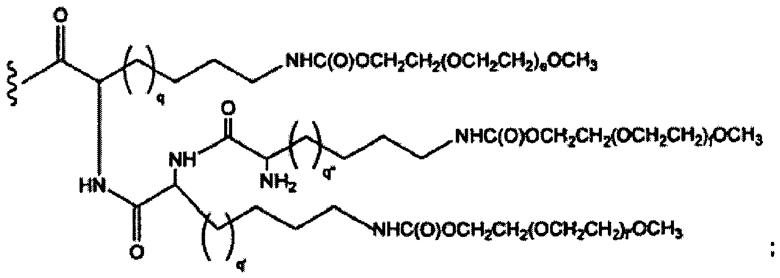 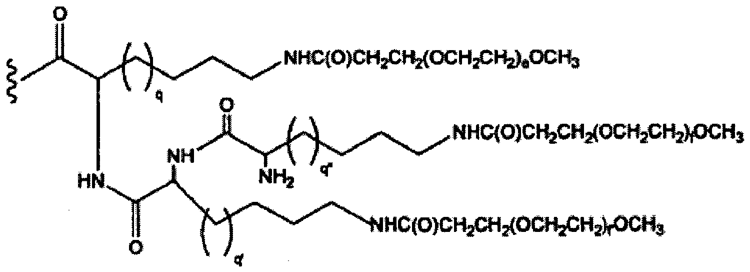 où
e, f et f' sont des entiers sélectionnés indépendamment entre 1 et 2500 ; et
q, q' et q'' sont des entiers sélectionnés indépendamment entre 1 et 20 ; et
L est un lieur qui est un élément sélectionné parmi une liaison, un alkyle substitué ou non substitué et un hétéroalkyle substitué ou non substitué,
de telle sorte que lorsque D est OH, G soit R¹-L-, et lorsque G est -C(O)(C₁-C₆)alkyle, D soit R¹-L-NH-.

2. Le peptide selon la revendication 1, où L-R¹ présente la formule : 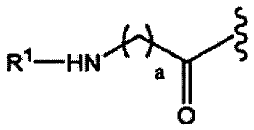 où
a est en entier de 0 à 20.

3. Le peptide selon une quelconque revendication précédente, où ledit groupe fonctionnel présente la formule :

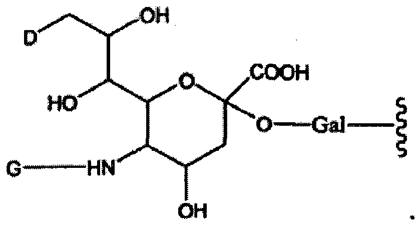

4. Le peptide FSC-G selon l'une quelconque des revendications 1 à 3, où ledit groupe fonctionnel présente la formule :

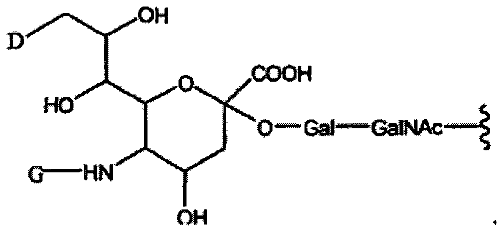

5. Le peptide FSC-G selon l'une quelconque des revendications 1 à 4, où ledit groupe fonctionnel présente la formule : 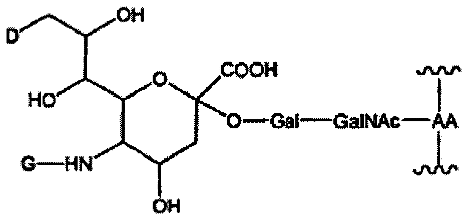 où
AA est un résidu d'acide aminé dudit peptide.

6. Le peptide selon la revendication 5, où ledit résidu d'acide aminé est de la serine ou de la thréonine.

7. Le peptide selon la revendication 5 ou la revendication 6, qui comprend la séquence d'acides aminés de SEQ ID N° 1.

8. Le peptide selon la revendication 7, où ledit résidu d'acide aminé est de la thréonine en position 133 de SEQ ID N° 1.

9. Le peptide selon une quelconque revendication précédente, qui comprend une séquence d'acides aminés sélectionnée parmi SEQ ID N° 1 et SEQ ID N° 2.

10. Le peptide selon une quelconque revendication précédente, qui comprend un groupe fonctionnel de la formule : 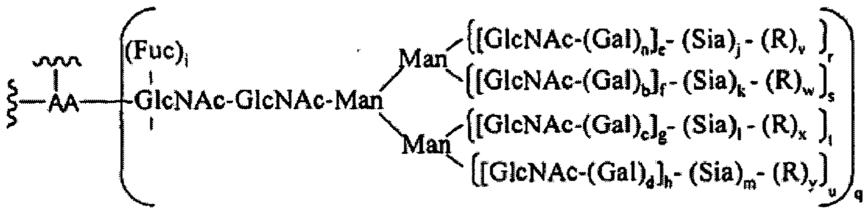 où
a, b, c, d, i, r, s, t, et u sont des entiers sélectionnés indépendamment parmi 0 et 1 ;
q est 1 ;
e, f, g, et h sont des éléments sélectionnés indépendamment parmi les entiers de 0 à 6 ;
j, k, l, et m sont des éléments sélectionnés indépendamment parmi les entiers de 0 à 100 ;
v, w, x, et y sont sélectionnés indépendamment parmi 0 et 1, et au moins l'un de v, w, x, et y est 1 ;
AA est un résidu d'acide aminé dudit peptide ;
Sia-(R) est un groupe fonctionnel de la formule :

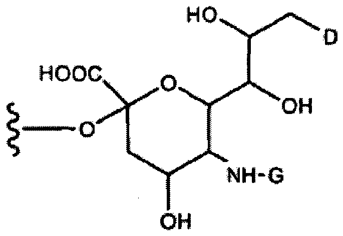

11. Le peptide selon la revendication 10, où ledit résidu d'acide aminé est un résidu d'asparagine.

12. Le peptide selon une quelconque revendication précédente, où ledit peptide est un peptide bioactif de Facteur de Stimulation de Colonies de Granulocytes.

13. Procédé de fabrication d'un conjugué de peptide FSC-G comprenant le groupe fonctionnel : 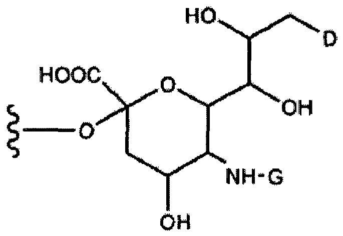 où
D est un élément sélectionné parmi -OH et R¹-L-HN- ;
G est un élément sélectionné parmi R¹-L- et -C(O)(C₁-C₆)alkyle ;
R¹ a une structure qui est un élément sélectionné parmi : 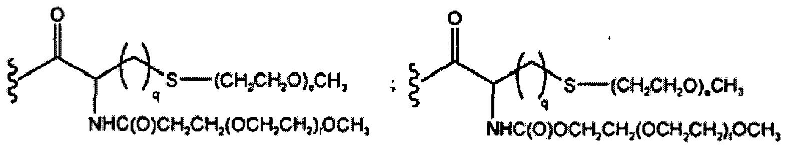 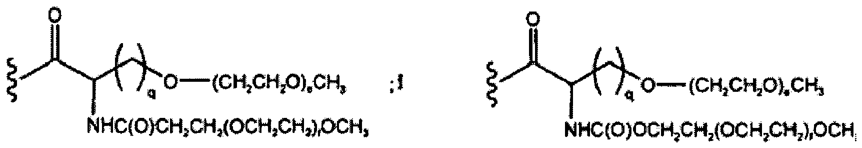 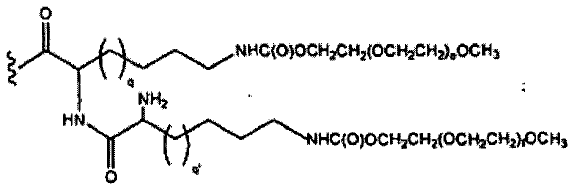 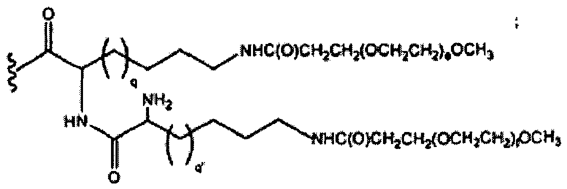 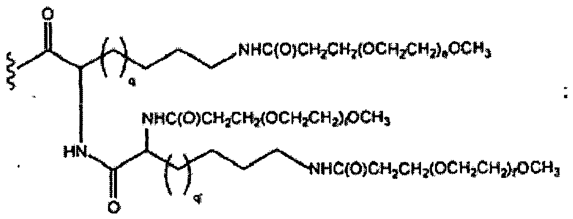 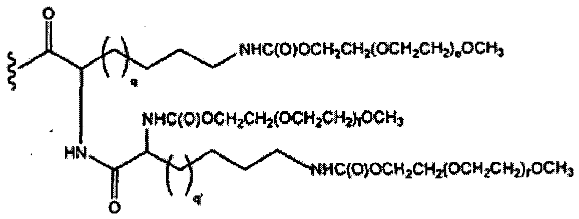 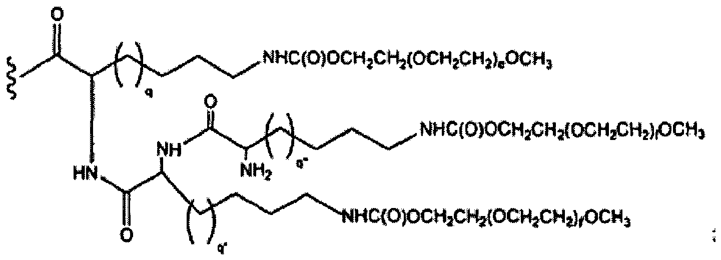 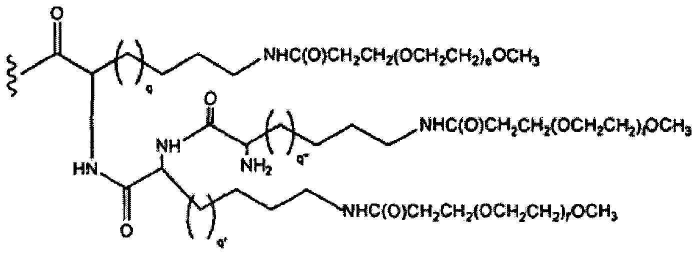 où
e, f et f' sont des entiers sélectionnés indépendamment entre 1 et 2500 ; et
q, q' et q'' sont des entiers sélectionnés indépendamment entre 1 et 20 ; et
L est un lieur qui est un élément sélectionné parmi une liaison, un alkyle substitué ou non substitué et un hétéroalkyle substitué ou non substitué,
de telle sorte que lorsque D est OH, G soit R¹-L-, et lorsque G est -C(O)(C₁-C₆)alkyle, D soit R¹-L-NH-,
ledit procédé comprenant :
(a) la mise en contact d'un peptide FSC-G substrat avec un groupe fonctionnel donneur d'acide sialique PEG présentant la formule : 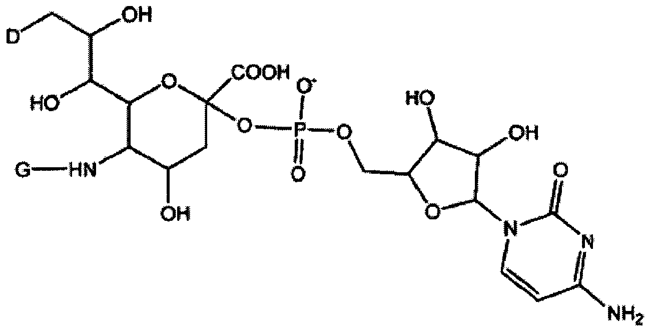
et un enzyme qui transfère ledit acide sialique PEG sur un résidu d'acide aminé ou de glycosyl dudit peptide FSC-G, dans des conditions appropriées pour le transfert.

14. Le procédé selon la revendication 13, où L-R¹ présente la formule : 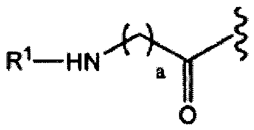 où
a est un entier de 0 à 20.

15. Le procédé de la revendication 13 ou 14, comprenant en outre, avant l'étape (a) :
(b) l'expression dudit peptide de Facteur de Stimulation de Colonies de Granulocytes substrat dans un hôte adéquat.

16. Le procédé de la revendication 15, où ledit hôte est sélectionné parmi une cellule d'insecte et une cellule de mammifère.

17. Le procédé de l'une quelconque des revendications 13 à 16, qui comprend en outre le fait de formuler le peptide dans une formulation pharmaceutique.

18. Utilisation d'un peptide selon l'une quelconque des revendications 1 à 12, pour la fabrication d'un médicament visant à stimuler la production de leucocytes inflammatoires chez un mammifère.

19. Utilisation d'un peptide selon l'une quelconque des revendications 1 à 12, pour la fabrication d'un médicament pour traiter l'infection.

20. Formulation pharmaceutique comprenant un peptide selon l'une quelconque des revendications 1 à 12 et un support pharmaceutiquement acceptable.

21. Peptide selon l'une quelconque des revendications 1 à 12, à usage thérapeutique.
